(19) Europäisches Patentamt
European Patent Office
Office européen des brevets

(11) Veröffentlichungsnummer: **0 237 482**
**A1**

# (12) EUROPÄISCHE PATENTANMELDUNG

(21) Anmeldenummer: **87810115.3**

(22) Anmeldetag: **02.03.87**

(51) Int.Cl.[3]: **C 07 D 493/22**
C 07 H 17/08, A 01 N 43/20
A 61 K 31/365
//(C07D493/22,313:00,311:00,
311:00,307:00)

(30) Priorität: **06.03.86 CH 918/86**

(43) Veröffentlichungstag der Anmeldung:
**16.09.87 Patentblatt 87/38**

(84) Benannte Vertragsstaaten:
**AT BE CH DE ES FR GB GR IT LI LU NL SE**

(71) Anmelder: **CIBA-GEIGY AG**
**Klybeckstrasse 141**
**CH-4002 Basel(CH)**

(72) Erfinder: **Gehret, Jean-Claude**
**Im Aeschfeld 12**
**CH-4147 Aesch(CH)**

(54) **C(29)-Carbonyloxi-milbemycin-Derivate zur Bekämpfung von tier- und pflanzenparasitären Schädlingen.**

(57) Es werden neue C(29)-Carbonyloxi-milbemycin-Derivate der Formel I, ihre Herstellung und ihre Verwendung zur Bekämpfung von Schädlingen, ferner Schädlingsbekämpfungsmittel, die mindestens eine dieser Verbindungen als Wirkstoff enthalten, beschrieben. Diese Verbindungen besitzen die allgemeine Formel I,

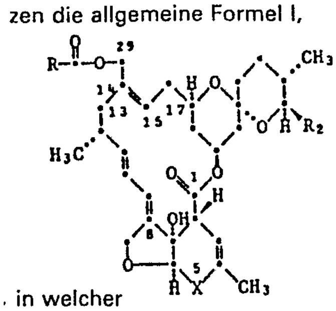

(I)

in welcher
X für $-CH(OR_1)-$, $-C(O)-$ oder $-C(=N-OH)-$ steht;
$R_1$ Wasserstoff, eine Silyl-, eine Acylgruppe oder einen Zuckerrest,
$R_2$ Methyl, Ethyl, Isopropyl oder sek.Butyl und
R Wasserstoff, unsubstituierte oder substituierte gerad- oder verzweigtkettige $C_1$-$C_{18}$-Alkyl-Gruppen, unsubstituierte oder substituierte $C_3$-$C_{10}$-Cycloalkyl-Gruppen, unsubstituierte oder substituierte $C_2$-$C_6$-Alkenyl-Gruppen, unsubstituierte oder substituierte $C_2$-$C_6$-Alkinyl-Gruppen oder unsubstituierte oder substituierte Phenyl-Gruppen bedeuten.

EP 0 237 482 A1

Croydon Printing Company Ltd.

0237482

CIBA-GEIGY AG
Basel (Schweiz)

5-15779/+

# C(29)-Carbonyloxi-milbemycin-Derivate zur Bekämpfung von tier- und pflanzenparasitären Schädlingen

Die vorliegende Erfindung betrifft neue C(29)-Carbonyloxi-milbemycin-Derivate der Formel I, ihre Herstellung und ihre Verwendung zur Bekämpfung von Schädlingen, ferner Schädlingsbekämpfungsmittel, die mindestens eine dieser Verbindungen als Wirkstoff enthalten. Darüberhinaus werden wichtige Zwischenprodukte der Formeln II und X beschrieben.

Die neuen Verbindungen entsprechen der allgemeinen Formel I

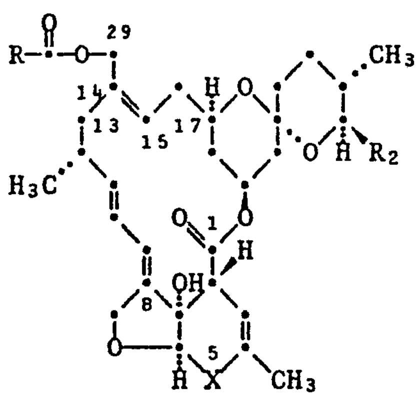

(I)

in welcher
X für $-CH(OR_1)-$, $-C(O)-$ oder $-C(=N-OH)-$ steht;
$R_1$ Wasserstoff, eine Silyl-, eine Acylgruppe oder einen Zuckerrest,
$R_2$ Methyl, Ethyl, Isopropyl oder sek.Butyl und
R Wasserstoff, unsubstituierte oder substituierte gerad- oder verzweigtkettige $C_1$-$C_{18}$-Alkyl-Gruppen, unsubstituierte oder substituierte $C_3$-$C_{10}$-Cycloalkyl-Gruppen, unsubstituierte oder sub-

stituierte $C_2$-$C_6$-Alkenyl-Gruppen, unsubstituierte oder substituierte $C_2$-$C_6$-Alkinyl-Gruppen oder unsubstituierte oder substituierte Phenyl-Gruppen bedeuten.

Im Rahmen der Formel I sind die Vertreter bevorzugt, worin X für $-CH(OR_1)-$ oder $-C(O)-$ steht.

Unter den vorgenannten Bedeutungen von R sind als bevorzugt anzusehen: $C_1$-$C_8$-Alkyl, $C_3$-$C_6$-Cycloalkyl, Phenyl, Halophenyl, $C_2$-$C_6$-Alkenyl und $C_2$-$C_6$-Alkinyl.

Als Substituenten der Alkyl-, Cycloalkyl-, Alkenyl- und Alkinyl-Gruppen kommen beispielsweise 1 bis 7 Halogenatome oder 1 bis 6 $C_1$-$C_6$-Alkoxi und als Substituenten der Phenyl-Gruppen 1 bis 3 Halogenatome, $C_1$-$C_6$-Alkyl, $C_1$-$C_6$-Alkoxi, $C_1$-$C_4$-Alkylthio oder Nitro in Betracht. Diese Substituenten können unabhängig voneinander in untereinander gemischter Anordnung auftreten. Als Substituent der Alkylgruppe kommt ausserdem auch eine unsubstituierte oder substituierte Phenoxigruppe in Frage, wie beispielsweise eine halogenierte, insbesondere eine durch 1 bis 3 Halogenatome substituierte Phenoxigruppe. Bei den Cycloalkylgruppen können als Substituenten auch $C_1$-$C_4$-Alkylgruppen vorliegen.

Unter dem Begriff Alkyl selbst oder als Bestandteil eines anderen Substituenten sind je nach Zahl der angegebenen Kohlenstoffatome beispielsweise folgende Gruppen zu verstehen: Methyl, Ethyl, Propyl, Butyl, Pentyl, Hexyl, Heptyl, Octyl, Nonyl, Decyl, usw. sowie die Isomeren, wie z.B. Isopropyl, Isobutyl, tert.-Butyl, Isopentyl usw.. Haloalkyl steht für einen einfach bis perhalogenierten Alkylsubstituenten, wie z.B. $CHCl_2$, $CHF_2$, $CH_2Cl$, $CCl_3$, $CF_3$, $CH_2F$, $CH_2CH_2Cl$, $CHBr_2$ usw.. Unter Halogen soll hier und im folgenden Fluor, Chlor, Brom oder Jod, vorzugsweise Fluor, Chlor oder Brom, verstanden werden. Alkenyl steht für einen mindestens durch eine C=C-Doppelbindung charakterisierten aliphatischen Kohlenwasserstoffrest, wie z.B. für Vinyl, Propenyl-(1), Allyl, Butenyl-(1), Butenyl-(2), Butenyl-(3), usw.. Haloalkenyl bedeutet dementsprechend einen

derartigen Alkenylrest, der ein- oder mehrfach halogeniert ist. Alkinyl steht für eine gerade oder verzweigte Kohlenstoffkette, die durch mindestens eine C≡C Dreifachbindung charakterisiert ist. Typische Vertreter sind: Ethinyl, Propionyl-(1), Propargyl, Butinyl-(1), usw.. Alkoxialkyl steht für eine unverzweigte oder verzweigte, durch ein Sauerstoffatom unterbrochene Alkylgruppe, somit z.B. für $CH_2OCH_3$, $CH_2CH_2OCH_3$, $CH_2CH(CH_3)OCH_3$, $CH_2OC_2H_5$, $CH_2OC_3H_7$-i, $CH_2CH_2CH_2$, $OCH_3$ usw. Alkoxialkoxialkyl steht für eine unverzweigte oder verzweigte Alkylgruppe, die an zwei Stellen durch jeweils ein Sauerstoffatom unterbrochen ist. Typische Beispiele sind: $CH_2OCH_2OCH_3$, $CH_2CH_2OCH_2OCH_3$, $CH_2OCH_2CH_2OCH_3$, $CH_2OCH_2OC_2H_5$, $-CH(CH_3)OCH_2OC_3H_7$-i usw..

Auf Grund ihrer ausgezeichneten Wirkung gegen Ektoparasiten am Nutztier, bilden die 5-Oxime [X = -C(=N-OH)-] eine wichtige Untergruppe im Umfang der Formel I.

Als Beispiele für R, die keine Limitierung darstellen, sind zu nennen Wasserstoff, Methyl, Ethyl, Propyl, Isopropyl, n-Butyl, sek.Butyl, tert.Butyl, Neopentyl, Chlormethyl, Trifluormethyl, Trichlormethyl, Trichlorethyl, Trichlor-tert.Butyl, 1,2,2,2-Tetrachlorethyl, 1,3,3,3-Tetrachlorpropyl, 3-Chlorpropyl, Ethenyl, Propenyl, Propinyl, Methoximethyl, Isopropoximethyl, 1-Methyl-1-methoxiethyl, 2,2-Dimethylvinyl, 1,2,2-Trichlorvinyl, 1,3,3,3-Tetrachlorpropyl, 1,1-Dichlor-2,2,2-trifluorethyl, 1,3-Pentadienyl, Ethinyl, 1-Propinyl, 1-Butinyl, Cyclopropyl, 2,2-Dimethylcyclopropyl, 1-Methylcyclopropyl, 2,2-Dimethyl-3-(2,2-dichlorvinyl)-cyclopropyl, Cyclobutyl, Cyclopentyl, Cyclohexyl Phenyl, Benzyl, p-Tolyl, p-Chlorphenyl, 2,6-Dichlorphenyl oder 2,4-Dinitrophenyl, Adamantyl oder 4-Fluorphenoximethyl.

Unter einem Zuckerrest sollen im Rahmen vorliegender Erfindung Mono-, Di- und Trisaccharide verstanden werden, deren Hydroxigruppen auch in veretherter oder veresterter Form vorliegen können. Typische Vertreter sind z.B. Monosaccharide: Glucose, Fructose, Altrose, Mannose, Sorbose, Gulose, Idose, Allose, Galactose, Ribose,

Arabinose, Xylose, Lyxose, Erythrose, Threose, Thamnose, Altrose, Talose sowie ihre entsprechenden Derivate wie Methylglucose, Trimethylglucose und Tetraacetylglucose sowie ein- oder mehrfach acetylierte Zucker,

Disaccharide: Lactose, Maltose, Cellobiose, Melibiose, Gentiobiose sowie ihre entsprechenden Derivate.

Weiterhin rechnen zu den für Formel I genannten Kohlehydraten Saccharide, die zusätzlich einen Aminorest, einen Thiolrest, einen aus zwei nachbarständigen OH-Gruppen und einem Aldehyd bzw. Keton gebildeten cyclischen Acetalrest enthalten.

Die Verknüpfung eines Saccharids in 5-Position der Verbindungen der Formel I kann als α-Anomeres oder β-Anomeres erfolgen. Die vorliegende Erfindung bezieht sich auf beide Bindungsarten.

Für die veretherte bzw. veresterte Form der Hydroxigruppen der Zuckerreste kommen primär folgende Substituenten in Frage Methyl, Benzyl, unsubstituiertes oder halogensubstituiertes $C_1$-$C_6$ aliphatisches Acyl, die Benzoylgruppe oder die $C_1$-$C_6$-Alkoxicarbonylgruppe.

Besonders bevorzugte Zuckerreste sind solche der Formel

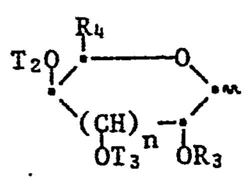

unter Einschluss ihrer Stellungsisomeren, wobei n für die Zahl 0 oder 1 steht; $R_4$ Wasserstoff, Methyl oder $-CH_2-O-T_1$ bedeutet und $R_4$, $T_1$, $T_2$ und $T_3$ unabhängig voneinander Wasserstoff, Methyl, Benzyl, eine unsubstituierte oder halogensubstituierte $C_1$-$C_6$-aliphatische Acylgruppe, eine Benzoylgruppe, oder eine $C_1$-$C_6$-Alkoxicarbonylgruppe bedeuten, oder wobei $T_1$ und $T_2$ zusammen mit dem Carbonyl-Kohlenstoffatom eines aliphatischen oder aromatischen Aldehyds oder

Ketons mit maximal 13 Kohlenstoffatomen ein cyclisches Acetal bilden. Besagte Zucker im Umfang der Formel I bilden eine interessante Gruppe von Milbemycinen.

Zur Bildung eines cyclischen Acetals an einem Zuckermolekül eignen sich einfache Aldehyde wie Acetaldehyd, Propionaldehyd, Butyraldehyd, Benzaldehyd oder Ketone wie Acetophenon, Cyclopentanon, Cyclohexanon, Cyclopentanon, Fluorenon, Methylethylketon, vor allem aber Aceton unter Bildung entsprechender Acetonide.

Verbindungen der Formel I, worin X für $-CH(OR_1)-$ steht, und $R_1$ Wasserstoff bedeutet, sind bevorzugt. Acyl- und Silylgruppen als $R_1$ sind im allgemeinen als Schutzgruppen aufzufassen, die ebenso wie Zuckerreste die biologische Aktivität der Substanzen nicht nachteilig beeinflussen.

Verbindungen, worin $R_2$ sek.Butyl darstellt, sollen hier und im folgenden gleichfalls zu den Milbemycin-Derivaten gerechnet werden, obwohl sie nach der üblichen Systematik von Avermectin-Derivaten abgeleitet sind. Avermectin-Aglykone (mit einer OH-Gruppe in 13α-Position) lassen sich jedoch gemäss US-PS 4,173,571 in Milbemycin-Homologe überführen.

In natürlich vorkommenden Milbemycinen ($R_1$=H; $R_2$=$CH_3$, $C_2H_5$ oder iso-$C_3H_7$ ist die 13-Position stets lediglich mit Wasserstoff besetzt. Bei Avermectinen dagegen steht in der 13-Position ein α-L-Oleandrosyl-α-L-oleandrose-Rest, der über Sauerstoff in α-Konfiguration mit dem Makrolid-Molekül verknüpft ist. Avermectine unterscheiden sich strukturell ausserdem durch eine 23-OH-Gruppe oder $\Delta^{22,23}$-Doppelbindung und in der Regel durch einen Substituenten $R_2$ = sek.$C_4H_9$ von den Milbemycinen. Durch Hydrolyse des Zucker-Restes der Avermectine gelangt man leicht zu den entsprechenden Avermectin-Aglykonen, die eine allylische 13α-Hydroxi-Gruppe besitzen. Die Avermectin-Aglykone sind wie vorstehend angegeben, in

die Milbemycin-Homologen umwandelbar. Bei den Milbemycin-Derivaten der vorliegenden Anmeldung liegt die $\Delta^{22,23}$-Doppelbindung stets in hydrierter Form vor.

Folgende Untergruppen von Verbindungen der Formel I sind auf Grund ihrer ausgeprägten parasitiziden und insektiziden Wirkung besonders bevorzugt:

Gruppe Ia: Verbindungen der Formel I, worin X für $-CH(OR_1)-$ steht; $R_1$ Wasserstoff darstellt, $R_2$ für Methyl, Ethyl, Isopropyl oder sek.Butyl steht und R folgende Bedeutungen hat:

- unsubstituiertes oder durch 1 bis 4 Halogenatome oder $C_1$-$C_4$-Alkoxi substituiertes $C_1$-$C_6$-Alkyl, $C_2$-$C_4$-Alkenyl, $C_2$-$C_4$-Alkinyl oder $C_3$-$C_6$-Cycloalkyl;
- unsubstituiertes oder ein bis dreifach durch Halogen, $C_1$-$C_4$-Alkyl, $C_1$-$C_4$-Alkoxi, $C_1$-$C_4$-Alkylthio oder Nitro substituiertes Phenyl.

Gruppe Ib: Verbindungen der Formel I, worin X für $-CH(OR_1)-$ steht; $R_1$ Wasserstoff darstellt, $R_2$ für Methyl, Ethyl, Isopropyl oder sek.Butyl steht und R folgende Bedeutungen hat:

- unsubstituiertes oder durch 1 bis 4 Chlor- oder Fluoratome oder Methoxi substituiertes $C_1$-$C_5$-Alkyl, $C_2$-$C_3$-Alkenyl, $C_2$-$C_3$-Alkinyl oder $C_3$-$C_6$-Cycloalkyl;
- unsubstituiertes oder durch Chlor, Fluor, $C_1$-$C_2$-Alkyl, $C_1$-$C_2$-Alkoxi, $C_1$-$C_2$-Alkylthio oder Nitro substituiertes Phenyl.

Gruppe Ic: Verbindungen der Formel I, worin X für $-CH(OR_1)-$ steht; $R_1$ Wasserstoff darstellt, $R_2$ für Methyl oder Ethyl steht und R folgende Bedeutungen hat:

- unsubstituiertes oder durch 1 bis 4 Chlor- oder Fluoratome oder Methoxi substituiertes $C_1$-$C_5$-Alkyl, $C_2$-$C_3$-Alkenyl, $C_2$-$C_3$-Alkinyl oder $C_3$-$C_6$-Cycloalkyl;

- unsubstituiertes oder durch Chlor, Fluor, $C_1$-$C_2$-Alkyl, $C_1$-$C_2$-Alkoxi, $C_1$-$C_2$-Alkylthio oder Nitro substituiertes Phenyl.

Gruppe Id: Verbindungen der Formel I, worin X für -CH($OR_1$)- steht; $R_1$ Wasserstoff darstellt, $R_2$ für Isopropyl oder sek.Butyl steht und R folgende Bedeutungen hat:

- unsubstituiertes oder durch 1 bis 4 Chlor- oder Fluoratome oder Methoxi substituiertes $C_1$-$C_5$-Alkyl, $C_2$-$C_3$-Alkenyl, $C_2$-$C_3$-Alkinyl oder $C_3$-$C_6$-Cycloalkyl;
- unsubstituiertes oder durch Chlor, Fluor, $C_1$-$C_2$-Alkyl, $C_1$-$C_2$-Alkoxi, $C_1$-$C_2$-Alkylthio oder Nitro substituiertes Phenyl.

Gruppe Ie: Verbindungen der Formel I, worin X für -CH($OR_1$)- steht; $R_1$ Wasserstoff darstellt, $R_2$ für Methyl oder Ethyl, Isopropyl oder sek.Butyl steht und R folgende Bedeutungen hat:

- unsubstituiertes oder durch 1 bis 3 Chloratome oder Fluoratome oder Methoxi substituiertes $C_1$-$C_5$-Alkyl, $C_2$-$C_3$-Alkenyl, $C_2$-$C_3$-Alkinyl oder $C_3$-$C_6$-Cycloalkyl;
- unsubstituiertes oder durch Chlor, Fluor, $C_1$-$C_2$-Alkyl, $C_1$-$C_2$-Alkoxi, $C_1$-$C_2$-Alkylthio oder Nitro substituiertes Phenyl.

Gruppe If: Verbindungen der Formel I, worin X für -CH($OR_1$)- steht; $R_1$ Wasserstoff darstellt, $R_2$ für Methyl oder Ethyl oder steht und R folgende Bedeutungen hat:

- unsubstituiertes oder durch 1 bis 3 Chloratome oder Fluoratome oder Methoxi substituiertes $C_1$-$C_5$-Alkyl, $C_2$-$C_3$-Alkenyl, $C_2$-$C_3$-Alkinyl oder $C_3$-$C_6$-Cycloalkyl.

Gruppe Ig: Verbindungen der Formel I, worin X für -CH($OR_1$)- steht; $R_1$ Wasserstoff darstellt, $R_2$ für Isopropyl oder sek.Butyl steht und R folgende Bedeutungen hat:

- unsubstituiertes oder durch 1 bis 3 Chloratome oder Fluoratome oder Methoxi substituiertes $C_1$-$C_5$-Alkyl, $C_2$-$C_3$-Alkenyl, $C_2$-$C_3$-Alkinyl oder $C_3$-$C_6$-Cycloalkyl.

Gruppe Ih: Verbindungen der Formel I, worin X für -C(=N-OH)- steht; $R_2$ für Methyl oder Ethyl steht und R folgende Bedeutung hat:

- unsubstituiertes oder durch 1 bis 3 Chloratome oder Fluoratome oder Methoxi substituiertes $C_1$-$C_5$-Alkyl, $C_2$-$C_3$-Alkenyl, $C_2$-$C_3$-Alkinyl oder $C_3$-$C_6$-Cycloalkyl;
- unsubstituiertes oder durch Chlor, Fluor, $C_1$-$C_2$-Alkyl, $C_1$-$C_2$-Alkoxi, $C_1$-$C_2$-Alkylthio oder Nitro substituiertes Phenyl.

Besonders bevorzugte 5-Hydroxi-Derivate der Formel I sind z.B.:

29-tert.Butylcarbonyloxi-milbemycin-D,
29-Cyclopropylcarbonyloxi-milbemycin-$A_4$,
29-tert.Butylcarbonyloxi-milbemycin-$A_4$,
29-iso-Butylcarbonyloxi-milbemycin-$A_4$,
29-(2,2-Dimethylpropyl)carbonyloxi-milbemycin-$A_4$ und
29-Acetoxi-milbemycin-D.

Bevorzugte an der 5-Hydroxigruppe mit einer Schutzgruppe versehene Verbindung der Formel I sind z.B. :

5-O-tert.Butyldimethylsilyl-29-tert.butylcarbonyloxi-milbemycin-D,
5-O-tert.Butyldimethylsilyl-29-cyclopropylcarbonyloxi-milbemycin-$A_4$,
5-O-tert.Butyldimethylsilyl-29-acetoxi-milbemycin-D,
5-O-tert.Butyldimethylsilyl-29-tert.butylcarbonyloxi-milbemycin-$A_4$,
5-O-tert.Butyldimethylsilyl-29-(2,2-dimethylcarbonyloxi-milbemycin-$A_4$,
5-O-tert.Butyldimethylsilyl-29-iso-butylcarbonyloxi-milbemycin-$A_4$ und
5-O-2',3',4'6'-Tetra-O-acetyl-galactose-29-tert.-butylcarbonyloxi-milbemycin-D.

Die Verbindungen der Formel I werden erfindungsgemäss dadurch hergestellt, dass man entweder eine Verbindung der Formel II

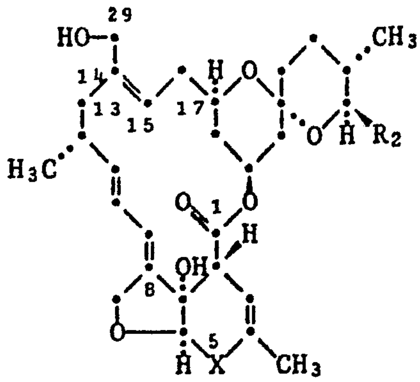

(II)

oder eine Verbindung der Formel III,

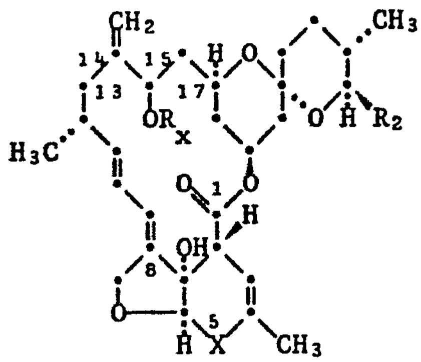

(III)

wobei in den Formeln II und III $R_2$ eine der unter Formel I angegebenen Bedeutungen hat, X für $-CH(OR_1)-$ steht; wobei $R_1$ eine OH-Schutzgruppe bedeutet oder X für $-C(O)-$ steht und $R_x$ in Formel III für Wasserstoff oder eine leicht abspaltbare Gruppe steht, mit einer Säure der Formel IV

R-COOH (IV)

worin R eine der unter Formel I angegebenen Bedeutungen hat, oder einen zur Veresterung befähigten Derivat dieser Säure reagieren lässt oder die Verbindung der Formel III durch Verseifung der $OR_x$-Gruppe zuerst in eine Verbindung der Formel II überführt und anschliessend mit IV umsetzt und die resultierende Verbindung der Formel I insofern gewünscht, durch Abspaltung der OH-Schutzgruppe in

ein 5-Hydroxi-Derivat der Formel überführt und letzteres, sofern gewünscht durch Nachsilylierung in ein Silylderivat oder durch Einführung eines Zuckerrestes in ein Zuckerderivat der Formel I überführt und sofern Verbindungen der Formel I, worin X für -C(=N-OH)- steht, das Herstellungsziel darstellen, ein 5-Keton der Formel I mit Hydroxylamin oder einem seiner Salze umsetzt.

Die Verbindungen der Formeln II und III sind auf Grund ihrer spezifischen Struktur für die Herstellung der wertvollen Endprodukte der Formel I prädestiniert und bilden einen Bestandteil vorliegender Erfindung.

Zur Veresterung befähigt Säurederivate der Säure IV sind z.B.:

a) ihre Säureamide der Formel V

$$RCON(Alkyl)_2 \qquad (V)$$

worin die Alkylreste 1 bis 4 Kohlenstoffatome umfassen und vorzugsweise Methyl bedeuten,

b) ihre Säurehalogenide der Formel VI

$$RCOhal \qquad (VI),$$

worin hal Halogen bedeutet und bevorzugt für Chlor oder Brom steht, sowie

c) ihr Säureanhydrid der Formel VII

$$(RCO)_2O \qquad (VII).$$

In den vorstehend angegebenen Formeln V bis VII besitzt R die unter Formel I angegebenen Bedeutungen.

Die Reaktionen zur Herstellung von Verbindungen der Formel I werden zweckmässigerweise mit Verbindungen der Formeln II oder III durchgeführt werden, deren reaktive 5-Hydroxi-Gruppe geschützt ist.

Unter OH-Schutzgruppen für den Substituenten $R_1$ sollen hier und im folgenden die in der organischen Chemie üblichen Schutzfunktionen verstanden werden. Dabei handelt es sich insbesondere um Acyl- und Silylgruppen. Geeignete Acylgruppen sind beispielsweise die Reste $R_5$-C(O)-, wobei $R_5$ für $C_1$-$C_{10}$-Alkyl, $C_1$-$C_{10}$-Haloalkyl oder eine unsubstituierte oder durch Halogen, $C_1$-$C_3$-Alkyl, $C_1$-$C_3$-Haloalkyl, $C_1$-$C_3$-Alkoxi, $C_1$-$C_3$-Haloalkoxi, Cyano und/oder Nitro substituierte Gruppe aus der Reihe Phenyl und Benzyl steht und vorzugsweise $C_1$-$C_6$-Alyl, $C_1$-$C_6$-Haloalkyl oder unsubstituiertes oder durch Halogen, $C_1$-$C_3$-Alkyl, $CF_3$ oder Nitro substituiertes Phenyl bedeutet. Als geeignete Silylgruppen für $R_1$ kommt der Rest -Si($R_6$)($R_7$)($R_8$) in Frage, wobei $R_6$, $R_7$ und $R_8$ vorzugsweise unabhängig voneinander für $C_1$-$C_4$-Alkyl, Benzyl oder Phenyl stehen und beispielsweise eine der Gruppen Trimethylsilyl, Diphenyl-tert.butylsilyl, bis(Isopropyl)-methylsilyl, Triphenylsilyl usw. und insbesondere tert.Butyl-dimethylsilyl bildet. Die 5-OH-Gruppe kann auch als Benzylether oder Methoxiethoximethylether vorliegen.

Verbindungen der Formel I, worin $R_1$ eine Schutzgruppe darstellt, lassen sich durch einfache, z.B. hydrolytische Abspaltung der Schutzfunktion in die hochaktiven freien 5-Hydroxi-derivate ($R_1$=H) überführen und haben somit auch Zwischenprodukte-Charakter. Im übrigen wird der biologische Wert dieser Verbindungen durch die Schutzgruppe oder den Zuckerrest nicht gemindert.

Das Verfahren wird im allgemeinen in einem reaktions-inerten Lösungsmittel oder in einem der beteiligten Reaktanden, sofern diese flüssig sind, durchgeführt. Geeignete Lösungsmittel sind z.B.: Ether und etherartige Verbindungen wie Dialkylether (Diethylether, Diisopropylether, tert.-Butylmethylether, Dimethoxiethan, Dioxan, Tetrahydrofuran, Anisol, usw.); halogenierte Kohlenwasserstoffe wie Chlorbenzol, Methylenchlorid, Ethylenchlorid, Chloroform, Tetra-

chlorkohlenstoff, Tetrachlorethylen, usw.; oder Sulfoxide wie Dimethylsulfoxid, wobei auch aromatische oder aliphatische Kohlenwasserstoffe, wie Benzol, Toluol, Xylole, Petrolether, Ligroin, Cyclohexan, usw. anwesend sein können. In manchen Fällen kann es von Vorteil sein, wenn die Reaktionen unter Schutzgasatmosphäre (z.B. Argon, Helium, Stickstoff, etc.) und/oder in absoluten Lösungsmitteln durchgeführt werden. Gewünschtenfalls können die Endprodukte auf übliche Art und Weise gereinigt werden, z.B. durch Waschen, Digerieren, Extraktion, Umkristallisation, Chromatographie usw..

Die Reaktion von Verbindungen der Formel II mit Säurehalogeniden oder Säureanhydriden der Formeln VI bzw. VII wird normalerweise in den obengenannten reaktionsinerten Lösungsmitteln im allgemeinen bei Temperaturen von 0° bis 100°C, vorzugsweise von 20° bis 60°C, durchgeführt. Zum Abfangen der dabei als Nebenprodukt gebildeten Säuren findet die Reaktion zweckmässigerweise in Gegenwart eines Neutralisationsmittels statt. Ferner kann auch ein Katalysator wie p-Dimethylaminopyridin zugesetzt werden.

Als solche kommen organische Basen in Betracht, z.B. tertiäre Amine wie Trialkylamine (Trimethylamin, Triethylamin, Diisopropylethylamin, Tripropylamin, usw.), Pyridin und Pyridinbasen (4-Dimethylaminopyridin, 4-Pyrrolidylaminopyridin usw.), bevorzugt ist Pyridin. Das Neutralisationsmittel wird üblicherweise in mindestens äquimolarer Menge in bezug auf die Ausgangsstoffe eingesetzt. Die organischen Basen können auch als Lösungsmittel eingesetzt werden.

Setzt man die Säure der Formel IV als Reagenz ein, so wird die Reaktion zweckmässigerweise in Gegenwart wasserabspaltender Reagenzien durchgeführt. Man arbeitet beispielsweise in Anwesenheit von Dicyclohexylcarbodiimid und Pyridin oder in Gegenwart von Azodicarbonsäuredialkylester und Triphenylphosphin.

Die Umsetzung von Verbindungen der Formel II mit Säureamiden der Formel V erfolgt vorzugsweise in Gegenwart von Orthoestern sowie in Gegenwart katalytischer Mengen einer Säure. Als dafür geeignete

Säuren können Protonensäuren oder Lewis-Säuren eingesetzt werden. Beispiele derartiger Säuren sind anorganische Säuren: Halogenwasserstoffsäure wie Chlorwasserstoffsäure, Bromwasserstoffsäure oder Jodwasserstoffsäure, Perchlorsäure sowie Schwefelsäure und organische Säuren wie Essigsäure, Trifluoressigsäure, Trichloressigsäure, Propionsäure, Oxalsäure, Ameisensäure, Benzolsulfonsäure, p-Toluolsulfonsäure, Methansulfonsäure usw. sowie Lewis-Säuren, wie $BF_3$, $AlCl_3$. $ZnCl_2$ usw. Besonders bevorzugt sind p-Toluolsulfonsäure und Schwefelsäure.

Die bei dieser Reaktion benötigten Orthoester haben die Formel VIII

$$R_yC(OR_z)_3 \qquad (VIII),$$

worin $R_y$ Wasserstoff oder $C_1$-$C_4$-Alkyl, bevorzugt Methyl, $R_z$ $C_1$-$C_4$-Alkyl, bevorzugt Methyl oder Ethyl bedeuten und R die unter Formel I angegebenen Bedeutungen besitzt.

Bei Verwendung von Säureamiden der Formel V zur Herstellung von Verbindungen der Formel I liegen die Reaktionstemperaturen im allgemeinen im Bereich von 0° bis 150°C, bevorzugt von 20° bis 130°C.

Die Umsetzung von 15-Derivate der Formel III mit einer Säure der Formel IV einem ihrer reaktionsfähigen Derivate erfolgt normalerweise in einem der oben genannten, inerten Lösungsmittel und kann entweder in Gegenwart einer der bereits genannten Basen oder alternativ im Beisein einer Säure durchgeführt werden. Als geeignete Säuren sind insbesondere Sulfonsäuren, wie z.B. die p-Toluolsulfonsäure, die Methansulfonsäure oder die Campfersulfonsäure zu nennen. Man versetzt die Substanz der Formel III im allgemeinen bei Temperaturen von 0° bis 100°C, vorzugsweise 30° bis 60°C, mit einer Säure der Formel IV.

Durch eine allylische Substitution (SN2') erhält man die 29-substituierten Milbemycine der Formel II. Bei Abwesenheit der Säure IV, aber in Gegenwart von Wasser, erhält man die 29-Hydroxi-milbemycine. Letztere allylische Substitution wird normalerweise bei 0° bis 80°C, vorzugsweise bei 20° bis 50°C durchgeführt.

Als leicht abspaltbare Gruppen $R_x$ in Formel III werden im Rahmen vorliegender Erfindung z.B. insbesondere Acylgruppen genannt, so z.B. Formyl, Acetyl, Benzoyl, Ethoxycarbonyl oder $P(=O)(O\ Alkyl)_2$ wie $P(O)(OEt)_2$, aber auch Alkylsulfonyl-Reste, wie Benzolsulfonyl, Paratosyl oder vorzugsweise Niederalkylsulfonyl, insbesondere Mesyl und in gewissen Fällen Tetrahydropyranyl.

Die Herstellung von Verbindungen der Formel I, die an das Sauerstoffatom in der 5-Position einen Zuckerreste gebunden haben, stellt eine Derivatisierung der reaktionssfähigen 5-Hydroxigruppe ($R_1$=H) mit einem geeigneten Zuckermolekül dar und wird nach einem der in der Zuckerchemie verwendeten Verknüpfungsmethoden, z.B. nach der Koenigs-Knorr-Synthese, dem Ag-Triflat-Prozess, nach dem sogenannten Orthoester-Verfahren, der Phenylthio-Synthese oder der 2-Pyridylthio-Synthese durchgeführt.

A) Nach der Koenigs-Knorr-Synthese oder dem Silber-Triflat-Prozess lässt sich ein 5-Hydroxi-Milbemycin der Formel I ($R_1$=H) in Gegenwart eines Silbersalzes oder Quecksilber-Salzes als Kondensationsmittel mit dem einzuführenden Zuckerrest, worin sämtliche OH-Gruppen geschützt sind, mit Ausnahme der durch Chlor oder Brom substituierten 1-OH-Gruppe im Temperaturbereich von -30°C bis +60°C, bevorzugt -5°C bis +30°C unter Licht-Ausschluss gewinnen.

Als Silbersalz lässt sich frisch gefälltes $Ag_2O$ verwenden, vorzugsweise jedoch $Ag_2CO_3$ oder $CF_3$-COOAg. Besonders bevorzugt ist Silber-Trifluormethansulfonat (Ag-Triflat = $CF_3$-$SO_3Ag$), in dessen Gegenwart die Glykosidierung bereits bei Minustemperaturen schnell abläuft. Zum Aktivieren der 5-OH-Gruppe des 5-Hydroxi-Milbemycins und zum Neutralisieren der gegebenenfalls entstehenden $CF_3$-$SO_3H$ bzw.

$CF_3-COOH$ setzt man zweckmässig ein tert.-Amin (Triethylamin, Diisopropylethylamin, Diazabicycloundecan u.a.) der Reaktionslösung zu.

Sofern gewünscht wird, können die Schutzgruppen durch milde Verseifung (z.B. $NH_3/CH_3OH$) anschliessend abgespalten werden. Als Lösungsmittel kommen bei diesem Teilschritt insbesondere wasserfreie aprotische Vertreter wie Dichlormethan, Acetonitril, Benzol, Toluol, Nitromethan, Dioxan, THF, Ethylenglykoldimethylether in Frage; Diethylether ist besonders geeignet.

Der geschützte 1-Chlor- oder 1-Brom-Zucker wird in äquimolarer Menge zum 5-Hydroxi-Milbemycin (I) eingesetzt, vorzugsweise jedoch in einem 1,5- bis 3-fachen Ueberschuss. Die Reaktionsdauer beträgt, um eine befriedigende Ausbeute zu erzielen, 5 bis 72 Stunden.

Anstatt des Silbersalzes lässt sich auch Hg-Cyanid oder eine Kombination von HgO mit wahlweise Hg-Chlorid oder Hg-Bromid verwenden (Helferich-Synthese).

Nach einer weiteren Variante lässt sich die Reaktivität in der 1'-Stellung des glykosidisch zu verknüpfenden Zuckers, dessen weitere OH-Gruppen geschützt sein müssen, durch anfängliche Umwandlung in das 1'-Phenylthio-Derivat und anschliessende Reaktion mit DAST (= Diethylaminoschwefeltrifluorid) in absolut trockenem Dichlormethan (Molekularsieb) bei +5°C bis -30°C zum 1'-Fluorderivat erhöhen. Reaktiver als das entsprechende, bei der Koenigs-Knorr-Synthese eingesetzte 1'-Chlor- oder 1'-Brom-Derivat lässt sich das so gewonnene 1'-Fluor-Derivat des Zucker-Reaktanden mit 5-Hydroxi-Milbemycin (I) in Gegenwart von $SnCl_2$ und $AgClO_4$ in einem trockenen aprotischen Lösungsmittel wie Diethylether unter Schutzgas wie Argon bei +5°C bis -30°C verknüpfen. (J. Am. Soc. 1984, 106, 4189-4192).

B) Eine bessere Reaktion wird erzielt, wenn das in 1'-Stellung zu aktivierende, gleichermassen geschützte Kohlehydrat mit 2,2'-Dithiopyridin in trockenem Dichlormethan bei ca. 0°C und unter Argon-

Atmosphäre in das 1'-S-(2-Pyridyl)-Kohlehydrat überführt wird, das mit der freien 5-OH-Gruppe des 5-Hydroxi-Milbemycins in Gegenwart von $Pb(ClO_4)_2$ oder $AgClO_4$ als Kondensationsmittel bei Raumtemperatur in Tetrahydrofuran (THF) als Lösungsmittel leicht unter Bildung der glykosidischen Bindung reagiert. (J. Org. Chem. 1983, 48, 3489-3493).

C) Glykosidische Verknüpfungen lassen sich auch in Gegenwart von Lewis-Säuren erzielen, wie $AlCl_3$, $AlBr_3$, $SnCl_4$, $ZnCl_2$, $BF_3$ (sowie vor allem das Etherat), wozu insbesondere acetylierte Zucker sehr geeignet sind. (Chimia 21, 1967, S. 537-538).

D) Nach der sogenannten Orthoester-Methode lassen sich glykosidische Bindungen auch durch Reaktion von Milbemycin mit dem zu verknüpfenden OH-geschützten Zucker in Gegenwart des Orthoesters eines niederen Alkohols erzielen, dessen eine alkoholische Komponente der Zucker-Reaktand ist.

Das Verfahren zur Herstellung von 5-Zucker-Milbemycin-Derivaten der Formel I, ist gekennzeichnet im engeren Sinne durch Reaktion von 5-Hydroxi-Milbemycin der Formel I

a) in Gegenwart eines Silbersalzes oder Quecksilber-Salzes als Kondensationsmittel mit dem einzuführenden Zuckerrest worin sämtliche OH-Gruppen geschützt sind, mit Ausnahme der in 1-Stellung durch Chlor oder Brom substituierten anomeren 1-OH-Gruppe unter Licht-Ausschluss im Temperaturbereich von -30°C bis +60°C, bevorzugt -5°C bis +30°C; oder

b) in Gegenwart von $SnCl_2$ und $AgClO_4$ als Kondensationsmittel mit dem einzuführenden Zuckerrest, worin sämtliche OH-Gruppen geschützt sind, mit Ausnahme der in 1-Stellung durch Fluor substituierten anomeren 1-OH-Gruppe unter Lichtausschluss bei +5°C bis -30°C;

und sofern gewünscht, milder Verseifung der Hydroxil-Schutzgruppen.

Die Herstellung der Oxime [X = -C(=N-OH)-] im Umfang der Formel I erfolgt durch Reaktion einer 5-Keto-Verbindung [X = -C(O)-] der Formel I mit Hydroxylamin oder eines seiner Salze, vorzugsweise eines seiner Mineralsäuresalze, insbesondere seinem Hydrochlorid. Die Reaktion wird zweckmässigerweise in einem geeigneten Lösungsmittel, z.B. einem Niederalkanol, wie Methanol, Ethanol, Propanol; einer etherartigen Verbindung, wie Tetrahydrofuran oder Dioxan; einer aliphatischen Carbonsäure, wie Essigsäure oder Propionsäure; Wasser oder in Gemischen dieser Lösungsmittel untereinander oder mit anderen üblichen reaktionsinerten Lösungsmitteln. Die Reaktionstemperaturen können in weiten Bereichen variieren. Man arbeitet zweckmässigerweise etwa im Bereich von +10° bis +100°C. Wird Hydroxylamin in Form eines seiner Salze, z.B. als Hydrochlorid eingesetzt, so ist es vorteilhaft, wenn man zum Abfangen der Säure (z.B. HCl) eine der für solche Zwecke üblichen Basen zusetzt und gegebenenfalls in Gegenwart eines Wasserbinders, z.B. eines Molekularsiebes, arbeitet. Als geeignete Basen kommen organische und anorganische Basen in Betracht, z.B. tertiäre Amine wie Trialkylamine (Trimethylamin, Triethylamin, Tripropylamin usw.), Pyridin und Pyridinbasen (4-Dimethylaminopyridin, 4-Pyrrolidylaminopyridin usw.), Oxide, Hydride und Hydroxide, Carbonate und Hydrogencarbonate von Alkali- und Erdalkalimetallen (CaO, BaO, NaOH, KOH, NaH, $Ca(OH)_2$, $KHCO_3$, $NaHCO_3$, $Ca(HCO_3)_2$, $K_2CO_3$, $Na_2CO_3$), sowie Alkaliacetate wie $CH_3COONa$ oder $CH_3COOK$. Darüberhinaus eignen sich auch Alkalialkoholate wie $C_2H_5ONa$, $C_3H_7$-nONa usw. Bevorzugt wird Triethylamin.

Die Verbindungen der Formel II werden erfindungsgemäss durch eine oxidative allylische Umlagerung aus 15-Hydroxi-Milbemycinderivaten der Formel IX

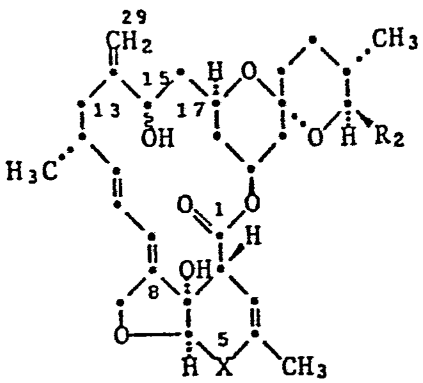

(IX),

worin X und $R_2$ die unter Formel I angegebenen Bedeutungen haben, und anschliessender selektiver Reduktion des intermediär erhaltenen 29-Aldehyds erhalten.

Die Reaktion kann wie folgt skizziert werden:

1. Teilschnitt: Oxidative allylische Umlagerung

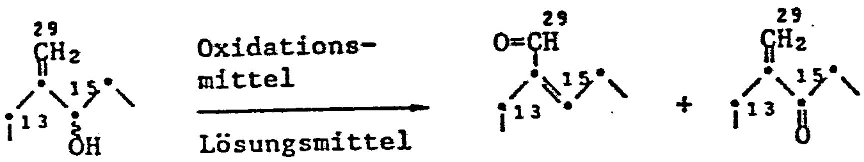


(IX) (X) (XI)

des Allylalkohols (IX) zu einer 29-Oxo-Verbindung (X). Hierbei wird der Allylalkohol (IX) mit einem geeigneten Oxidationsmittel in einem reaktionsinerten Lösungsmittel oxidativ in den entsprechenden Aldehyd (=29-Oxo-Verbindung) (X) umgelagert. Dabei entsteht im allgemeinen als Nebenprodukt das entsprechende ungesättigte Keton (XI), das seinerseits Zwischenproduktcharakter aufweist, da es auf Grund seiner Reaktivität zur Synthese weiterer Milbemycin-Derivate verwendet werden kann. Normalerweise entstehen nebeneinander trans- und cis-Form des Aldehyds (X), wobei die trans-Form im allgemeinen überwiegt.

Die 29-Oxo-Verbindungen der Formel X haben folgende chemische Struktur

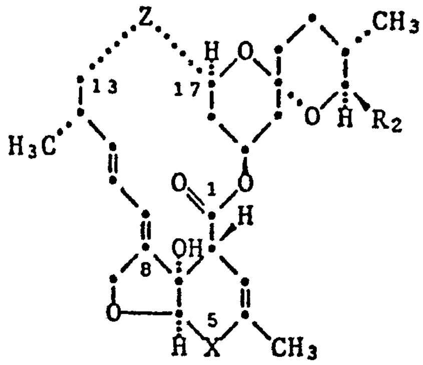

(X),

worin Z für eine der Gruppen

α) 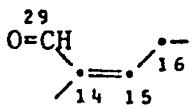 [= $\Delta^{14,15\text{-trans}}$-29-Oxo = Verbindungen der Formel X]

oder

β) 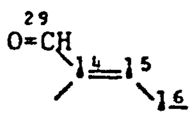 [= $\Delta^{14,15\text{-cis}}$-29-Oxo = Verbindungen der Formel X]

steht und X und $R_2$ die unter Formel I angegebenen Bedeutungen haben, sind auf Grund ihrer spezifischen Struktur für die Herstellung der wertvollen Endprodukte der Formel I prädestiniert, da sie die unmittelbare Vorstufe zur Herstellung der Verbindungen der Formel II bilden; sie stellen daher einen weiteren Bestandteil der vorliegenden Erfindung dar.

Als (oxidative) Umlagerungsreagenzien kommen insbesondere Cr-VI-Verbindungen, wie z.B. Pyridiniumdichromat, Pyridiniumchlorochromat, usw., in Frage. Man arbeitet zweckmässigerweise in einem reakionsinerten Lösungsmittel. Geeignete Lösungsmittel sind z.B. Ether und etherartige Verbindungen wie Dialkylether (Diethylether, Diisopropylether, tert.Butylmethylether, Dimethoxiethan), Dioxan, Tetrahydrofuran, Anisol, usw.; halogenierte Kohlenwasserstoffe wie Chlorbenzol, Methylenchlorid, Ethylenchlorid, Chloroform, Tetrachlorkohlenstoff, Tetrachlorethylen, usw.; Sulfoxide wie Dimethylsulfoxid; Amide wie N,N-Dimethylformamid; Ester wie Ethylacetat, Propylacetat, Butylacetat, usw.; sowie Mischungen dieser Lösungsmittel untereinander oder mit Wasser und/oder anderen üblichen

inerten Lösungsmitteln wie Benzol, Xylol, Petrolether, Ligroin, Cyclohexan, usw. In manchen Fällen kann es von Vorteil sein, wenn die Reaktion oder Teilschritte davon unter Schutzgasatmosphäre (z.B. Argon, Helium, Stickstoff, etc.) und/oder in absoluten Lösungsmitteln durchgeführt werden. Gewünschtenfalls können Zwischenprodukte aus dem Reaktionsmedium isoliert und falls erwünscht, vor der Weiterreaktion, auf übliche Art und Weise gereinigt werden, z.B. durch Waschen, Dispergieren, Extraktion, Umkristallisation, Chromatographie usw. Man kann jedoch auch auf derartige Reinigungsschritte verzichten und diese erst mit den entsprechenden Endprodukten durchführen. Bei der oxidativen allylischen Umlagerung beträgt die Reaktionstemperatur normalerweise -50° bis +50°C, vorzugsweise -10° bis +30°C. Die Reaktionszeit hängt im wesentlichen von den Reaktionstemperaturen ab und variiert im allgemeinen zwischen 10 Minuten und ca. 12 Stunden.

Die Ketone der Formel XI

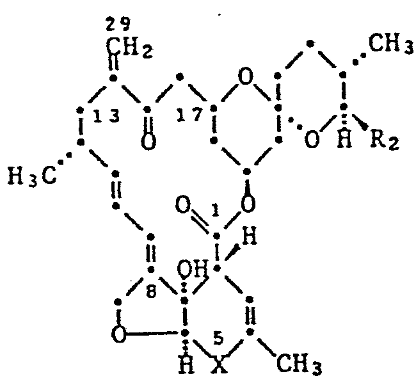

(XI),

worin

X und $R_2$ die unter Formel I angegebenen Bedeutungen haben, sind auf Grund ihres Zwischenprodukte-Charakters, zur Herstellung weiterer Milbemycin-derivate und ihrer eigenen parazitischen Wirkung, ein Bestandteil dieser Erfindung.

2. Teilschritt: Reduktion der 29-Oxo-Verbindung X zur 29-Hydroxi-Verbindung II

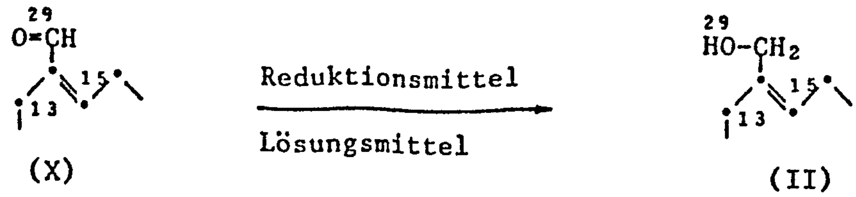


(X) (II) .

Der im eingangs beschriebenen 1. Teilschritt erhaltene Aldehyd X wird in einem geeigneten Lösungsmittel zu 29-Hydroxi-Verbindungen der Formel II reduziert.

Als Reduktionsmittel eignen sich z.B. Hydride wie Lithiumaluminiumhydrid, Natriumborhydrid, Natriumcyanoborhydrid, Lithium-tri-sek.-butyl-borohydrid, Lithium-triethylborohydrid, Lithium-tri-tert.-butoxi-aluminiumhydrid und Verbindungen wie 9-Borobicyclo[3.3.1]-nonan usw. Als Lösungsmittel können, je nach Reduktionsmittel, z.B. Alkohole, insbesondere Alkanole wie Methanol, Ethanol, Propanol, Butanol, etc., aber auch Wasser, Carbonsäuren wie Essigsäure, aromatische Kohlenwasserstoffe wie Benzol, Toluol, Xylole, etc. oder etherartige Substanzen wie Dialkylether, z.B. Diethylether, Diisopropylether, tert.-Butyl-methylether, Dioxan, Tetrahydrofuran und ähnliche eingesetzt werden. Unter Umständen ist es von Vorteil, wenn man einen Katalysator zusetzt, so z.B. eine katalytische Menge einer starken Säure wie Schwefelsäure, Salzsäure, usw. oder eines Cerium-III-Salzes. Die Temperaturen liegen bei dieser Reduktion bei -20° bis +40°C, vorzugsweise 0° bis +30°C.

Die Reduktion der trans-Form von X führt zur trans-Form II und entsprechend die Reduktion der cis-Form von X zur cis-Form II.

Liegt der Aldehyd X in trans-Form vor, und enthält er keine Schutzgruppe (Silylgruppe) am Sauerstoffatom in der 5-Position, so beobachtet man bereits bei Raumtemperatur eine trans-cis-Isomerisierung.

Die intermediär auftretenden 29-Oxo-Verbindungen, die auch isoliert werden können und die allgemeine Formel X aufweisen

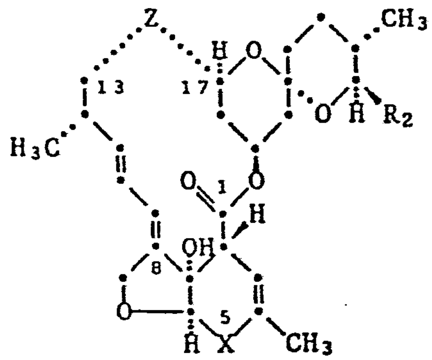

(X),

worin Z für eine der Gruppen

α) 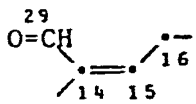

[ = $\Delta^{14,15\text{-trans}}$-29-Oxo
= Verbindungen der Formel III ]

oder

β) 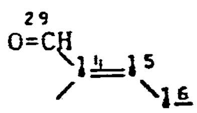

[ = $\Delta^{14,15\text{-cis}}$-29-Oxo
= Verbindungen der Formel III ]

steht und X und $R_2$ die unter Formel I angegebenen Bedeutungen haben, sind nicht nur auf Grund ihrer spezifischen Struktur für die Herstellung der Verbindungen der Formel II prädestiniert, sie zeigen ihrerseits bereits ekto- und endoparasitizide sowie teilweise auch insektizide Wirkung und zwar auf dem gleichen Indikationsgebiet wie die Endprodukte der Formel I.

Die Verbindungen der Formel X sind somit ebenfalls Bestandteil der vorliegenden Erfindung.

Die Ausgangsverbindungen der Formel IX lassen sich durch Singulett-Sauerstoff-Oxidation aus entsprechend substituierten Milbemycin-Derivaten der Formel XII

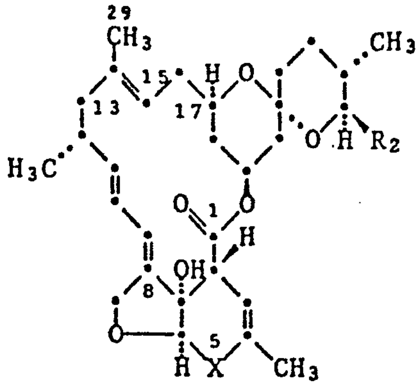

(XII),

worin X und $R_2$ die für Formel I gegebene Bedeutung haben, und anschliessende selektive Reduktion des intermediär entstandenen 15-Peroxids

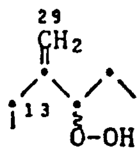

15-Peroxid

mit Natriumborhydrid, Lithiumaluminiumhydrid oder Triphenylphosphin herstellen. Die Reaktion wird bei sichtbarem Licht in Gegenwart eines Sensibilisators bei Normaldruck und bei einer Temperatur von -90°C bis +45°C, vorzugsweise 0°C bis +20°C, in einem inerten Lösungsmittel durchgeführt. Vorzugsweise wird in einer Bestrahlungsapparatur gearbeitet.

Der Reaktionsverlauf lässt sich schematisch so darstellen:

1) Sauerstoff+Licht+ Sensibilisator
2) Reduktion

Verbindung XII ⟶ Verbindung IX

(Vgl. H.H. Wassermann et al. "Singulett Oxygen"; Academic Press, New York 1979; oder B. Ranby et al. "Singulett Oxygen Reactions with Organic Compounds and Polymers", Wiley, New York 1978).

Als Lösungsmittel eignen sich z.B. Ether und etherartige Verbindungen wie Diethylether, Diisopropylether, Dioxan und Tetrahydrofuran; aromatische Kohlenwasserstoffe wie Benzol, Toluol und Xylol; Ketone wie Aceton, Methylethylketon und Cyclohexanon; Nitrile wie Acetonitril; Ester wie Aethylacetat und Butylacetat; sowie Dimethylformamid, Dimethylsulfoxid und halogenierte Kohlenwasserstoffe, oder Mischungen dieser Lösungsmittel mit Wasser.

Als Sensibilisator eignen sich Farbstoffe wie z.B. Methylenblau, Bengalrosa, Chlorophyll, Erysathrosin, Eosin, Zinktetraphenylporphin, Haematoporphyrin, Riboflavin, Fluorescein oder Acridinorange. Ohne weitere Aufarbeitung wird nach Abschluss der Oxydation bei einer Temperatur von 0° bis 20°C selektiv reduziert.

Als Lichtquelle empfiehlt sich eine Lampe mit einer Leistung von 60-500 Watt, bevorzugt 100-350 Watt. Sofern Schutzgruppen für die 5-Hydroxi-Gruppe gewünscht werden, kommen die bereits für $R_1$ genannten Silyl- und Acyl-Gruppen in Frage oder z.B. ein Benzylether, Methoxiethoximetzhylether oder Dihydrofuran- oder Dihydropyran-Reste. Diese Schutzgruppen können in Verbindungen der Formel XII eingeführt und später auf übliche Art wieder abgespalten werden.

Die Verbindungen der Formel XII, worin $R_1$ Wasserstoff bedeutet, sind entweder aus der US-PS 3.950 360 bekannt geworden und wurden ursprünglich als "Antibiotika B-41-A", später als "Milbemycin-A"-Verbindungen bezeichnet, oder sind aus der US-PS 4. 346 171d bekannt und werden als "B-41" oder "Milbemycin-D" bezeichnet oder sind aus der US-PS 4.173.571 bekannt geworden und werden als 13-Deoxi-22,23-dihydro-Avermectin ($R_2$ = sec.Butyl) bezeichnet. Sie besitzen die Struktur der Formel XIII

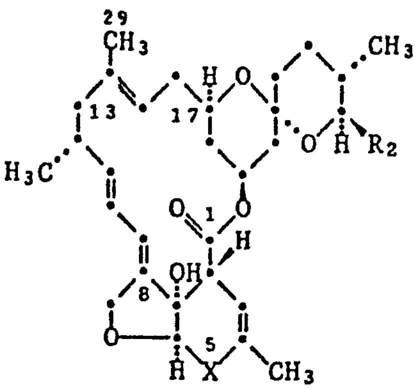

(XIII)

$R_2 = CH_3$ Milbemycin $A_3$

$R_2 = C_2H_5$ Milbemycin $A_4$

$R_2 = isoC_3H_7$ Milbemycin D

$R_2 = sec.C_4H_9$ 13-Deoxi-22,23-dihydro-C-076-Bla-aglycon, oder 13-Deoxi-22,23-dihydro-avermectin-Bla-aglykon.

Die Verbindungen der Formel III lassen sich durch Einführen der abspaltbaren Gruppe $R_x$ aus den zugrundeliegenden 15-Hydroxi-Milbemycinen der Formel IX

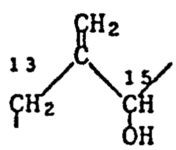

(IX)

herstellen, indem man eine Verbindung der Formel IX durch Umsetzung mit dem Säurerest, wie z.B. einem Säureanhydrid oder Säurehalogenid, vorzugsweise Säurechlorid oder -bromid und in Gegenwart einer der eingangs genannten Basen in einem reaktionsinerten Lösungsmittel verestert. Diese Veresterung wird üblicherweise bei -30° bis 80°C, bevorzugt bei 0 bis 50°C durchgeführt.

Durch Acylierung oder Silylierung der 5-OH-Gruppe werden alle jene Milbemycin-Derivate hergestellt, bei denen $R_1$ eine andere Bedeutung als Wasserstoff ($R_1$ = OH-Schutzgruppe) hat. Zur Silylierung verwendet man zweckmässigerweise ein Silan der Formel $Y-Si(R_6)(R_7)(R_8)$, worin $R_6$, $R_7$ und $R_8$ einen der eingangs genannten

Reste darstellen, und wobei Y eine Silylabgangsgruppe bedeutet. Zu den Silylabgangsgruppen Y zählen beispielsweise Bromid, Chlorid, Cyanid, Azid, Acetamid, Trifluoracetat, Trifluormethansulfonat. Diese Aufzählung stellt keine Limitierung dar, der Fachmann kennt weitere typische Silylabgangsgruppen.

5-O-Silylierungen werden in wasserfreiem Milieu, vorzugsweise in inerten Lösungsmitteln und besonders bevorzugt in aprotischen Lösungsmitteln durchgeführt. Die Reaktion läuft vorteilhaft im Temperaturbereich von 0° bis +80°C, bevorzugt bei +10° bis +40°C, ab. Vorzugsweise wird eine organische Base zugegeben. Es kommen als solche beispielsweise tertiäre Amine wie Triethylamin, Triethylendiamin, Triazol und bevorzugt Pyridin, Imidazol oder 1,8-Diazabicyclo[5.4.0]-undec-7-en (DBU) in Frage.

Die Entfernung dieser Silylreste $R_1$ in der 5-Position geschieht durch selektive milde Hydrolyse ($\longrightarrow R_1=H$) mit z.B. Arylsulfonsäure in alkoholischer Lösung oder nach einer anderen dem Fachmann geläufigen Methode.

Das beschriebene Verfahren zur Herstellung der Verbindungen der Formel I ist in allen Teilschritten ein Bestandteil vorliegender Erfindung.

Die Verbindungen der Formel I eignen sich ausgezeichnet zur Bekämpfung von Schädlingen an Tieren und Pflanzen, darunter insbesondere von tierparasitären Ekto-Parasiten. Zu letzteren zählen unter der Ordnung Acarina insbesondere Schädlinge der Familien Ixodidae, Dermanyssidae, Sarcoptidae, Psoroptidae; die Ordnungen Mallophaga; Siphonaptera, Anoplura (z.B. Familie der Haemotopinidae); unter der Ordnung Diptera insbesondere Schädlinge der Familien Muscidae, Calliphoridae, Oestridae, Tabanidae, Hippoboscidae, Gastrophilidae.

Die Verbindungen I sind auch einsetzbar gegen Hygiene-Schädlinge, insbesondere der Ordnungen Diptera mit den Familien Sarcophagidae, Anophilidae, Culicidae; der Ordnung Orthoptera, der Ordnung Dictyoptera (z.B. Familie Blattidae) und der Ordnung Hymenoptera (z.B. Familie Formicidae).

Die Verbindungen I besitzen auch nachhaltige Wirksamkeit bei pflanzenparasitären Milben und Insekten. Bei Spinnmilben der Ordnung Acarina sind sie wirksam gegen Eier, Nymphen und Adulte von Tetranychidae (Tetranychus spp. und Panonychus spp.).

Hohe Aktivität besitzen sie bei den saugenden Insekten der Ordnung Homoptera, insbesondere gegen Schädlinge der Familien Aphididae, Delphacidae, Cicadellidae, Psyllidae, Loccidae, Diaspididae und Eriophydidae (z.B. die Rostmilbe auf Citrusfrüchten): Der Ordnungen Hemiptera; Heteroptera und Thysanoptera; sowie bei den pflanzenfressenden Insekten der Ordnungen Lepidoptera; Coleoptera; Diptera und Orthoptera.

Sie sind ebenfalls als Bodeninsektizid gegen Schädlinge im Erdboden geeignet.

Die Verbindungen der Formel I sind daher gegen alle Entwicklungsstadien saugender und fressender Insekten an Kulturen wie Getreide, Baumwolle, Reis, Mais, Soja, Kartoffeln, Gemüse, Früchten, Tabak, Hopfen, Citrus, Avocados und anderen wirksam.

Die Verbindungen der Formel I sind auch wirksam gegen Pflanzen-Nematoden der Arten Meloidogyne, Heterodera, Pratylenchus, Ditylenchus, Radopholus, Rizoglyphus und andere.

Ferner sind die Verbindungen gegen Helminthen wirksam, unter denen die endoparasitären Nematoden die Ursache schwerer Erkrankungen an Säugetieren und Geflügel sein können, z.B. an Schafen, Schweinen, Ziegen, Rindern, Pferden, Eseln, Hunden, Katzen, Meerschweinchen, Ziervögeln. Typische Nematoden dieser Indikation sind: Haemonchus,

Trichostrongylus, Ostertagia, Nematodirus, Cooperia, Ascaris, Bunostonum, Oesophagostonum, Charbertia, Trichuris, Strongylus, Trichonema, Dictyocaulus, Capillaria, Heterakis, Toxocara, Ascaridia, Oxyuris, Ancylostoma, Uncinaria, Toxascaris und Parascaris. Der besondere Vorteil der Verbindungen der Formel I ist ihre Wirksamkeit gegen solche Parasiten, die gegen Wirkstoffe auf Benzimidazol-Basis resistent sind.

Gewisse Spezies der Arten Nematodirus, Cooperia und Oesophagostomum greifen den Intestinaltrakt des Wirtstiers an, während andere der Arten Haemonchus und Ostertagia im Magen und solche der Art Dictyocaulus im Lungengewebe parasitieren. Parasiten der Familien Filariidae und Setariidae finden sich im internen Zellgewebe und den Organen, z.B. dem Herzen, den Blutgefässen, den Lymphgefässen und dem subcutanen Gewebe. Hier ist vor allem der Herzwurm des Hundes, Dirofilaria immitis, zu nennen. Die Verbindungen der Formel I sind gegen diese Parasiten hoch wirksam.

Weiter sind die Verbindungen der Formel I zur Bekämpfung von humanpathogenen Parasiten geeignet, unter denen als typische, im Verdauungstrakt vorkommende Vertreter solche der Arten Ancylostoma, Necator, Ascaris, Strongyloides, Trichinella, Capillaria, Trichuris und Enterobius zu nennen sind. Wirksam sind die Verbindungen vorliegender Erfindung auch gegen Parasiten der Arten Wuchereria, Brugia, Onchocerca und Loa aus der Familie der Filariidae, die im Blut, im Gewebe und verschiedenen Organen vorkommen, ferner gegen Dracunculus und Parasiten der Arten Strongyloides und Trichinella, die speziell den Gastro-Intestinalkanal infizieren.

Die Verbindungen der Formel I werden in unveränderter Form oder vorzugsweise zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen,

Vernebeln, Verstäuben, Verstreuen oder Giessen werden gleich wie die Art der Mittel den angestrebten Zielen und den gegebenen Verhältnissen entsprechend gewählt.

Die Verbindungen der Formel I werden bei Warmblütern in Aufwandmengen von 0,01 bis 10 mg/kg Körpergewicht dosiert. Ueber geschlossenen Kultur-Anbauflächen in Mengen von 10 g bis 1000 g pro Hektar angewendet. Sie kommen ferner in Pferchen, Gehegen, Stallungen oder sonstigen Räumen zur Anwendung.

Die Formulierungen, d.h. die den Wirkstoff der Formel I enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder -ethylether, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüber

hinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes nichtionogene, kation-und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können, genannt. Ferner sind auch die Fettsäure-methyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2-Sulfonsäuregruppen und einen Fettsäurerest mit 8 bis 22 C-Atomen. Alkylarylsulfonate sind

z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxid-Adduktes oder Phospholipide in Frage.

Die in der Formulierungstechnik gebräuchlichen Tenside sind u.a. in folgender Publikation beschrieben:

"Mc Cutcheon's Detergents and Emulsifiers Annual"
MC Publishing Corp., Ridgewood, New Jersey, 1982.

Die pestiziden Zubereitungen enthalten in der Regel 0,01 bis 95 %, insbesondere 0,1 bis 80 %, Wirkstoff der Formel I, 5 bis 99,99 % eines festen oder flüssigen Zusatzstoffes und 0 bis 25 %, insbesondere 0,1 bis 25 %, eines Tensides.

Während als Handelsware eher konzentrierte Mittel bevorzugt werden, verwendet der Endverbraucher in der Regel verdünnte Mittel mit 1-10'000 ppm Wirkstoffgehalt.

Ein weiterer Gegenstand vorliegender Erfindung betrifft daher Schädlingsbekämpfungsmittel, die neben üblichen Trägerstoffen und/oder Verteilungsmitteln als Wirkstoff mindestens eine Verbindung der Formel I enthalten.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

## Herstellungsbeispiele

## Herstellung von Ausgangs- und Zwischenprodukten

### Beispiel A1: Herstellung der Verbindungen $\Delta^{14,29}$-15-Hydroxi-milbemycin D (Formel IX) und 14-Hydroxi-$\Delta^{15,16}$-milbemycin D aus Milbemycin D

Eine Lösung von 5,56 g Milbemycin D und 0,03 g Methylenblau in 400 ml Acetonitril wird in einer Bestrahlungsapparatur aus Glas unter Sauerstoffzugabe während 10 Stunden bei einer Temperatur von 20°C (Projektorlampe 200 W) mit sichtbarem Licht bestrahlt.

Anschliessend wird im Reaktionsgemisch bei einer Temperatur von 20°C mit 3,9 g Triphenylphosphin reduziert.

Das Reaktionsgemisch wird nach dem Einengen über einer Silicagelkolonne mit Methylenchlorid/Essigester = 3:1 als Eluiermittel chromatographiert.

Man erhält 4,10 g $\Delta^{14,29}$-15-Hydroxi-Milbemycin D, Smp. 228-229°C; Massenspektrum m/e: 572 ($M^+$), 554.

Daneben erhält man 0,34 g 14-Hydroxi-$\Delta^{15,16}$-milbemycin D, Smp. 252-254°C; Massenspektrum m/e: 572 ($M^+$), 554.

### Beispiel A2: Herstellung der Verbindungen 5-Keto-$\Delta^{14,29}$-15-hydroxi-milbemycin D (Formel IX) und 5-Keto-14-hydroxi-$\Delta^{15,16}$-milbemycin D aus 5-Keto-milbemycin D

a) Herstellung von 5-Keto-milbemycin D

1 g Milbemycin D, 2 g aktiviertes Mangandioxid und 50 ml wasserfreies Methylenchlorid werden bei 20-25°C 4 Stunden gerührt. Die Reaktionsmischung wird filtriert und das Filtrat wird über eine

kurze (ca. 30 cm) Schicht Silicagel gereinigt. Man erhält 1 g 5-Keto-Milbemycin in Form einer gelblichen amorphen Masse, Smp. 152-157°C.

b) Die Singulett-Oxydation des unter a) hergestellten 5-Keto-Milbemycins und die weitere Aufarbeitung erfolgt nach der im Beispiel 1 angegebenen Methode.

Man erhält nach Chromatograpie an Silicagel 0,6 g 5-Keto-$\Delta^{14,29}$-15-hydroxi-milbemycin D, Smp. 160-165°C;
Massenspektrum m/e: 570 ($M^+$), 552.

Daneben erhält man 30 mg 5-Keto-14-hydroxi-$\Delta^{15,16}$-milbemycin D, Smp. 170-174°C.

Beispiel A3: Herstellung von 5-Keto-$\Delta^{14,29}$-15-hydroxi-milbemycin D (Formel IX) und 5-Keto-14-hydroxi-$\Delta^{15,16}$-milbemycin D aus Milbemycin D

Die Mangandioxid-Oxidation als Folgeschritt mit den nach Beispiel 1 durch Singulett-Sauerstoff-Oxidation gewonnenen Verbindungen $\Delta^{14,29}$-15-Hydroxi-milbemycin D und 14-Hydroxi-$\Delta^{15,16}$-milbemycin D liefert in quantitativer Ausbeute 5-Keto-$\Delta^{14,29}$-15-hydroxi-milbemycin D und 5-Keto-14-hydroxi-$\Delta^{15,16}$-milbemycin D.

Beispiel A4: Herstellung von 5-Acetyloxi-$\Delta^{14,29}$-15-hydroxi-milbemycin D (Formel IX) und von 5-Acetyloxi-14-hydroxi-$\Delta^{15,16}$-milbemycin D aus Milbemycin D

a) Herstellung von 5-Acetyloxi-milbemycin D

560 mg (1,0 mMol) Milbemycin D werden in 20 ml Pyridin mit 160 mg (1,6 mMol) Acetanhydrid versetzt und bei Raumtemperatur über Nacht gerührt. Nach dem Eindampfen des Pyridins wird der Rückstand in 20 ml Ethylacetat aufgenommen und die organische Phase einmal mit 10 ml 1N Chlorwasserstoffsäure-Lösung dann mit 10 ml gesättigter $NaHCO_3$-Lösung und zum Schluss mit 10 ml gesättigter NaCl-Lösung geschüttelt. Nach dem Abtrennen der organischen Phase wird diese

über $Na_2SO_4$ getrocknet, filtriert und eingeengt. Man erhält 580 mg 5-Acetyloxi-milbemycin D (amorphes, schwachgelbes Pulver), Smp. 115-120°C.

Auf analoge Art lassen sich auch die Acyl-Derivate Milbemycine $A_3$, $A_4$ und das 13-Desoxi-avermectin-Derivat ($R_2$ = sek.Butyl) gewinnen.

b) 560 mg 5-Acetyloxi-milbemycin D werden mit 20 mg Methylenblau in 40 ml Acetonitril mit einer Bestrahlungsapparatur (Projektlampe 200 W) während 8 Stunden bei 18-22°C mit Sauerstoff behandelt. Das Reaktionsgemisch wird anschliessend bei Raumtemperatur mit 40 mg Triphenylphosphin reduziert.

Nach dem Einengen wird das Reaktionsgemisch über einer Silicasäule (Laufmittel Methylenchlorid/Ethylacetat 3:1) chromatographiert.

Man erhält 390 mg 5-Acetyloxi-$\Delta^{14,29}$-15-hydroxi-milbemycin D, Smp. 153-156°C;
Massenspektrum m/e: 614 ($M^+$), 596.

Daneben erhält man 42 mg 5-Acetyloxi-14-hydroxi-$\Delta^{15,16}$-milbemycin D, Smp. 151-154°C.

Beispiel A5: Herstellung von $\Delta^{14,29}$-15-Hydroxi-milbemycin $A_4$ (Formel IX) und 14 Hydroxi-$\Delta^{15,16}$-milbemycin $A_4$ aus Milbemycin $A_4$

540 mg (1 mMol) Milbemycin $A_4$ werden in 100 ml Acetonitril analog Beispiel 1 mit Singulett-Sauerstoff oxidiert und anschliessend mit Triphenylphosphin reduziert. Nach Reinigung durch Flash-Chromatographie an Silicagel (Laufmittel Cyclohexan/Ethylacetat 1:1) erhält man 310 mg $\Delta^{14,29}$-15-hydroximilbemycin $A_4$
Smp. 222-225°C;
Massenspektrum m/e: 558 ($M^+$), 540.

Daneben erhält man 40 mg 14-Hydroxi-$\Delta^{15,16}$-milbemycin $A_4$, Smp. 147-152°C; Massenspektrum m/e: 558 ($M^+$), 540.

Beispiel A6: Herstellung von 5-Dimethyl-tert.butylsilyloxi-$\Delta^{14,29}$-15-hydroxi-milbemycin $A_3$ (Formel IX) und von 5-Dimethyl-tert.-butylsilyloxi-14-hydroxi-$\Delta^{14,15}$-milbemycin $A_3$ aus Milbemycin $A_3$

a) Herstellung von 5-Dimethyl-tert.butylsilyloxi-milbemycin $A_3$.
Bei Raumtemperatur werden 480 mg (7 mMol) Imidazol und 460 mg (3 mMol) Dimethyl-tert.butylchlorsilan in 20 ml Methylenchlorid vorgelegt. Unter Rühren tropft man langsam eine Lösung von 655 mg (1,2 mMol) Milbemycin $A_3$ in 10 ml Methylenchlorid dazu. Anschliessend wird das Reaktionsgemisch über Nacht unter Rückfluss (~ 40°C) erwärmt. Nach dem Einengen, Reinigen über Silicagel und Trocknen erhält man 730 mg amorphes 5-Dimethyl-tert.butylsilyl-milbemycin $A_3$, Smp. 55-60°C.

Auf gleiche Weise lassen sich auch die Milbemycine $A_4$, D und das 13-Desoxi-Avemectin-Derivat ($R_2$ = sek.Butyl) silylieren, wobei auch vorteilhaft Methyldiphenylchlorsilan oder bis(Isopropyl)methylchlorsilan eingesetzt werden können.

b) Nach der Vorschrift des Beispiels A4b) werden aus 720 mg 5-Dimethyl-tert.butylsilyl-milbemycin $A_3$ durch Singulett-Sauerstoff-Oxidation mit Bengalrosa als Sensibilisator und anschliessende Reduktion der Peroxide mit Triphenylphosphin 550 mg 5-Dimethyl-tert.butylsilyloxi-$\Delta^{14,29}$-15-hydroxi-milbemycin $A_3$ gewonnen,
Smp. 238-240°C;
Massenspektrum m/e: 658 ($M^+$), 640.

Daneben werden 42 mg 5-Dimethyl-tert.butylsilyloxi-14-hydroxi-$\Delta^{15,16}$-milbemycin $A_3$ in amorpher Form erhalten. Smp. 45-50°C.

Beispiel A7: Herstellung von $\Delta^{14,29}$-15-Hydroxi-milbemycin $A_3$ (Formel IX) und von 14-Hydroxi-$\Delta^{15,16}$-milbemycin $A_3$
120 mg 5-Dimethyl-tert.butylsilyloxi-$\Delta^{14,29}$-15-hydroxi-milbemycin $A_3$ werden mit 2 ml einer 1%igen Lösung von p-Toluolsulfonsäure in Methanol 9 Stunden bei Raumtemperatur gerührt und dann mit 5%iger wässriger $NaHCO_3$-Lösung behandelt. Durch Ausschütteln mit dreimal

2 ml Diethylether, Einengen der organischen Phase und Chromatographie des Rohprodukts an 20 g Kieselgel (Laufmittel Aceton/-Methylenchlorid 1:12) erhält man 67 mg $\Delta^{14,29}$-15-Hydroxi-milbemycin $A_3$, Smp. 219-222°C.

Entsprechend erhält man aus 60 mg 5-Dimethyl-tert.butylsilyloxi-14-hydroxi-$\Delta^{15,16}$-milbemycin $A_3$ 38 mg 14-Hydroxi-$\Delta^{15,16}$-milbemycin $A_3$, Smp. 128-132°C.

Beispiel A8: Herstellung von 29-Oxo-5-acetyloxi-$\Delta^{14,15\text{-trans}}$-milbemycin D (Formel X) und von 15-Oxo-5-acetyloxi-$\Delta^{14,29}$-milbemycin D (Formel XI)

600 mg 15-Hydroxi-5-acetyloxi-$\Delta^{14,29}$-milbemycin D werden bei 10°C in 35 ml absolutem Dimethylformamid mit 570 mg Pyridiniumdichromat versetzt und 2 Stunden bei Raumtemperatur kräftig gerührt. Das Lösungsmittel wird im Hochvakuum entfernt und das resultierende Harz in Diethylether aufgeschlämmt und filtriert. Die flüssige Phase wird mit Wasser und gesättigter Kochsalzlösung gewaschen, über Natriumsulfat getrocknet und filtriert. Das gelbliche Rohprodukt wird säulenchromatographisch (Kieselgel: Methylenchlorid/Diethylether 15:1) gereinigt und führt zu 330 mg 29-Oxo-5-acetyloxi-$\Delta^{14,15}$-milbemycin D mit einem Schmelzpunkt von 155-159°C sowie als Nebenprodukt zu 200 mg 15-Oxo-5-acetyloxi-$\Delta^{14,29}$-milbemycin D mit Schmelzpunkt 139-142°C.

Beispiel A9:
Herstellung von 29-Oxo-$\Delta^{14,15\text{cis}}$-milbemycin D (Formel X) und von 29-Oxo-$\Delta^{14,15\text{trans}}$-milbemycin D (Formel X)

136 mg 29-Oxo-$\Delta^{14,15\text{trans}}$-5-dimethyl-tert.butyl-silyloxi-milbemycin D werden bei Raumtemperatur in 15 ml Methanol gelöst, mit 2 ml p-Toluolsulfonsäure versetzt und 1 Stunde gerührt. Anschliessend wird das Lösungsmittel im Hochvakuum entfernt und das Rohprodukt säulenchromatographisch gereinigt (Kieselgel: Methylenchlorid/Diethylether 3:1) Man erhält 125 mg des trans-Produktes in Form eines weissen amorphen Pulvers. Smp. ca. 150°C. Die Hälfte dieses trans-Produktes wird in Methanol gelöst, mit einigen Tropfen

verdünnter Schwefelsäure versetzt und 3 Stunden bei ca. 30°C gerührt. Dann wird das Lösungsmittel im Hochvakuum entfernt, das Rohprodukt in Methylenchlorid gelöst und durch eine kurze (5 cm lange) Silicagelsäule filtriert. Man erhält auf diese Weise 60 mg des stabileren Cisproduktes, das sich bei ca. 250°C zersetzt.

Beispiel A10:

a) Herstellung von 29-Hydroxi-$\Delta^{14,15cis}$-milbemycin D (Formel II)

95 mg 29-Oxo-$\Delta^{14,15cis}$-milbemycin D werden in 5 ml Methanol gelöst und bei Raumtemperatur unter Rühren mit 10 mg Natriumborhydrid versetzt. Nach 30 Minuten werden 30 ml Methylenchlorid zugesetzt. Die Lösung wird mit 30 ml Wasser gewaschen, über Natriumsulfat getrocknet und eingedampft. Das Rohprodukt wird säulenchromatographisch (Kieselgel: Methylenchorid/Diethylether 2:1) gereinigt. Ausbeute 85 mg. Smp. 160-165°C.

b) Herstellung von 29-Hydroxi-$\Delta^{14,15trans}$-milbemycin D (Formel II)

136 mg 29-Oxo-5-dimethyl-tert.butyl-silyloxi-milbemycin D werden bei Raumtemperatur in 10 ml Methanol mit 14 mg Natriumborhydrid versetzt. Die Aufarbeitung gemäss Beispiel A8 liefert 120 mg 29-Hydroxi-$\Delta^{14,15trans}$-5-dimethyl-tert.butyl-silyloxi-milbemycin D. Smp. 140-145°C.

100 mg dieses Silylderivats werden bei Raumtemperatur in 5 ml Methanol gelöst und mit 2 mg p-Toluolsulfonsäure versetzt. Nach 30 Minuten wird das Lösungsmittel im Hochvakuum entfernt und der Rückstand mit Methylenchlorid/Diethylethersäulenchromatographisch (Kieselgel) gereinigt. Man erhält 65 mg 29-Hydroxi-$\Delta^{14,15trans}$-milbemycin D. Smp. 153-158°C.

Beispiel A11: Herstellung von 15-Mesyloxi-$\Delta^{14,29}$-5-dimethyl-tert.butyl-silyloxi-milbemycin-$A_4$

Zu einer Lösung von 670 mg (1 mmol) 15-Hydroxi-$\Delta^{14,29}$-5-dimethyl-tert.butyl-silyloxi-milbemycin-$A_4$ und 405 mg (4 mmol) Triäthylamin in 40 ml trockenem Tetrahydrofuran werden unter Argon bei ca. -10°C

230 mg (2 mmol) Methansulfochlorid in 2 ml Tetrahydrofuran zugegeben. Unter gutem Rühren wird die Lösung langsam auf ca. +10°C erwärmt und 30 Minuten weitergerührt. Dieses in situ hergestellte Zwischenprodukt kann ohne Reinigungsschritt weiterverarbeitet werden.

Beispiel A12: Herstellung von 29-Hydroxi-5-dimethyl-tert.butylsilyloxi-milbemycin-$A_4$

Das im vorigen Beispiel Nr. A11 hergestellte $A_4$-Derivat in der Tetrahydrofuranreaktionsmischung wird bei ca. 10°C mit etwa 5.4 ml (300 mmol) Wasser versetzt und über Nacht bei Raumtemperatur gerührt.

Nach Zugabe von 200 ml Essigester wird mit gesättigter Kochsalzlösung ausgeschüttelt und dann über einem geeigneten Trocknungsmittel wie z.B. Natriumsulfat getrocknet. Anschliessend wird das Lösungsmittel im Vakuum entfernt. Die Reinigung mittels einer Silikagel-Kolonne (Methylenchlorid : Diethylether = 20:1) ergibt nach der Gefriertrocknung 470 mg der Titelsubstanz, welche bei 142-145°C schmilzt.

Beispiel A13: Herstellung von 29-Hydroxi-milbemycin-$A_4$

Wird im vorherigen Beispiel Nr. A12 vor oder nach der Wasserzugabe das Reaktionsgemisch mit p-Toluolsulfonsäure oder Methansulfonsäure angesäuert, erhält man die Titelverbindung, welche nach der Gefriertrocknung bei 143-147°C schmilzt.

Beispiel A14: Herstellung von 5,15-Bisacetyloxi-$\Delta^{14,29}$-milbemycin D

Zu einer Lösung von 290 mg (0.5 mmol) 15-Hydroxi-$\Delta^{14,29}$-milbemycin D und 100 mg (1.26 mmol) Pyridin in 30 ml Methylenchlorid werden bei ca. 10°C unter gutem Rühren 100 mg (1.27 mmol) Acetylchlorid in 3 ml Methylenchlorid zugegeben und anschliessend über Nacht bei ca. 35°C ausgerührt. Es wird mit 100 ml Essigester verdünnt, mit 0.5 N

Salzsäure und dann mit Kochsalzlösung ausgeschüttelt. Die Reinigung mittels einer Silikagelkolonne (Methylenchlorid : Ether = 2:1) ergibt 275 mg weisses Pulver, welches bei 227-231°C zersetzt.

$^1$H-NMR (250 MHz, $CDCl_3$):

3,03 ppm (br.d.; 8 Hz) ($C_{25}$-H);

4,96 ppm (AB-System);

2,16 ppm und 2,20 ppm (2 Acetyl);

Massenspektrum (FD) m/e 656 ($M^+$, $C_{37}H_{52}O_{10}$).

## Herstellung der Endprodukte

### Beispiel E1: Herstellung von 29-Acetoxi-milbemycin-$A_4$

Zu einer Lösung von 150 mg (0.22 mmol) 15-Hydroxi-$\Delta^{14,29}$-5-dimethyl-tert.butyl-silyloxi-milbemycin-$A_4$, 89 mg (0.88 mmol) Triethylamin und 1 mg p-Dimethylaminopyridin in 30 ml trockenem Tetrahydrofuran werden unter Stickstoff bei ca. -10°C 76 mg (0.44 mmol) Methansulfonsäureanhydrid zugegeben. Unter gutem Rühren wird die Lösung langsam auf Raumtemperatur erwärmt und ca. eine halbe Stunde weitergerührt. Nun werden 2.64 g (44 mmol) Eisessig beigegeben und für 24 Stunden bei 60°C gerührt.

Nach der üblichen Aufarbeitung erhält man 35 mg der Titelsubstanz als weisses amorphes Pulver, welches bei 73 bis 76°C schmilzt.

$^1$H-NMR (300 MHz, $CDCl_3$):

5,22 ppm (dd; 5 und 10 Hz) $C_{15}$-H);

3,05 ppm (dt; 3 und 10 Hz) $C_{25}$-H);

4,51 ppm (AB-System) ($C_{29}$-$H_2$);

Massenspektrum (FD) m/e 600 ($M^+$, $C_{34}H_{48}O_9$).

### Beispiel E2: Herstellung von 29-Cyclopropancarbonyloxi-milbemycin-$A_4$:

Eine Lösung aus 300 mg (0.45 mmol) 29-Hydroxi-5-dimethyl-tert.-butyl-silyloxi-milbemycin-$A_4$, 95 mg (0.9 mmol) Cyclopropansäurechlorid, 180 mg (1.8 mmol) Triethylamin und ca. 5 mg p-Dimethylaminopyridin in 30 ml trockenem Tetrahydrofuran wird während 2 Stunden unter Rückfluss gerührt. Das Lösungsmittel wird abgedampft, der Rückstand in 100 ml Essigester aufgenommen und mit Wasser extrahiert. Nach dem Trocknen über Natriumsulfat wird die organische Phase abdestilliert. Das erhaltene rohe 29-Cyclopropanoyl-5-O-(dimethyl-tert.butyl-silyloxi)-milbemycin-$A_4$ wird in 20 ml Methanol mit 1 % p-Toluolsulfonsäure aufgenommen und bei Raumtemperatur während einer Stunde gerührt. Nach dem Abdestillieren des Lösungmittels wird mittels einer Silika-Kolonne (Diethylether: Hexan = 2:1) gereinigt.
Man erhält nach der Gefriertrocknung 188 mg 29-Cyclopropanoyl-milbemycin-$A_4$ als weisses amorphes Pulver, welches bei 103-107°C schmilzt.
$^1$H-NMR (300 MHz, $CDCl_3$):
5,12 ppm (dd; 5 und 10 Hz) ($C_{15}$-H);
3,05 ppm (dt; 2 und 10 Hz) ($C_{25}$-H);
4,52 ppm (AB-System) ($C_{29}$-$H_2$);
Massenspektrum (FD) m/e 626 ($C_{36}H_{50}O_9$).

Beispiel E3: Herstellung von 29-tert.Butylcarbonyloxi-milbemycin-D:
Eine Lösung aus 690 mg (1 mmol) 29-Hydroxi-5-dimethyl-tert.-butyl-silyloxi-milbemycin-D, 560 mg (3 mmol) Pivaloylanhydrid, ca. 5 mg p-Dimethylaminopyridin in 30 ml Pyridin wird während 2 Stunden bei 90°C gerührt. Nach dem Abdestillieren des Lösungsmittels wird der Rückstand in 20 ml Methanol aufgenommen und mit 1 % p-Toluolsulfonsäure aufgenommen und während einer Stunde bei Raumtemperatur gerührt. Das Lösungsmittel wird abdestilliert und das erhaltene rohe Produkt mittels Silika-Säulenchromatographie (Methylenchlorid: Diethylether: = 4:1) gereinigt.
Man erhält 605 mg der Titelsubstanz als weisses amorphes Pulver, welches bei 75-80°C schmilzt.
$^1$H-NMR (300 MHz, $CDCl_3$):
5,10 ppm (dd; 3 und 11 Hz) ($C_{15}$-H);
2,94 ppm (dd; 1 und 8 Hz) ($C_{25}$-H);
4,42 ppm (AB-System) ($C_{29}$-$H_2$);
Massenspektrum (FD) m/e 656 ($C_{38}H_{56}O_9$).

0237482

<u>Beispiel E4:</u> <u>Herstellung von 5-Oximino-29-tert.butylcarbonyloxi-milbemycin-$A_4$:</u>

Eine Lösung aus 220 mg (0,34 mmol) 5-Keto-29-tert.butylcarbonyloxi-milbemycin-$A_4$, 30 ml Methanol, 10 ml Tetrahydrofuran und 300 mg (4,3 mmol) Hydroxylaminhydrochlorid, unter Zugabe von 2,0 g Molekularsieb, wird über Nacht bei Raumtemperatur gut gerührt. Nach der Filtration wird das Lösungsmittel unter Vakuum abgedampft und der Rückstand mittels Säulenchromatographie (Silikagel, Methylenchlorid: Diethylether = 10 :1) gereinigt. Man erhält so 192 mg der amorphen Titelsubstanz, welche nach der Gefriertrocknung bei 145-150°C schmilzt.

Analog zu den beschriebenen Arbeitsweisen werden auch die nachfolgend genannten Verbindungen hergestellt:

Tabelle 1: Typische Vertreter von Zwischenprodukten der Formel II

[ = $\Delta^{14,15trans}$-29-Hydroxi ]

| Verb. Nr. | $R_2$ | X | Smp. [°C] |
|---|---|---|---|
| 1.1 | $CH_3$ | -C(O)- | |
| 1.2. | $C_2H_5$ | -C(O)- | |
| 1.3. | $C_3H_7$-iso | -C(O)- | |
| 1.4. | $C_4H_9$-sek | -C(O)- | |
| 1.5. | $CH_3$ | -C(OH)- | |
| 1.6. | $C_2H_5$ | -C(OH)- | 143-147 |
| 1.7. | $C_3H_7$-iso | -C(OH)- | 153-158 |
| 1.8. | $C_4H_9$-sek | -C(OH)- | |
| 1.9. | $CH_3$ | -C[OSi($CH_3$)$_2$$C_4H_9$-tert] | |
| 1.10 | $C_2H_5$ | -C[OSi($CH_3$)$_2$$C_4H_9$-tert] | 142-145 |
| 1.11 | $C_3H_7$-iso | -C[OSi($CH_3$)$_2$$C_4H_9$-tert] | 140-145 |
| 1.12 | $C_4H_9$-sek | -C[OSi($CH_3$)$_2$$C_4H_9$-tert] | |
| 1.13 | $CH_3$ | -C[OC(O)$CH_3$] | |
| 1.14 | $C_2H_5$ | -C[OC(O)$CH_3$] | |
| 1.15 | $C_3H_7$-iso | -C[OC(O)$CH_3$] | 144-147 |
| 1.16 | $C_4H_9$-tert | -C[OC(O)$CH_3$] | |

Diese Auflistung hat keinen limitierenden Charakter

Tabelle 2: Typische Vertreter von Zwischenprodukten der Formel II

[ = $\Delta^{14,15cis}$-29-Hydroxi ]

| Verb. Nr. | $R_2$ | X | Smp. [°C] |
|---|---|---|---|
| 2.1 | $CH_3$ | -C(O)- | |
| 2.2. | $C_2H_5$ | -C(O)- | |
| 2.3. | $C_3H_7$-iso | -C(O)- | |
| 2.4. | $C_4H_9$-sek | -C(O)- | |
| 2.5. | $CH_3$ | -C(OH)- | |
| 2.6. | $C_2H_5$ | -C(OH)- | |
| 2.7. | $C_3H_7$-iso | -C(OH)- | 160-165 |
| 2.8. | $C_4H_9$-sek | -C(OH)- | |
| 2.9. | $CH_3$ | -C[OSi($CH_3$)$_2$$C_4H_9$-tert] | |
| 2.10 | $C_2H_5$ | -C[OSi($CH_3$)$_2$$C_4H_9$-tert] | |
| 2.11 | $C_3H_7$-iso | -C[OSi($CH_3$)$_2$$C_4H_9$-tert] | |
| 2.12 | $C_4H_9$-sek | -C[OSi($CH_3$)$_2$$C_4H_9$-tert] | |
| 2.13 | $CH_3$ | -C[OC(O)$CH_3$] | |
| 2.14 | $C_2H_5$ | -C[OC(O)$CH_3$] | |
| 2.15 | $C_3H_7$-iso | -C[OC(O)$CH_3$] | |
| 2.16 | $C_4H_9$-tert | -C[OC(O)$CH_3$] | |

Diese Auflistung hat keinen limitierenden Charakter

Tabelle 3: Typische Vertreter von Zwischenprodukten der Formel X

[ = $\Delta^{14,15trans}$-29-Oxo ]

| Verb. Nr. | $R_2$ | X | Smp. [°C] |
|---|---|---|---|
| 3.1 | $CH_3$ | -C(O)- | |
| 3.2. | $C_2H_5$ | -C(O)- | 115-118 |
| 3.3. | $C_3H_7$-iso | -C(O)- | 120-125 |
| 3.4. | $C_4H_9$-sek | -C(O)- | |
| 3.5. | $CH_3$ | -C(OH)- | |
| 3.6. | $C_2H_5$ | -C(OH)- | |
| 3.7. | $C_3H_7$-iso | -C(OH)- | 120-124 |
| 3.8. | $C_4H_9$-sek | -C(OH)- | |
| 3.9. | $CH_3$ | -C[OSi($CH_3$)$_2$$C_4H_9$-tert] | |
| 3.10 | $C_2H_5$ | -C[OSi($CH_3$)$_2$$C_4H_9$-tert] | 250-253 |
| 3.11 | $C_3H_7$-iso | -C[OSi($CH_3$)$_2$$C_4H_9$-tert] | 215-220 |
| 3.12 | $C_4H_9$-sek | -C[OSi($CH_3$)$_2$$C_4H_9$-tert] | |
| 3.13 | $CH_3$ | -C[OC(O)$CH_3$] | |
| 3.14 | $C_2H_5$ | -C[OC(O)$CH_3$] | |
| 3.15 | $C_3H_7$-iso | -C[OC(O)$CH_3$] | 155-159 |
| 3.16 | $C_4H_9$-tert | -C[OC(O)$CH_3$] | |

Diese Auflistung hat keinen limitierenden Charakter

Tabelle 4: Typische Vertreter von Zwischenprodukten der Formel X

$$\left[ = \Delta^{14,29cis}\text{-29-Oxo} \right]$$

| Verb. Nr. | $R_2$ | X | Smp. [°C] |
|---|---|---|---|
| 4.1 | $CH_3$ | -C(O)- | |
| 4.2. | $C_2H_5$ | -C(O)- | |
| 4.3. | $C_3H_7$-iso | -C(O)- | |
| 4.4. | $C_4H_9$-sek | -C(O)- | |
| 4.5. | $CH_3$ | -C(OH)- | |
| 4.6. | $C_2H_5$ | -C(OH)- | amorph, ca. 60 |
| 4.7. | $C_3H_7$-iso | -C(OH)- | ab 250 Zer. |
| 4.8. | $C_4H_9$-sek | -C(OH)- | |
| 4.9. | $CH_3$ | -C[OSi($CH_3$)$_2$$C_4H_9$-tert] | |
| 4.10 | $C_2H_5$ | -C[OSi($CH_3$)$_2$$C_4H_9$-tert] | 212-215 |
| 4.11 | $C_3H_7$-iso | -C[OSi($CH_3$)$_2$$C_4H_9$-tert] | 78-83 |
| 4.12 | $C_4H_9$-sek | -C[OSi($CH_3$)$_2$$C_4H_9$-tert] | |
| 4.13 | $CH_3$ | -C[OC(O)$CH_3$] | |
| 4.14 | $C_2H_5$ | -C[OC(O)$CH_3$] | |
| 4.15 | $C_3H_7$-iso | -C[OC(O)$CH_3$] | |
| 4.16 | $C_4H_9$-tert | -C[OC(O)$CH_3$] | |

Diese Auflistung hat keinen limitierenden Charakter

Tabelle 5

Typische Vertreter von Verbindungen der Formel I, worin X für -CH(OH)- steht:

| Verb. Nr. | $R_2$ | R | Physik.Konst. Smp. [°C] |
|---|---|---|---|
| 5.1 | $CH_3$ | H | |
| 5.2 | $C_2H_5$ | H | |
| 5.3 | $C_3H_7$-iso | H | |
| 5.4 | $C_4H_9$-sek | H | |
| 5.5 | $CH_3$ | $CH_3$ | |
| 5.6 | $C_2H_5$ | $CH_3$ | 73-76 |
| 5.7 | $C_3H_7$-iso | $CH_3$ | |
| 5.8 | $C_4H_9$-sek | $CH_3$ | |
| 5.9 | $CH_3$ | $C(CH_3)_3$ | |
| 5.10 | $C_2H_5$ | $C(CH_3)_3$ | 110-115 |
| 5.11 | $C_3H_7$-iso | $C(CH_3)_3$ | 75-80 |
| 5.12 | $C_4H_9$-sek | $C(CH_3)_3$ | |
| 5.13 | $CH_3$ | $CH_3OCH_2$ | |
| 5.14 | $C_2H_5$ | $CH_3OCH_2$ | |
| 5.15 | $C_3H_7$-iso | $CH_3OCH_2$ | |
| 5.16 | $C_4H_9$-sek | $CH_3OCH_2$ | |
| 5.17 | $CH_3$ | $CH_3O(CH_3)_2C$ | |
| 5.18 | $C_2H_5$ | $CH_3O(CH_3)_2C$ | |
| 5.19 | $C_3H_7$-iso | $CH_3O(CH_3)_2C$ | |
| 5.20 | $C_4H_9$-sek | $CH_3O(CH_3)_2C$ | |
| 5.21 | $CH_3$ | $(CH_3)_2CH-CH_2$ | |
| 5.22 | $C_2H_5$ | $(CH_3)_2CH-CH_2$ | 95-100 |
| 5.23 | $C_3H_7$-iso | $(CH_3)_2CH-CH_2$ | |
| 5.24 | $C_4H_9$-sek | $(CH_3)_2CH-CH_2$ | |
| 5.25 | $CH_3$ | $CCl_3$ | |
| 5.26 | $C_2H_5$ | $CCl_3$ | |
| 5.27 | $C_3H_7$-iso | $CCl_3$ | |
| 5.28 | $C_4H_9$-sek | $CCl_3$ | |
| 5.29 | $CH_3$ | $CF_3$ | |
| 5.30 | $C_2H_5$ | $CF_3$ | |
| 5.31 | $C_3H_7$-iso | $CF_3$ | |
| 5.32 | $C_4H_9$-sek | $CF_3$ | |
| 5.33 | $CH_3$ | $Cl_3CCHCl$ | |
| 5.34 | $C_2H_5$ | $Cl_3CCHCl$ | |
| 5.35 | $C_3H_7$-iso | $Cl_3CCHCl$ | |
| 5.36 | $C_4H_9$-sek | $Cl_3CCHCl$ | |
| 5.37 | $CH_3$ | $ClCH_2CH_2CH_2$ | |
| 5.38 | $C_2H_5$ | $ClCH_2CH_2CH_2$ | |

0237482

Tabelle 5 (Fortsetzung)

| Verb. Nr. | $R_2$ | R | Physik.Konst. Smp. [°C] |
|---|---|---|---|
| 5.39 | $C_3H_7$-iso | $ClCH_2CH_2CH_2$ | |
| 5.40 | $C_4H_9$-sek | $ClCH_2CH_2CH_2$ | |
| 5.41 | $CH_3$ | $CH_2$=CH | |
| 5.42 | $C_2H_5$ | $CH_2$=CH | |
| 5.43 | $C_3H_7$-iso | $CH_2$=CH | |
| 5.44 | $C_4H_9$-sek | $CH_2$=CH | |
| 5.45 | $CH_3$ | $CH_2$=CH-$CH_2$ | |
| 5.46 | $C_2H_5$ | $CH_2$=CH-$CH_2$ | |
| 5.47 | $C_3H_7$-iso | $CH_2$=CH-$CH_2$ | |
| 5.48 | $C_4H_9$-sek | $CH_2$=CH-$CH_2$ | |
| 5.49 | $CH_3$ | CH≡C-$CH_2$ | |
| 5.50 | $C_2H_5$ | CH≡C-$CH_2$ | |
| 5.51 | $C_3H_7$-iso | CH≡C-$CH_2$ | |
| 5.52 | $C_4H_9$-sek | CH≡C-$CH_2$ | |
| 5.53 | $CH_3$ | $(CH_3)_2C$=CH | |
| 5.54 | $C_2H_5$ | $(CH_3)_2C$=CH | |
| 5.55 | $C_3H_7$-iso | $(CH_3)_2C$=CH | |
| 5.56 | $C_4H_9$-sek | $(CH_3)_2C$=CH | |
| 5.57 | $CH_3$ | $(Cl)_2C$=C(Cl) | |
| 5.58 | $C_2H_5$ | $(Cl)_2C$=C(Cl) | |
| 5.59 | $C_3H_7$-iso | $(Cl)_2C$=C(Cl) | |
| 5.60 | $C_4H_9$-sek | $(Cl)_2C$=C(Cl) | |
| 5.61 | $CH_3$ | $CF_3CCl_2$ | |
| 5.62 | $C_2H_5$ | $CF_3CCl_2$ | |
| 5.63 | $C_3H_7$-iso | $CF_3CCl_2$ | |
| 5.64 | $C_4H_9$-sek | $CF_3CCl_2$ | |
| 5.65 | $CH_3$ | Cyclopropyl | |
| 5.66 | $C_2H_5$ | Cyclopropyl | 103-108 |
| 5.67 | $C_3H_7$-iso | Cyclopropyl | |
| 5.68 | $C_4H_9$-sek | Cyclopropyl | |
| 5.69 | $CH_3$ | 2,2-Dimethyl-cyclopropyl | |
| 5.70 | $C_2H_5$ | 2,2-Dimethyl-cyclopropyl | |
| 5.71 | $C_3H_7$-iso | 2,2-Dimethyl-cyclopropyl | |
| 5.72 | $C_4H_9$-sek | 2,2-Dimethyl-cyclopropyl | |
| 5.73 | $CH_3$ | 2,2-Dimethyl-3-(2,2-dichlorvinyl)-cyclopropyl | |
| 5.74 | $C_2H_5$ | 2,2-Dimethyl-3-(2,2-dichlorvinyl)-cyclopropyl | |

Tabelle 5 (Fortsetzung)

| Verb. Nr. | $R_2$ | R | Physik.Konst. Smp. [°C] |
|---|---|---|---|
| 5.75 | $C_3H_7$-iso | 2,2-Dimethyl-3-(2,2-dichlorvinyl)-cyclopropyl | |
| 5.76 | $C_4H_9$-sek | 2,2-Dimethyl-3-(2,2-dichlorvinyl)-cyclopropyl | |
| 5.77 | $CH_3$ | Cyclobutyl | |
| 5.78 | $C_2H_5$ | Cyclobutyl | |
| 5.79 | $C_3H_7$-iso | Cyclobutyl | |
| 5.80 | $C_4H_9$-sek | Cyclobutyl | |
| 5.81 | $CH_3$ | Cyclohexyl | |
| 5.82 | $C_2H_5$ | Cyclohexyl | |
| 5.83 | $C_3H_7$-iso | Cyclohexyl | |
| 5.84 | $C_4H_9$-sek | Cyclohexyl | |
| 5.85 | $CH_3$ | Phenyl | |
| 5.86 | $C_2H_5$ | Phenyl | |
| 5.87 | $C_3H_7$-iso | Phenyl | |
| 5.88 | $C_4H_9$-sek | Phenyl | |
| 5.89 | $CH_3$ | p-Chlorphenyl | |
| 5.90 | $C_2H_5$ | p-Chlorphenyl | |
| 5.91 | $C_3H_7$-iso | p-Chlorphenyl | |
| 5.92 | $C_4H_9$-sek | p-Chlorphenyl | |
| 5.93 | $CH_3$ | p-Tolyl | |
| 5.94 | $C_2H_5$ | p-Tolyl | |
| 5.95 | $C_3H_7$-iso | p-Tolyl | |
| 5.96 | $C_4H_9$-sek | p-Tolyl | |
| 5.97 | $CH_3$ | p-Nitrophenyl | |
| 5.98 | $C_2H_5$ | p-Nitrophenyl | 125-130 |
| 5.99 | $C_3H_7$-iso | p-Nitrophenyl | |
| 5.100 | $C_4H_9$-sek | p-Nitrophenyl | |
| 5.101 | $CH_3$ | $(CH_3)_3C-CH_2$ | |
| 5.102 | $C_2H_5$ | $(CH_3)_3C-CH_2$ | 103-105 |
| 5.103 | $C_3H_7$-iso | $(CH_3)_3C-CH_2$ | |
| 5.104 | $C_4H_9$-sek | $(CH_3)_3C-CH_2$ | |
| 5.105 | $CH_3$ | m-Chlorphenyl | |
| 5.106 | $C_2H_5$ | m-Chlorphenyl | 110-115 |
| 5.107 | $C_3H_7$-iso | m-Chlorphenyl | |
| 5.108 | $C_4H_9$-sek. | m-Chlorphenyl | |
| 5.109 | $CH_3$ | p-Methoxyphenyl | |
| 5.110 | $C_2H_5$ | p-Methoxyphenyl | 105-110 |
| 5.111 | $C_3H_7$-iso | p-Methoxyphenyl | |
| 5.112 | $C_4H_9$-sek | p-Methoxyphenyl | |
| 5.113 | $CH_3$ | 2,6-Difluorphenyl | |
| 5.114 | $C_2H_5$ | 2,6-Difluorphenyl | 107-111 |
| 5.115 | $C_3H_7$-iso | 2,6-Difluorphenyl | |
| 5.116 | $C_4H_9$-sek | 2,6-Difluorphenyl | |

Der Inhalt der Tabelle besitzt illustrativen Charakter und stellt keine Begrenzung dar.

Tabelle 6

Typische Vertreter von Verbindungen der Formel I, worin X für $-CH[OSi(CH_3)_2C_4H_9-t]$ steht:

| Verb. Nr. | $R_2$ | R | Physik.Konst. Smp. [°C] |
|---|---|---|---|
| 6.1 | $CH_3$ | H | |
| 6.2 | $C_2H_5$ | H | |
| 6.3 | $C_3H_7$-iso | H | |
| 6.4 | $C_4H_9$-sek | H | |
| 6.5 | $CH_3$ | $CH_3$ | |
| 6.6 | $C_2H_5$ | $CH_3$ | |
| 6.7 | $C_3H_7$-iso | $CH_3$ | 78-83 |
| 6.8 | $C_4H_9$-sek | $CH_3$ | |
| 6.9 | $CH_3$ | $C(CH_3)_3$ | |
| 6.10 | $C_2H_5$ | $C(CH_3)_3$ | 65-70 |
| 6.11 | $C_3H_7$-iso | $C(CH_3)_3$ | 86-90 |
| 6.12 | $C_4H_9$-sek | $C(CH_3)_3$ | |
| 6.13 | $CH_3$ | $CH_3OCH_2$ | |
| 6.14 | $C_2H_5$ | $CH_3OCH_2$ | |
| 6.15 | $C_3H_7$-iso | $CH_3OCH_2$ | |
| 6.16 | $C_4H_9$-sek | $CH_3OCH_2$ | |
| 6.17 | $CH_3$ | $CH_3O(CH_3)_2C$ | |
| 6.18 | $C_2H_5$ | $CH_3O(CH_3)_2C$ | |
| 6.19 | $C_3H_7$-iso | $CH_3O(CH_3)_2C$ | |
| 6.20 | $C_4H_9$-sek | $CH_3O(CH_3)_2C$ | |
| 6.21 | $CH_3$ | $(CH_3)_2CH-CH_2$ | |
| 6.22 | $C_2H_5$ | $(CH_3)_2CH-CH_2$ | 65-70 |
| 6.23 | $C_3H_7$-iso | $(CH_3)_2CH-CH_2$ | |
| 6.24 | $C_4H_9$-sek | $(CH_3)_2CH-CH_2$ | |
| 6.25 | $CH_3$ | $CCl_3$ | |
| 6.26 | $C_2H_5$ | $CCl_3$ | |
| 6.27 | $C_3H_7$-iso | $CCl_3$ | |
| 6.28 | $C_4H_9$-sek | $CCl_3$ | |
| 6.29 | $CH_3$ | $CF_3$ | |
| 6.30 | $C_2H_5$ | $CF_3$ | |
| 6.31 | $C_3H_7$-iso | $CF_3$ | |
| 6.32 | $C_4H_9$-sek | $CF_3$ | |
| 6.33 | $CH_3$ | $Cl_3CCHCl$ | |
| 6.34 | $C_2H_5$ | $Cl_3CCHCl$ | |
| 6.35 | $C_3H_7$-iso | $Cl_3CCHCl$ | |
| 6.36 | $C_4H_9$-sek | $Cl_3CCHCl$ | |
| 6.37 | $CH_3$ | $ClCH_2CH_2CH_2$ | |
| 6.38 | $C_2H_5$ | $ClCH_2CH_2CH_2$ | |

Tabelle 6 (Fortsetzung)

| Verb. Nr. | $R_2$ | R | Physik.Konst. Smp. [°C] |
|---|---|---|---|
| 6.39 | $C_3H_7$-iso | $ClCH_2CH_2CH_2$ | |
| 6.40 | $C_4H_9$-sek | $ClCH_2CH_2CH_2$ | |
| 6.41 | $CH_3$ | $CH_2$=CH | |
| 6.42 | $C_2H_5$ | $CH_2$=CH | |
| 6.43 | $C_3H_7$-iso | $CH_2$=CH | |
| 6.44 | $C_4H_9$-sek | $CH_2$=CH | |
| 6.45 | $CH_3$ | $CH_2$=CH-$CH_2$ | |
| 6.46 | $C_2H_5$ | $CH_2$=CH-$CH_2$ | |
| 6.47 | $C_3H_7$-iso | $CH_2$=CH-$CH_2$ | |
| 6.48 | $C_4H_9$-sek | $CH_2$=CH-$CH_2$ | |
| 6.49 | $CH_3$ | CH≡C-$CH_2$ | |
| 6.50 | $C_2H_5$ | CH≡C-$CH_2$ | |
| 6.51 | $C_3H_7$-iso | CH≡C-$CH_2$ | |
| 6.52 | $C_4H_9$-sek | CH≡C-$CH_2$ | |
| 6.53 | $CH_3$ | $(CH_3)_2C$=CH | |
| 6.54 | $C_2H_5$ | $(CH_3)_2C$=CH | |
| 6.55 | $C_3H_7$-iso | $(CH_3)_2C$=CH | |
| 6.56 | $C_4H_9$-sek | $(CH_3)_2C$=CH | |
| 6.57 | $CH_3$ | $(Cl)_2C$=C(Cl) | |
| 6.58 | $C_2H_5$ | $(Cl)_2C$=C(Cl) | |
| 6.59 | $C_3H_7$-iso | $(Cl)_2C$=C(Cl) | |
| 6.60 | $C_4H_9$-sek | $(Cl)_2C$=C(Cl) | |
| 6.61 | $CH_3$ | $CF_3CCl_2$ | |
| 6.62 | $C_2H_5$ | $CF_3CCl_2$ | |
| 6.63 | $C_3H_7$-iso | $CF_3CCl_2$ | |
| 6.64 | $C_4H_9$-sek | $CF_3CCl_2$ | |
| 6.65 | $CH_3$ | Cyclopropyl | |
| 6.66 | $C_2H_5$ | Cyclopropyl | 91-95 |
| 6.67 | $C_3H_7$-iso | Cyclopropyl | |
| 6.68 | $C_4H_9$-sek | Cyclopropyl | |
| 6.69 | $CH_3$ | 2,2-Dimethyl-cyclopropyl | |
| 6.70 | $C_2H_5$ | 2,2-Dimethyl-cyclopropyl | |
| 6.71 | $C_3H_7$-iso | 2,2-Dimethyl-cyclopropyl | |
| 6.72 | $C_4H_9$-sek | 2,2-Dimethyl-cyclopropyl | |
| 6.73 | $CH_3$ | 2,2-Dimethyl-3-(2,2-dichlorvinyl)-cyclopropyl | |
| 6.74 | $C_2H_5$ | 2,2-Dimethyl-3-(2,2-dichlorvinyl)-cyclopropyl | |

Tabelle 6 (Fortsetzung)

| Verb. Nr. | $R_2$ | R | Physik.Konst. Smp. [°C] |
|---|---|---|---|
| 6.75 | $C_3H_7$-iso | 2,2-Dimethyl-3-(2,2-dichlorvinyl)-cyclopropyl | |
| 6.76 | $C_4H_9$-sek | 2,2-Dimethyl-3-(2,2-dichlorvinyl)-cyclopropyl | |
| 6.77 | $CH_3$ | Cyclobutyl | |
| 6.78 | $C_2H_5$ | Cyclobutyl | |
| 6.79 | $C_3H_7$-iso | Cyclobutyl | |
| 6.80 | $C_4H_9$-sek | Cyclobutyl | |
| 6.81 | $CH_3$ | Cyclohexyl | |
| 6.82 | $C_2H_5$ | Cyclohexyl | |
| 6.83 | $C_3H_7$-iso | Cyclohexyl | |
| 6.84 | $C_4H_9$-sek | Cyclohexyl | |
| 6.85 | $CH_3$ | Phenyl | |
| 6.86 | $C_2H_5$ | Phenyl | |
| 6.87 | $C_3H_7$-iso | Phenyl | |
| 6.88 | $C_4H_9$-sek | Phenyl | |
| 6.89 | $CH_3$ | p-Chlorphenyl | |
| 6.90 | $C_2H_5$ | p-Chlorphenyl | |
| 6.91 | $C_3H_7$-iso | p-Chlorphenyl | |
| 6.92 | $C_4H_9$-sek | p-Chlorphenyl | |
| 6.93 | $CH_3$ | p-Tolyl | |
| 6.94 | $C_2H_5$ | p-Tolyl | |
| 6.95 | $C_3H_7$-iso | p-Tolyl | |
| 6.96 | $C_4H_9$-sek | p-Tolyl | |
| 6.97 | $CH_3$ | p-Nitrophenyl | |
| 6.98 | $C_2H_5$ | p-Nitrophenyl | 120-125 |
| 6.99 | $C_3H_7$-iso | p-Nitrophenyl | |
| 6.100 | $C_4H_9$-sek | p-Nitrophenyl | |
| 6.101 | $CH_3$ | $(CH_3)_3C-CH_2$ | |
| 6.102 | $C_2H_5$ | $(CH_3)_3C-CH_2$ | 90-95 |
| 6.103 | $C_3H_7$-iso | $(CH_3)_3C-CH_2$ | |
| 6.104 | $C_4H_9$-sek | $(CH_3)_3C-CH_2$ | |
| 6.105 | $CH_3$ | m-Chlorphenyl | |
| 6.106 | $C_2H_5$ | m-Chlorphenyl | 103-106 |
| 6.107 | $C_3H_7$-iso | m-Chlorphenyl | |
| 6.108 | $C_4H_9$-sek | m-Chlorphenyl | |
| 6.109 | $CH_3$ | p-Methoxyphenyl | |
| 6.110 | $C_2H_5$ | p-Methoxyphenyl | 100-105 |
| 6.111 | $C_3H_7$-iso | p-Methoxyphenyl | |
| 6.112 | $C_4H_9$-sek | p-Methoxyphenyl | |
| 6.113 | $CH_3$ | 2,6-Difluorphenyl | |
| 6.114 | $C_2H_5$ | 2,6-Difluorphenyl | 108-110 |
| 6.115 | $C_3H_7$-iso | 2,6-Difluorphenyl | |
| 6.116 | $C_4H_9$-sek | 2,6-Difluorphenyl | |

Der Inhalt der Tabelle besitzt illustrativen Charakter und stellt keine Begrenzung dar.

Tabelle 7: Typische Vertreter von Verbindungen der Formel I, worin $R_1$ für die Gruppe

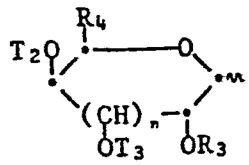

steht und $R_2$ für $CH_3$, $C_2H_5$, $C_3H_7$-iso oder $C_4H_9$-sek und R $CH_3$, $C_2H_5$, $C_3H_7$-i, $C_4H_9$-t, $(CH_3)_2CH-CH_2$ oder $(CH_3)_3C-CH_2$ bedeuten sind:

| Verb. Nr. | $R_1$ |
|---|---|
| 7.1 | 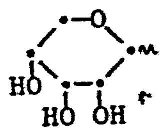 $C_5$-D-ribose |
| 7.2 | 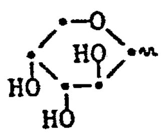 $C_5$-D-arabionose |
| 7.3 | 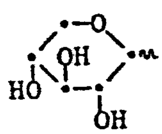 $C_5$-D-xylose |
| 7.4 | 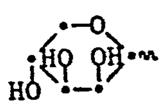 $C_5$-D-lyxose |
| 7.5 | 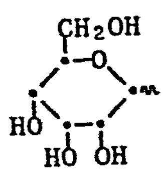 $C_6$-D-allose |

Tabelle 7 (Fortsetzung)

| Verb. Nr. | $R_1$ |
|---|---|
| 7.6 | 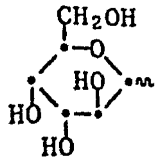 $C_6$-D-altrose |
| 7.7 | 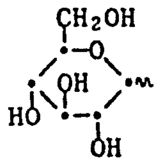 $C_6$-D-glukose |
| 7.8 | 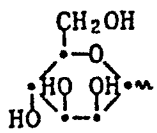 $C_6$-D-mannose |
| 7.9 | 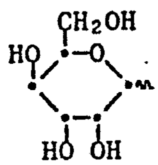 $C_6$-D-gulose |
| 7.10 | 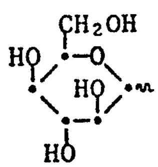 $C_6$-D-idose |
| 7.11 | 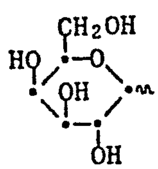 $C_6$-D-galactose |

Tabelle 7 (Fortsetzung)

| Verb. Nr. | $R_1$ |
|---|---|
| 7.12 | 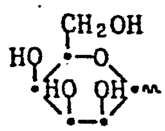 $C_6$-D-talose |
| 7.13 | 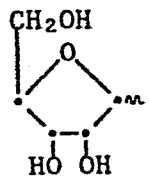 $C_5$-D-ribose |
| 7.14 | 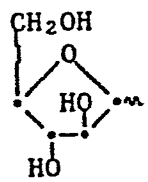 $C_5$-D-arabinose |
| 7.15 | 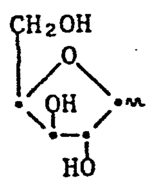 $C_5$-D-xylose |
| 7.16 | 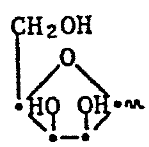 $C_5$-D-lyxose |
| 7.17 | 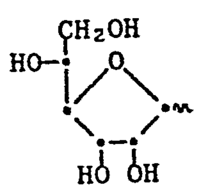 $C_6$-D-allose |

Tabelle 7 (Fortsetzung)

| Verb. Nr. | $R_1$ |
|---|---|
| 7.18 | 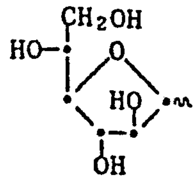 $C_6$-D-altrose |
| 7.19 | 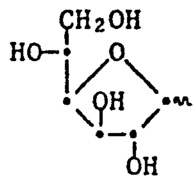 $C_6$-D-glukose |
| 7.20 | 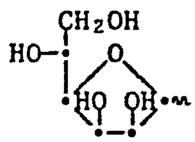 $C_6$-D-mannose |
| 7.21 | 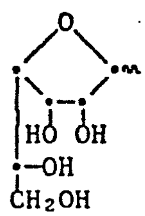 $C_6$-D-gulose |
| 7.22 | 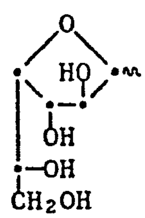 $C_6$-D-idose |

Tabelle 7 (Fortsetzung)

| Verb. Nr. | $R_1$ |
|---|---|
| 7.23 | 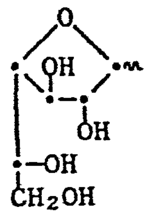<br>$C_6$-D-galactose |
| 7.24 | 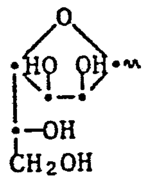<br>$C_6$-D-talose |
| 7.25 | 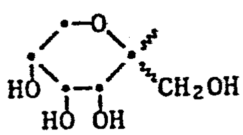<br>$C_6$-D-psicose |
| 7.26 | 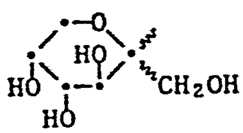<br>$C_6$-D-fructose |
| 7.27 | 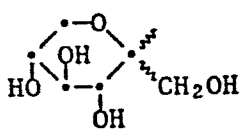<br>$C_6$-D-sorbose |

Tabelle 7 (Fortsetzung)

| Verb. Nr. | $R_1$ |
|---|---|
| 7.28 | 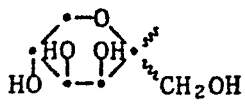<br>$C_6$-D-tagatose |
| 7.29 | 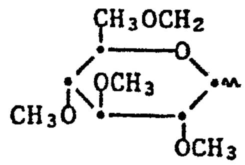<br>2,3,4,6-Tetra-O-methyl-($C_6$-D-glucose) |
| 7.30 | 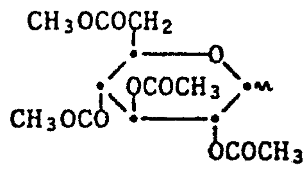<br>2,3,4,6-Tetra-O-acetyl-($C_6$-D-glucose) |
| 7.31 | 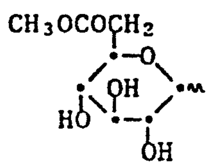<br>6-O-Acetyl-($C_6$-D-glucose) |
| 7.32 | 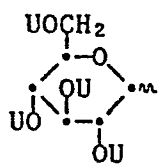 mit U = benzoyl<br>2,3,4,6-Tetra-O-benzoyl-($C_6$-D-glucose |
| 7.33 | 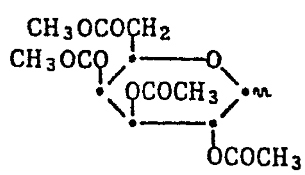 $R = C_4H_9(t)$<br>$R_2$ = iso-Propyl<br>Smp. 98-103°C<br>2,3,4,5-Tetra-O-acetyl-($C_6$-D-galactose) |

Tabelle 7 (Fortsetzung)

| Verb. Nr. | $R_1$ |
|---|---|
| 7.34 | 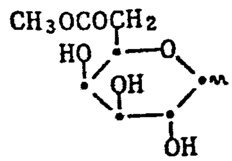<br>6-O-Acetyl-($C_6$-D-galactose) |
| 7.35 | 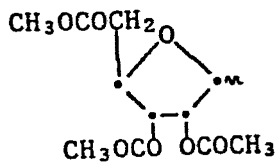<br>2,3,5-Tri-O-acetyl-($C_5$-D-ribose) |
| 7.36 | 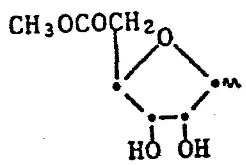<br>5-O-Acetyl-($C_5$-D-ribose) |
| 7.37 | 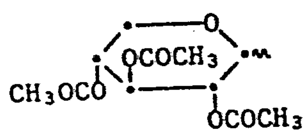<br>2,3,4-Tri-O-acetyl-($C_5$-D-xylose) |
| 7.38 | 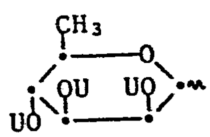 mit U=acetyl<br>2,3,4-Tri-O-acetyl-($C_6$-D-rhamnose) |

Tabelle 8

Typische Vertreter von Verbindungen der Formel I, worin X für -C(O)- steht:

| Verb. Nr. | $R_2$ | R | Physik.Konst. Smp. [°C] |
|---|---|---|---|
| 8.1 | $CH_3$ | H | |
| 8.2 | $C_2H_5$ | H | |
| 8.3 | $C_3H_7$-iso | H | |
| 8.4 | $C_4H_9$-sek | H | |
| 8.5 | $CH_3$ | $CH_3$ | |
| 8.6 | $C_2H_5$ | $CH_3$ | |
| 8.7 | $C_3H_7$-iso | $CH_3$ | |
| 8.8 | $C_4H_9$-sek | $CH_3$ | |
| 8.9 | $CH_3$ | $C(CH_3)_3$ | |
| 8.10 | $C_2H_5$ | $C(CH_3)_3$ | 120-123 |
| 8.11 | $C_3H_7$-iso | $C(CH_3)_3$ | |
| 8.12 | $C_4H_9$-sek | $C(CH_3)_3$ | |
| 8.13 | $CH_3$ | $CH_3OCH_2$ | |
| 8.14 | $C_2H_5$ | $CH_3OCH_2$ | |
| 8.15 | $C_3H_7$-iso | $CH_3OCH_2$ | |
| 8.16 | $C_4H_9$-sek | $CH_3OCH_2$ | |
| 8.17 | $CH_3$ | $CH_3O(CH_3)_2C$ | |
| 8.18 | $C_2H_5$ | $CH_3O(CH_3)_2C$ | |
| 8.19 | $C_3H_7$-iso | $CH_3O(CH_3)_2C$ | |
| 8.20 | $C_4H_9$-sek | $CH_3O(CH_3)_2C$ | |
| 8.21 | $CH_3$ | $(CH_3)_2CH-CH_2$ | |
| 8.22 | $C_2H_5$ | $(CH_3)_2CH-CH_2$ | |
| 8.23 | $C_3H_7$-iso | $(CH_3)_2CH-CH_2$ | |
| 8.24 | $C_4H_9$-sek | $(CH_3)_2CH-CH_2$ | |
| 8.25 | $CH_3$ | $CCl_3$ | |
| 8.26 | $C_2H_5$ | $CCl_3$ | |
| 8.27 | $C_3H_7$-iso | $CCl_3$ | |
| 8.28 | $C_4H_9$-sek | $CCl_3$ | |
| 8.29 | $CH_3$ | $CF_3$ | |
| 8.30 | $C_2H_5$ | $CF_3$ | |
| 8.31 | $C_3H_7$-iso | $CF_3$ | |
| 8.32 | $C_4H_9$-sek | $CF_3$ | |
| 8.33 | $CH_3$ | $Cl_3CCHCl$ | |
| 8.34 | $C_2H_5$ | $Cl_3CCHCl$ | |
| 8.35 | $C_3H_7$-iso | $Cl_3CCHCl$ | |
| 8.36 | $C_4H_9$-sek | $Cl_3CCHCl$ | |
| 8.37 | $CH_3$ | $ClCH_2CH_2CH_2$ | |
| 8.38 | $C_2H_5$ | $ClCH_2CH_2CH_2$ | |

Tabelle 8 (Fortsetzung)

| Verb. Nr. | $R_2$ | R |
|---|---|---|
| 8.39 | $C_3H_7$-iso | $ClCH_2CH_2CH_2$ |
| 8.40 | $C_4H_9$-sek | $ClCH_2CH_2CH_2$ |
| 8.41 | $CH_3$ | $CH_2$=CH |
| 8.42 | $C_2H_5$ | $CH_2$=CH |
| 8.43 | $C_3H_7$-iso | $CH_2$=CH |
| 8.44 | $C_4H_9$-sek | $CH_2$=CH |
| 8.45 | $CH_3$ | $CH_2$=CH-$CH_2$ |
| 8.46 | $C_2H_5$ | $CH_2$=CH-$CH_2$ |
| 8.47 | $C_3H_7$-iso | $CH_2$=CH-$CH_2$ |
| 8.48 | $C_4H_9$-sek | $CH_2$=CH-$CH_2$ |
| 8.49 | $CH_3$ | CH≡C-$CH_2$ |
| 8.50 | $C_2H_5$ | CH≡C-$CH_2$ |
| 8.51 | $C_3H_7$-iso | CH≡C-$CH_2$ |
| 8.52 | $C_4H_9$-sek | CH≡C-$CH_2$ |
| 8.53 | $CH_3$ | $(CH_3)_2C$=CH |
| 8.54 | $C_2H_5$ | $(CH_3)_2C$=CH |
| 8.55 | $C_3H_7$-iso | $(CH_3)_2C$=CH |
| 8.56 | $C_4H_9$-sek | $(CH_3)_2C$=CH |
| 8.57 | $CH_3$ | $(Cl)_2C$=C(Cl) |
| 8.58 | $C_2H_5$ | $(Cl)_2C$=C(Cl) |
| 8.59 | $C_3H_7$-iso | $(Cl)_2C$=C(Cl) |
| 8.60 | $C_4H_9$-sek | $(Cl)_2C$=C(Cl) |
| 8.61 | $CH_3$ | $CF_3CCl_2$ |
| 8.62 | $C_2H_5$ | $CF_3CCl_2$ |
| 8.63 | $C_3H_7$-iso | $CF_3CCl_2$ |
| 8.64 | $C_4H_9$-sek | $CF_3CCl_2$ |
| 8.65 | $CH_3$ | Cyclopropyl |
| 8.66 | $C_2H_5$ | Cyclopropyl |
| 8.67 | $C_3H_7$-iso | Cyclopropyl |
| 8.68 | $C_4H_9$-sek | Cyclopropyl |
| 8.69 | $CH_3$ | 2,2-Dimethyl-cyclopropyl |
| 8.70 | $C_2H_5$ | 2,2-Dimethyl-cyclopropyl |
| 8.71 | $C_3H_7$-iso | 2,2-Dimethyl-cyclopropyl |
| 8.72 | $C_4H_9$-sek | 2,2-Dimethyl-cyclopropyl |
| 8.73 | $CH_3$ | 2,2-Dimethyl-3-(2,2-dichlorvinyl)-cyclopropyl |
| 8.74 | $C_2H_5$ | 2,2-Dimethyl-3-(2,2-dichlorvinyl)-cyclopropyl |

Tabelle 8 (Fortsetzung)

| Verb. Nr. | $R_2$ | R |
|---|---|---|
| 8.75 | $C_3H_7$-iso | 2,2-Dimethyl-3-(2,2-dichlorvinyl)-cyclopropyl |
| 8.76 | $C_4H_9$-sek | 2,2-Dimethyl-3-(2,2-dichlorvinyl)-cyclopropyl |
| 8.77 | $CH_3$ | Cyclobutyl |
| 8.78 | $C_2H_5$ | Cyclobutyl |
| 8.79 | $C_3H_7$-iso | Cyclobutyl |
| 8.80 | $C_4H_9$-sek | Cyclobutyl |
| 8.81 | $CH_3$ | Cyclohexyl |
| 8.82 | $C_2H_5$ | Cyclohexyl |
| 8.83 | $C_3H_7$-iso | Cyclohexyl |
| 8.84 | $C_4H_9$-sek | Cyclohexyl |
| 8.85 | $CH_3$ | Phenyl |
| 8.86 | $C_2H_5$ | Phenyl |
| 8.87 | $C_3H_7$-iso | Phenyl |
| 8.88 | $C_4H_9$-sek | Phenyl |
| 8.89 | $CH_3$ | p-Chlorphenyl |
| 8.90 | $C_2H_5$ | p-Chlorphenyl |
| 8.91 | $C_3H_7$-iso | p-Chlorphenyl |
| 8.92 | $C_4H_9$-sek | p-Chlorphenyl |
| 8.93 | $CH_3$ | p-Tolyl |
| 8.94 | $C_2H_5$ | p-Tolyl |
| 8.95 | $C_3H_7$-iso | p-Tolyl |
| 8.96 | $C_4H_9$-sek | p-Tolyl |
| 8.97 | $CH_3$ | p-Nitrophenyl |
| 8.98 | $C_2H_5$ | p-Nitrophenyl |
| 8.99 | $C_3H_7$-iso | p-Nitrophenyl |
| 8.100 | $C_4H_9$-sek | p-Nitrophenyl |
| 8.101 | $CH_3$ | $(CH_3)_3C-CH_2$ |
| 8.102 | $C_2H_5$ | $(CH_3)_3C-CH_2$ |
| 8.103 | $C_3H_7$-iso | $(CH_3)_3C-CH_2$ |
| 8.104 | $C_4H_9$-sek | $(CH_3)_3C-CH_2$ |
| 8.105 | $CH_3$ | m-Chlorphenyl |
| 8.106 | $C_2H_5$ | m-Chlorphenyl |
| 8.107 | $C_3H_7$-iso | m-Chlorphenyl |
| 8.108 | $C_4H_9$-sek. | m-Chlorphenyl |
| 8.109 | $CH_3$ | p-Methoxyphenyl |
| 8.110 | $C_2H_5$ | p-Methoxyphenyl |
| 8.111 | $C_3H_7$-iso | p-Methoxyphenyl |
| 8.112 | $C_4H_9$-sek | p-Methoxyphenyl |
| 8.113 | $CH_3$ | 2,6-Difluorphenyl |
| 8.114 | $C_2H_5$ | 2,6-Difluorphenyl |
| 8.115 | $C_3H_7$-iso | 2,6-Difluorphenyl |
| 8.116 | $C_4H_9$-sek | 2,6-Difluorphenyl |

Der Inhalt der Tabelle besitzt illustrativen Charakter und stellt keine Begrenzung dar.

Tabelle 9

Typische Vertreter von Verbindungen der Formel I, worin X für -C(=N-OH)- steht:

| Verb. Nr. | $R_2$ | R | Physik.Konst. Smp. [°C] |
|---|---|---|---|
| 9.1 | $CH_3$ | H | |
| 9.2 | $C_2H_5$ | H | |
| 9.3 | $C_3H_7$-iso | H | |
| 9.4 | $C_4H_9$-sek | H | |
| 9.5 | $CH_3$ | $CH_3$ | |
| 9.6 | $C_2H_5$ | $CH_3$ | |
| 9.7 | $C_3H_7$-iso | $CH_3$ | |
| 9.8 | $C_4H_9$-sek | $CH_3$ | |
| 9.9 | $CH_3$ | $C(CH_3)_3$ | 145-150 |
| 9.10 | $C_2H_5$ | $C(CH_3)_3$ | |
| 9.11 | $C_3H_7$-iso | $C(CH_3)_3$ | |
| 9.12 | $C_4H_9$-sek | $C(CH_3)_3$ | |
| 9.13 | $CH_3$ | $CH_3OCH_2$ | |
| 9.14 | $C_2H_5$ | $CH_3OCH_2$ | |
| 9.15 | $C_3H_7$-iso | $CH_3OCH_2$ | |
| 9.16 | $C_4H_9$-sek | $CH_3OCH_2$ | |
| 9.17 | $CH_3$ | $CH_3O(CH_3)_2C$ | |
| 9.18 | $C_2H_5$ | $CH_3O(CH_3)_2C$ | |
| 9.19 | $C_3H_7$-iso | $CH_3O(CH_3)_2C$ | |
| 9.20 | $C_4H_9$-sek | $CH_3O(CH_3)_2C$ | |
| 9.21 | $CH_3$ | $(CH_3)_2CH-CH_2$ | |
| 9.22 | $C_2H_5$ | $(CH_3)_2CH-CH_2$ | |
| 9.23 | $C_3H_7$-iso | $(CH_3)_2CH-CH_2$ | |
| 9.24 | $C_4H_9$-sek | $(CH_3)_2CH-CH_2$ | |
| 9.25 | $CH_3$ | $CCl_3$ | |
| 9.26 | $C_2H_5$ | $CCl_3$ | |
| 9.27 | $C_3H_7$-iso | $CCl_3$ | |
| 9.28 | $C_4H_9$-sek | $CCl_3$ | |
| 9.29 | $CH_3$ | $CF_3$ | |
| 9.30 | $C_2H_5$ | $CF_3$ | |
| 9.31 | $C_3H_7$-iso | $CF_3$ | |
| 9.32 | $C_4H_9$-sek | $CF_3$ | |
| 9.33 | $CH_3$ | $Cl_3CCHCl$ | |
| 9.34 | $C_2H_5$ | $Cl_3CCHCl$ | |
| 9.35 | $C_3H_7$-iso | $Cl_3CCHCl$ | |
| 9.36 | $C_4H_9$-sek | $Cl_3CCHCl$ | |
| 9.37 | $CH_3$ | $ClCH_2CH_2CH_2$ | |
| 9.38 | $C_2H_5$ | $ClCH_2CH_2CH_2$ | |

Tabelle 9 (Fortsetzung)

| Verb. Nr. | $R_2$ | R |
|---|---|---|
| 9.39 | $C_3H_7$-iso | $ClCH_2CH_2CH_2$ |
| 9.40 | $C_4H_9$-sek | $ClCH_2CH_2CH_2$ |
| 9.41 | $CH_3$ | $CH_2$=CH |
| 9.42 | $C_2H_5$ | $CH_2$=CH |
| 9.43 | $C_3H_7$-iso | $CH_2$=CH |
| 9.44 | $C_4H_9$-sek | $CH_2$=CH |
| 9.45 | $CH_3$ | $CH_2$=CH-$CH_2$ |
| 9.46 | $C_2H_5$ | $CH_2$=CH-$CH_2$ |
| 9.47 | $C_3H_7$-iso | $CH_2$=CH-$CH_2$ |
| 9.48 | $C_4H_9$-sek | $CH_2$=CH-$CH_2$ |
| 9.49 | $CH_3$ | CH≡C-$CH_2$ |
| 9.50 | $C_2H_5$ | CH≡C-$CH_2$ |
| 9.51 | $C_3H_7$-iso | CH≡C-$CH_2$ |
| 9.52 | $C_4H_9$-sek | CH≡C-$CH_2$ |
| 9.53 | $CH_3$ | $(CH_3)_2C$=CH |
| 9.54 | $C_2H_5$ | $(CH_3)_2C$=CH |
| 9.55 | $C_3H_7$-iso | $(CH_3)_2C$=CH |
| 9.56 | $C_4H_9$-sek | $(CH_3)_2C$=CH |
| 9.57 | $CH_3$ | $(Cl)_2C$=C(Cl) |
| 9.58 | $C_2H_5$ | $(Cl)_2C$=C(Cl) |
| 9.59 | $C_3H_7$-iso | $(Cl)_2C$=C(Cl) |
| 9.60 | $C_4H_9$-sek | $(Cl)_2C$=C(Cl) |
| 9.61 | $CH_3$ | $CF_3CCl_2$ |
| 9.62 | $C_2H_5$ | $CF_3CCl_2$ |
| 9.63 | $C_3H_7$-iso | $CF_3CCl_2$ |
| 9.64 | $C_4H_9$-sek | $CF_3CCl_2$ |
| 9.65 | $CH_3$ | Cyclopropyl |
| 9.66 | $C_2H_5$ | Cyclopropyl |
| 9.67 | $C_3H_7$-iso | Cyclopropyl |
| 9.68 | $C_4H_9$-sek | Cyclopropyl |
| 9.69 | $CH_3$ | 2,2-Dimethyl-cyclopropyl |
| 9.70 | $C_2H_5$ | 2,2-Dimethyl-cyclopropyl |
| 9.71 | $C_3H_7$-iso | 2,2-Dimethyl-cyclopropyl |
| 9.72 | $C_4H_9$-sek | 2,2-Dimethyl-cyclopropyl |
| 9.73 | $CH_3$ | 2,2-Dimethyl-3-(2,2-dichlorvinyl)-cyclopropyl |
| 9.74 | $C_2H_5$ | 2,2-Dimethyl-3-(2,2-dichlorvinyl)-cyclopropyl |

Tabelle 9 (Fortsetzung)

| Verb. Nr. | $R_2$ | R |
|---|---|---|
| 9.75 | $C_3H_7$-iso | 2,2-Dimethyl-3-(2,2-dichlorvinyl)-cyclopropyl |
| 9.76 | $C_4H_9$-sek | 2,2-Dimethyl-3-(2,2-dichlorvinyl)-cyclopropyl |
| 9.77 | $CH_3$ | Cyclobutyl |
| 9.78 | $C_2H_5$ | Cyclobutyl |
| 9.79 | $C_3H_7$-iso | Cyclobutyl |
| 9.80 | $C_4H_9$-sek | Cyclobutyl |
| 9.81 | $CH_3$ | Cyclohexyl |
| 9.82 | $C_2H_5$ | Cyclohexyl |
| 9.83 | $C_3H_7$-iso | Cyclohexyl |
| 9.84 | $C_4H_9$-sek | Cyclohexyl |
| 9.85 | $CH_3$ | Phenyl |
| 9.86 | $C_2H_5$ | Phenyl |
| 9.87 | $C_3H_7$-iso | Phenyl |
| 9.88 | $C_4H_9$-sek | Phenyl |
| 9.89 | $CH_3$ | p-Chlorphenyl |
| 9.90 | $C_2H_5$ | p-Chlorphenyl |
| 9.91 | $C_3H_7$-iso | p-Chlorphenyl |
| 9.92 | $C_4H_9$-sek | p-Chlorphenyl |
| 9.93 | $CH_3$ | p-Tolyl |
| 9.94 | $C_2H_5$ | p-Tolyl |
| 9.95 | $C_3H_7$-iso | p-Tolyl |
| 9.96 | $C_4H_9$-sek | p-Tolyl |
| 9.97 | $CH_3$ | p-Nitrophenyl |
| 9.98 | $C_2H_5$ | p-Nitrophenyl |
| 9.99 | $C_3H_7$-iso | p-Nitrophenyl |
| 9.100 | $C_4H_9$-sek | p-Nitrophenyl |
| 9.101 | $CH_3$ | $(CH_3)_3C-CH_2$ |
| 9.102 | $C_2H_5$ | $(CH_3)_3C-CH_2$ |
| 9.103 | $C_3H_7$-iso | $(CH_3)_3C-CH_2$ |
| 9.104 | $C_4H_9$-sek | $(CH_3)_3C-CH_2$ |
| 9.105 | $CH_3$ | m-Chlorphenyl |
| 9.106 | $C_2H_5$ | m-Chlorphenyl |
| 9.107 | $C_3H_7$-iso | m-Chlorphenyl |
| 9.108 | $C_4H_9$-sek. | m-Chlorphenyl |
| 9.109 | $CH_3$ | p-Methoxyphenyl |
| 9.110 | $C_2H_5$ | p-Methoxyphenyl |
| 9.111 | $C_3H_7$-iso | p-Methoxyphenyl |
| 9.112 | $C_4H_9$-sek | p-Methoxyphenyl |
| 9.113 | $CH_3$ | 2,6-Difluorphenyl |
| 9.114 | $C_2H_5$ | 2,6-Difluorphenyl |
| 9.115 | $C_3H_7$-iso | 2,6-Difluorphenyl |
| 9.116 | $C_4H_9$-sek | 2,6-Difluorphenyl |

Der Inhalt der Tabelle besitzt illustrativen Charakter und stellt keine Begrenzung dar.

Formulierungsbeispiele für den Wirkstoff der Formel I

(% = Gewichtsprozent)

| Spritzpulver | a) | b) | c) |
|---|---|---|---|
| Wirkstoff aus den Tabellen | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyethylenglykol-ether (7-8 Mol AeO) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| Emulsions-Konzentrat | |
|---|---|
| Wirkstoff aus den Tabellen | 10 % |
| Octylphenolpolyethylenglykolether (4-5 Mol AeO) | 3 % |
| Ca-Dodecylbenzolsulfonat | 3 % |
| Ricinusölpolyglykolether (36 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| Stäubemittel | a) | b) |
|---|---|---|
| Wirkstoff aus den Tabellen | 5 % | 8 % |
| Talkum | 95 % | - |
| Kaolin | - | 92 % |

Man erhält anwendungsfertige Stäubemittel, indem der Wirkstoff mit dem Träger vermischt und auf einer geeigneten Mühle vermahlen wird.

Extruder Granulat

| | |
|---|---|
| Wirkstoff aus den Tabellen | 10 % |
| Na-Ligninsulfonat | 2 % |
| Carboximethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

Tabellen bzw. Boli

| | | |
|---|---|---|
| I | Ein Wirkstoff aus den Tabellen | 33,0 % |
| | Methylcellulose | 0,80 % |
| | Kieselsäure hochdispers | 0,80 % |
| | Maisstärke | 8,40 % |

Methylcellulose in Wasser einrühren und quellen lassen; Kieselsäure in die Quellung einrühren und homogen suspendieren. Wirkstoff und Maisstärke mischen. In diese Mischung die wässrige Suspension einarbeiten und zu einem Teig kneten. Diese Masse durch ein Sieb (Maschenweite 12 M) granulieren und dann trocknen.

| | | |
|---|---|---|
| II | Milchzucker krist. | 22,50 % |
| | Maisstärke | 17,00 % |
| | mikrokrist. Cellulose | 16,50 % |
| | Magnesiumstearat | 1,00 % |

Alle 4 Hilfsstoffe gut mischen

Phasen I und II mischen und zu Tabletten oder Boli verpressen.

Falls die Verbindungen der Formel I bzw. entsprechende Mittel zur Bekämpfung von endoparasitären Nematoden, Cestoden und Trematoden bei Haus- und Nutztieren, wie Rindern, Schafen, Ziegen, Katzen und Hunden, verwendet werden, können sie den Tieren sowohl als Einzeldosis wie auch wiederholt verabreicht werden, wobei die einzelnen Gaben je nach Tierart vorzugsweise zwischen 0,1 und 10 mg pro kg Körpergewicht betragen. Durch eine protrahierte Verabreichung erzielt man in manchen Fällen eine bessere Wirkung oder man kann mit geringeren Gesamtdosen auskommen. Der Wirkstoff bzw. die ihn enthaltenden Mittel können auch dem Futter oder den Tränken zugesetzt werden. Das Fertigfutter enthält die Wirkstoffkombinationen vorzugsweise in einer Konzentration von 0,005 bis 0,1 Gew. %. Die Mittel können in Form von Lösungen, Emulsionen, Suspensionen, Pulver, Tabletten, Bolussen oder Kapseln peroral den Tieren verabreicht werden. Soweit die physikalischen und toxikologischen Eigenschaften von Lösungen oder Emulsionen dies zulassen, können die Verbindungen der Formel I bzw. die enthaltende Mittel an Tieren auch beispielsweise subcutan injiziert, intraruminal verabreicht oder mittels der Pour-on-Methode auf den Körper der Tiere appliziert werden. Ferner ist eine Verabreichung des Wirkstoffs an die Tiere auch durch Lecksteine (Salz) oder Molasse-Blöcke möglich.

## Biologische Beispiele

### B-1. Insektizide Frassgift-Wirkung bei Spodoptera littoralis

Eingetopfte Baumwollpflanzen im 5-Blatt-Stadium werden mit einer acetonisch/wässrigen Versuchslösung besprüht, die 3, 12,5 oder 50 ppm der zu prüfenden Verbindung enthält.

Nach dem Antrocknen des Belags werden die Pflanzen mit ca. 30 Larven ($L_1$-Stadium) von Spodoptera littoralis besetzt. Pro Versuchsverbindung und pro Test-Spezies verwendet man zwei Pflanzen. Der Versuch wird bei ca. 24°C und 60 % relativer Luftfeuchtigkeit durchgeführt. Auswertungen und Zwischenauswertungen auf moribunde Tiere, Wachstum und Larven und Frassschäden erfolgen nach 24 Stunden, 48 Stunden und 72 Stunden.

Die Verbindungen der Formeln I, wie z.B. Nr. 5.10, 5.11, 5.22, 5.65, 5.103 und 7.33 erzielten bereits bei einer Wirkstoffkonzentration von 3 ppm eine vollständige Abtötung nach 24 Stunden.

B-2. Wirkung gegen pflanzenschädigende Akariden

OP-sensible Tetranychus urticae

Die Primärblätter von Bohnenpflanzen (Phaseolus vulgaris) werden 16 Stunden vor dem Versuch mit einem durch T. urticae infestierten, aus einer Massenzucht stammenden Blattstück belegt. Die so mit allen Milben-Stadien infestierten Pflanzen werden nach Entfernung des Blattstücks mit einer Versuchslösung bis zur Tropfnässe besprüht, die wahlweise 0,4 ppm oder 1,6 ppm der zu prüfenden Verbindung enthält. Die Temperatur in der Gewächshauskabine beträgt ca. 25°C.

Nach sieben Tagen wird unter dem Binokular auf Prozentsatz mobiler Stadien (Adulte und Nymphen) und auf vorhandene Eier ausgewertet.

Die Verbindungen der Formel I, wie z.B. Nr. 5.10, 5.11, 5.22, 5.65, 5.103 und 7.33 erzielten bereits bei einer Wirkstoffkonzentration von 0,4 ppm vollständige Abtötung.

B-3. Wirkung gegen $L_1$-Larven von Lucilia sericata

1 ml einer wässrigen Suspension der zu prüfenden Aktivsubstanz werden so mit 3 ml eines speziellen Larvenzuchtmediums bei ca. 50°C vermischt, dass ein Homogenisat von wahlweise 250 ppm oder 125 ppm Wirkstoffgehalt entsteht. In jede Reagenzglas-Probe werden ca. 30 Lucilia-Larven ($L_1$) eingesetzt. Nach 4 Tagen wird die Mortalitätsrate bestimmt. Die Verbindungen der Formel I wie z.B. Nr.5.11, 5.10, 5.66, 5.98, 5.102, 5.110 und 9.10 erzielten mit 125 ppm eine Wirkung von 100 %.

B-4. Akarizide Wirkung gegen Boophilus microplus (Biarra-Stamm)

Auf einer PVC-Platte wird waagrecht ein Klebstreifen so befestigt, das darauf 10 mit Blut vollgesogene Zecken-Weibchen von Boophilus microplus (Biarra-Stamm) nebeneinander in einer Reihe mit dem Rücken aufgeklebt werden können. Jeder Zecke wird mit einer Injektionsnadel 1 µl einer Flüssigkeit injiziert, die eine 1:1-Mischung von Polyethylenglykol und Aceton darstellt und in der eine bestimmte Wirkstoffmenge von 1,0 µg pro Zecke gelöst ist. Kontrolltiere erhalten eine wirkstofffreie Injektion. Nach der Behandlung werden die Tiere unter Normalbedingungen in einem Insektarium bei ca. 28°C und 80 % relativer Luftfeuchtigkeit gehalten, bis die Eiablage erfolgt und die Larven aus den Eiern der Kontrolltiere geschlüpft sind.

Die Aktivität einer geprüften Substanz wird mit der $IR_{90}$ bestimmt, d.h. es wird jene Wirkstoffdosis ermittelt, bei der noch nach 30 Tagen 9 von 10 Zeckenweibchen (= 90 %) Eier ablegen, die nicht schlupffähig sind.

Die Verbindungen der Formeln I, wie z.B. Nr. 5.10, 5.11, 5.22, 5.65, 5.103 und 7.33 erzielen eine $IR_{90}$ von 1,0 µg.

B-5. Versuch an mit Nematoden (Haemonchus concortus und Trichostrongylus colubriformis) infizierten Schafen

Der Wirkstoff wird als Suspension formuliert mit einer Magensonde oder durch Injektion in den Pansen eines Schafes gegeben, das mit Haemonchus concortus und Trychostrongylus colubriformis künstlich infiziert worden ist. Pro Dosis werden 1 bis 3 Tiere verwendet. Jedes Schaf wird nur einmal mit einer einzigen Dosis behandelt, und zwar wahlweise mit 1 mg oder 0,5 mg/kg Körpergewicht. Die Evaluierung erfolgt durch Vergleich der Anzahl der vor und nach Behandlung im Kot der Schafe ausgeschiedenen Wurmeier.

Gleichzeitig und gleichartig infizierte aber unbehandelte Schafe dienen als Kontrolle. Schafe, die mit einer der Verbindungen der Formeln I, wie z.B. Nr. 5.10, 5.11, 5.22, 5.66, 5.102, 5.110, 5.98,

5.106, 5.6 und 7.33 bei 1 mg/kg behandelt wurden, zeigten im Vergleich zu unbehandelten, aber infizierten Vergleichsgruppen keinen Nematodenbefall (= komplette Reduktion der Wurmeier im Kot).

B-6. Kontaktwirkung auf Aphis craccivora

Erbsenkeimlinge, die mit sämtlichen Entwicklungsstadien der Laus infiziert sind, werden mit einer aus einem Emulsionskonzentrat hergestellten Wirkstofflösung besprüht, die wahlweise 50 ppm, 25 ppm oder 12,5 ppm Wirkstoff enthält. Nach 3 Tagen wird auf mehr als 80 % tote bzw. abgefallene Blattläuse ausgewertet. Nur bei dieser Wirkungshöhe wird ein Präparat als wirksam eingestuft.

Die Verbindungen der Formel I, wie z.B. Nr. 5.10, 5.11, 5.22, 5.66, 5.102, 5.6, 5.98, 5.110, 5.106 und 7.33 erzielten bei einer Konzentration von 12,5 ppm eine vollständige Abtötung (= 100 %).

B-7. Larvizidwirkung gegen Aedes aegypti

Auf die Oberfläche von 150 ml Wasser, das sich in einem Behälter befindet, wird soviel einer 0,1 %igen acetonischen Lösung des Wirkstoffes pipettiert, dass Konzentrationen von wahlweise 10 ppm, 3,3 ppm und 1,6 ppm erhalten werden. Nach Verdunsten des Acetons wird der Behälter mit ca. 30-40 3 Tage alten Aedes-Larven beschickt. Nach 1, 2 und 5 Tagen wird die Mortalität geprüft.

Die Verbindungen der Formeln I, wie z.B. Nr. 5.10, 5.11, 5.22, 5.65, 5.103, 5.98, 5.110, 5.6, 5.106 und 7.33 bewirkten in diesem Test bei einer Konzentration von 1,6 ppm bereits nach einem Tag eine vollständige Abtötung sämtlicher Larven.

Patentansprüche (für alle Vertragsstaaten ausser AT und ES)

1. Verbindung der Formel I

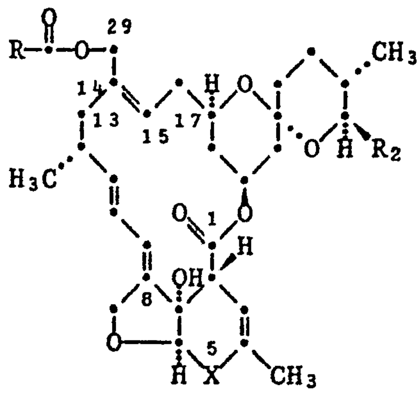

(I)

in welcher
X für -CH($OR_1$)-, -C(O)- oder -C(=N-OH)- steht;
$R_1$ Wasserstoff, eine Silyl-, eine Acylgruppe oder einen Zuckerrest,
$R_2$ Methyl, Ethyl, Isopropyl oder sek.Butyl und
R Wasserstoff, unsubstituierte oder substituierte gerad- oder verzweigtkettige $C_1$-$C_{18}$-Alkyl-Gruppen, unsubstituierte oder substituierte $C_3$-$C_{10}$-Cycloalkyl-Gruppen, unsubstituierte oder substituierte $C_2$-$C_6$-Alkenyl-Gruppen, unsubstituierte oder substituierte $C_2$-$C_6$-Alkinyl-Gruppen oder unsubstituierte oder substituierte Phenyl-Gruppen bedeuten.

2. Verbindungen der Formel I nach Anspruch 1, worin
X für -CH($OR_1$)- oder -C(O)- steht;
$R_1$ Wasserstoff, eine Silyl-, eine Acylgruppe oder einen Zuckerrest,
$R_2$ Methyl, Ethyl, Isopropyl oder sek.Butyl und
R Wasserstoff, unsubstituierte oder substituierte gerad- oder verzweigtkettige $C_1$-$C_{18}$-Alkyl-Gruppen, unsubstituierte oder substituierte $C_3$-$C_{10}$-Cycloalkyl-Gruppen, unsubstituierte oder substituierte $C_2$-$C_6$-Alkenyl-Gruppen, unsubstituierte oder substituierte $C_2$-$C_6$-Alkinyl-Gruppen oder unsubstituierte oder substituierte Phenyl-Gruppen bedeuten.

3. Verbindungen der Formel I nach Anspruch 2, worin X für $-CH(OR_1)-$ steht; $R_1$ Wasserstoff darstellt, $R_2$ für Methyl, Ethyl, Isopropyl oder sek.Butyl steht und R folgende Bedeutungen hat:

- unsubstituiertes oder durch 1 bis 4 Halogenatome oder $C_1$-$C_4$-Alkoxi substituiertes $C_1$-$C_6$-Alkyl, $C_2$-$C_4$-Alkenyl, $C_2$-$C_4$-Alkinyl oder $C_3$-$C_6$-Cycloalkyl;
- unsubstituiertes oder ein bis dreifach durch Halogen, $C_1$-$C_4$-Alkyl, $C_1$-$C_4$-Alkoxi, $C_1$-$C_4$-Alkylthio oder Nitro substituiertes Phenyl.

4. Verbindungen der Formel I nach Anspruch 3, worin X für $-CH(OR_1)-$ steht; $R_1$ Wasserstoff darstellt, $R_2$ für Methyl, Ethyl, Isopropyl oder sek.Butyl steht und R folgende Bedeutungen hat:

- unsubstituiertes oder durch 1 bis 4 Chlor- oder Fluoratome oder Methoxi substituiertes $C_1$-$C_5$-Alkyl, $C_2$-$C_3$-Alkenyl, $C_2$-$C_3$-Alkinyl oder $C_3$-$C_6$-Cycloalkyl;
- unsubstituiertes oder durch Chlor, Fluor, $C_1$-$C_2$-Alkyl, $C_1$-$C_2$-Alkoxi, $C_1$-$C_2$-Alkylthio oder Nitro substituiertes Phenyl.

5. Verbindungen der Formel I nach Anspruch 4, worin X für $-CH(OR_1)-$ steht; $R_1$ Wasserstoff darstellt, $R_2$ für Methyl oder Ethyl steht und R folgende Bedeutungen hat:

- unsubstituiertes oder durch 1 bis 4 Chlor- oder Fluoratome oder Methoxi substituiertes $C_1$-$C_5$-Alkyl, $C_2$-$C_3$-Alkenyl, $C_2$-$C_3$-Alkinyl oder $C_3$-$C_6$-Cycloalkyl;
- unsubstituiertes oder durch Chlor, Fluor, $C_1$-$C_2$-Alkyl, $C_1$-$C_2$-Alkoxi, $C_1$-$C_2$-Alkylthio oder Nitro substituiertes Phenyl.

6. Verbindungen der Formel I nach Anspruch 4, worin X für $-CH(OR_1)-$ steht; $R_1$ Wasserstoff darstellt, $R_2$ für Isopropyl oder sek.Butyl steht und R folgende Bedeutungen hat:

- unsubstituiertes oder durch 1 bis 4 Chlor- oder Fluoratome oder Methoxi substituiertes $C_1$-$C_5$-Alkyl, $C_2$-$C_3$-Alkenyl, $C_2$-$C_3$-Alkinyl oder $C_3$-$C_6$-Cycloalkyl;
- unsubstituiertes oder durch Chlor, Fluor, $C_1$-$C_2$-Alkyl, $C_1$-$C_2$-Alkoxi, $C_1$-$C_2$-Alkylthio oder Nitro substituiertes Phenyl.

7. Verbindungen der Formel I nach Anspruch 4, worin X für $-CH(OR_1)-$ steht; $R_1$ Wasserstoff darstellt, $R_2$ für Methyl oder Ethyl, Isopropyl oder sek.Butyl steht und R folgende Bedeutungen hat:

- unsubstituiertes oder durch 1 bis 3 Chloratome oder Fluoratome oder Methoxi substituiertes $C_1$-$C_5$-Alkyl, $C_2$-$C_3$-Alkenyl, $C_2$-$C_3$-Alkinyl oder $C_3$-$C_6$-Cycloalkyl;
- unsubstituiertes oder durch Chlor, Fluor, $C_1$-$C_2$-Alkyl, $C_1$-$C_2$-Alkoxi, $C_1$-$C_2$-Alkylthio oder Nitro substituiertes Phenyl.

8. Verbindungen der Formel I nach Anspruch 7, worin X für $-CH(OR_1)-$ steht; $R_1$ Wasserstoff darstellt, $R_2$ für Methyl oder Ethyl oder steht und R folgende Bedeutungen hat:

- unsubstituiertes oder durch 1 bis 3 Chloratome oder Fluoratome oder Methoxi substituiertes $C_1$-$C_5$-Alkyl, $C_2$-$C_3$-Alkenyl, $C_2$-$C_3$-Alkinyl oder $C_3$-$C_6$-Cycloalkyl.

9. Verbindungen der Formel I nach Anspruch 7, worin X für $-CH(OR_1)-$ steht; $R_1$ Wasserstoff darstellt, $R_2$ für Isopropyl oder sek.Butyl steht und R folgende Bedeutungen hat:

- unsubstituiertes oder durch 1 bis 3 Chloratome oder Fluoratome oder Methoxi substituiertes $C_1$-$C_5$-Alkyl, $C_2$-$C_3$-Alkenyl, $C_2$-$C_3$-Alkinyl oder $C_3$-$C_6$-Cycloalkyl.

10. Eine Verbindung der Formel I nach Anspruch 2, ausgewählt aus der Reihe:
29-tert.Butylcarbonyloxi-milbemycin-D,
29-Cyclopropylcarbonyloxi-milbemycin-$A_4$,

29-tert.Butylcarbonyloxi-milbemycin-$A_4$,
29-iso-Butylcarbonyloxi-milbemycin-$A_4$,
29-(2,2-Dimethylpropyl)carbonyloxi-milbemycin-$A_4$ und
29-Acetoxi-milbemycin-D.

11. Eine Verbindung der Formel I nach Anspruch 2, ausgewählt aus der Reihe:
5-O-tert.Butyldimethylsilyl-29-tert.butylcarbonyloxi-milbemycin-D,
5-O-tert.Butyldimethylsilyl-29-cyclopropylcarbonyloxi-milbemycin-$A_4$,
5-O-tert.Butyldimethylsilyl-29-acetoxi-milbemycin-D,
5-O-tert.Butyldimethylsilyl-29-tert.butylcarbonyloxi-milbemycin-$A_4$,
5-O-tert.Butyldimethylsilyl-29-(2,2-dimethylcarbonyloxi-milbemycin-$A_4$,
5-O-tert.Butyldimethylsilyl-29-iso-butylcarbonyloxi-milbemycin-$A_4$ und
5-O-2',3',4',6'-Tetra-O-acetyl-galactose-29-tert.butylcarbonyloxi-milbemycin-D.

12. Verfahren zur Herstellung von Verbindungen der Formel I

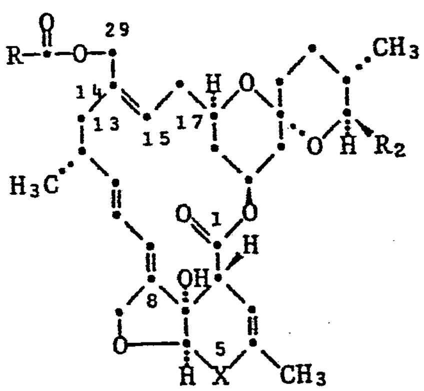

(I)

in welcher
X für $-CH(OR_1)-$ , $-C(O)-$ oder $-C(=N-OH-)-$ steht;
$R_1$ Wasserstoff, eine Silyl-, eine Acylgruppe oder einen Zuckerrest,
$R_2$ Methyl, Ethyl, Isopropyl oder sek.Butyl und
R Wasserstoff, unsubstituierte oder substituierte gerad- oder verzweigtkettige $C_1$-$C_{18}$-Alkyl-Gruppen, unsubstituierte oder substituierte $C_3$-$C_{10}$-Cycloalkyl-Gruppen, unsubstituierte oder sub-

stituierte $C_2$-$C_6$-Alkenyl-Gruppen, unsubstituierte oder substituierte $C_2$-$C_6$-Alkinyl-Gruppen oder unsubstituierte oder substituierte Phenyl-Gruppen bedeuten, dadurch gekennzeichnet, dass man entweder eine Verbindung der Formel II

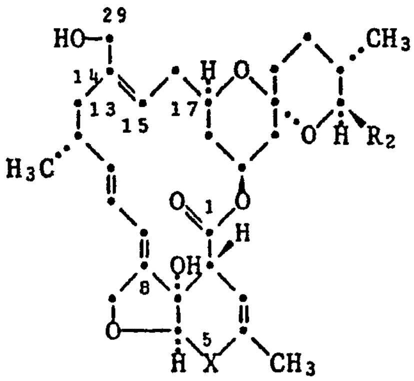

(II)

oder eine Verbindung der Formel III,

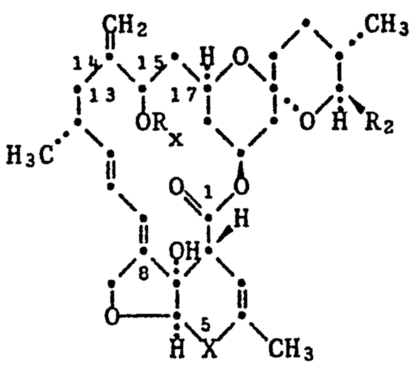

(III)

wobei in den Formeln II und III $R_2$ eine der unter Formel I angegebenen Bedeutungen hat, X für $-CH(OR_1)-$ steht; wobei $R_1$ eine OH-Schutzgruppe bedeutet und $R_x$ in Formel III für Wasserstoff oder eine leicht abspaltbare Gruppe steht, mit einer Säure der Formel IV

R-COOH (IV)

worin R eine der unter Formel I angegebenen Bedeutungen hat, oder einen zur Veresterung befähigten Derivat dieser Säure reagieren lässt oder die Verbindung der Formel III durch Verseifung der $OR_x$-Gruppe zuerst in eine Verbindung der Formel II überführt und anschliessend mit IV umsetzt und die resultierende Verbindung der

Formel I, insofern gewünscht, durch Abspaltung der OH-Schutzgruppe in ein 5-Hydroxi-Derivat der Formel I überführt und letzteres, sofern gewünscht, durch Nachsilylierung in ein Silylderivat oder durch Einführung eines Zuckerrestes in ein Zuckerderivat der Formel I überführt und sofern Verbindungen der Formel I, worin X für -C(=N-OH)- steht, das Herstellungsziel darstellen, ein 5-Keton der Formel I mit Hydroxylamin oder einem seiner Salze umsetzt.

13. Verfahren nach Anspruch 12, dadurch gekennzeichnet, dass man als ein zur Veresterung befähigtes Säurederivat der Säure IV

a) ihre Säureamide der Formel V

$$RCON(Alkyl)_2 \qquad (V)$$

worin die Alkylreste 1 bis 4 Kohlenstoffatome umfassen und vorzugsweise Methyl bedeuten,

b) ihre Säurehalogenide der Formel VI

$$RCOhal \qquad (VI),$$

worin hal Halogen bedeutet und bevorzugt für Chlor oder Brom steht, sowie

c) ihr Säureanhydrid der Formel VII

$$(RCO)_2O \qquad (VII)$$

worin R die unter Formel I angegebenen Bedeutungen hat, einsetzt.

14. Verfahren nach Anspruch 13, dadurch gekennzeichnet, dass die Reaktion von Verbindungen der Formel II oder III mit einem Säurechlorid oder Säurebromid der Formel VI oder einem Säureanhydrid der Formel VII in einem reaktionsinerten Lösungsmittel bei Temperaturen von 0° bis 100°C durchgeführt wird.

15. Verfahren nach Anspruch 14, dadurch gekennzeichnet, dass als Neutralisationsmittel eine Base eingesetzt wird.

16. Mittel zur Bekämpfung von Schädlingen, dadurch gekennzeichnet, dass es neben Trägermitteln, Verteilungsmitteln oder Träger- und Verteilungsmitteln als Wirkstoff mindestens eine Verbindung der Formel I enthält,

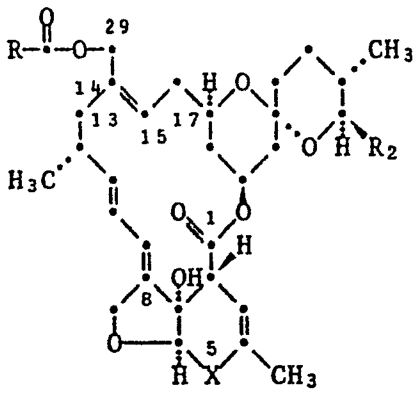

(I)

in welcher

X für $-CH(OR_1)-$, $-C(O)-$ oder $-C(=N-OH)-$ steht;

$R_1$ Wasserstoff, eine Silyl-, eine Acylgruppe oder einen Zuckerrest,

$R_2$ Methyl, Ethyl, Isopropyl oder sek.Butyl und

R Wasserstoff, unsubstituierte oder substituierte gerad- oder verzweigtkettige $C_1$-$C_{18}$-Alkyl-Gruppen, unsubstituierte oder substituierte $C_3$-$C_{10}$-Cycloalkyl-Gruppen, unsubstituierte oder substituierte $C_2$-$C_6$-Alkenyl-Gruppen, unsubstituierte oder substituierte $C_2$-$C_6$-Alkinyl-Gruppen oder unsubstituierte oder substituierte Phenyl-Gruppen bedeuten.

17. Mittel nach Anspruch 16, dadurch gekennzeichnet, dass es als Wirkstoff eine Verbindung der Formel I gemäss einem der Ansprüche 2 bis 11 enthält.

18. Verfahren zur Bekämpfung von Schädlingen, dadurch gekennzeichnet, dass man eine pestizid-wirksame Menge mindestens einer Verbindung der Formel I

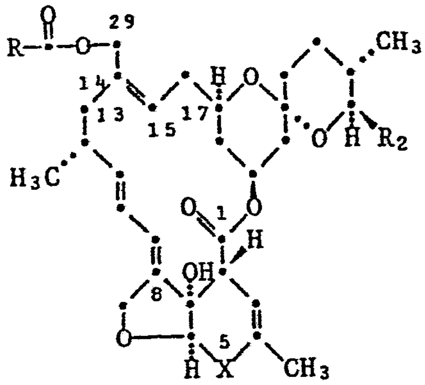

(I)

in welcher

X für -CH($OR_1$)-, -C(O)- oder -C(=N-OH)- steht;

$R_1$ Wasserstoff, eine Silyl-, eine Acylgruppe oder einen Zuckerrest,

$R_2$ Methyl, Ethyl, Isopropyl oder sek.Butyl und

R Wasserstoff, unsubstituierte oder substituierte gerad- oder verzweigtkettige $C_1$-$C_{18}$-Alkyl-Gruppen, unsubstituierte oder substituierte $C_3$-$C_{10}$-Cycloalkyl-Gruppen, unsubstituierte oder substituierte $C_2$-$C_6$-Alkenyl-Gruppen, unsubstituierte oder substituierte $C_2$-$C_6$-Alkinyl-Gruppen oder unsubstituierte oder substituierte Phenyl-Gruppen bedeuten, auf oder in die Wirtstiere, Wirtspflanzen oder sonstigen Lebensräume der Schädlinge appliziert.

19. Verfahren gemäss Anspruch 18, dadurch gekennzeichnet, dass man eine Verbindung der Formel I gemäss einem der Ansprüche 2 bis 11 appliziert.

20. Verfahren nach Anspruch 18, dadurch gekennzeichnet, dass die zu bekämpfenden Schädlinge Tiere befallende Endo- oder Ektoparasiten sind.

21. Verfahren nach Anspruch 20, dadurch gekennzeichnet, dass die zu bekämpfenden Schädlinge pflanzenschädigende Parasiten sind.

0237482

22. Die Verbindung der Formel II

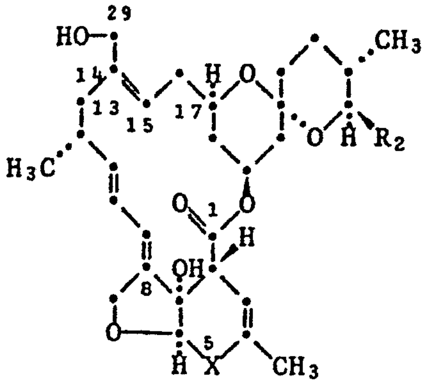

(II)

,

worin X und $R_2$ die unter Formel I angegebenen Bedeutungen haben.

23. Verwendung von Verbindungen der Formel II nach Anspruch 22 zur Herstellung von Pestiziden.

24. Verbindungen der Formel X,

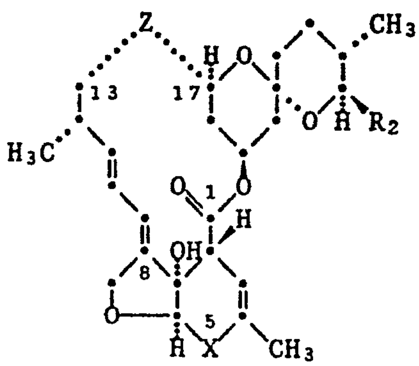

(X),

worin Z für eine der Gruppen

α) 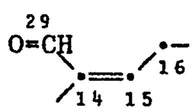 [ = $\Delta^{14,15\text{-trans}}$-29-Oxo = Verbindungen der Formel X ]

oder

β) 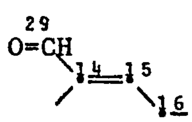 [ = $\Delta^{14,15\text{-cis}}$-29-Oxo = Verbindungen der Formel X ]

steht und X und $R_2$ die unter Formel I angegebenen Bedeutungen haben.

25. Verwendung der Verbindungen der Formel X gemäss Anspruch 24 zur Herstellung von Verbindungen der Formel II gemäss Anspruch 22.

FO 7.5/HL/bg*

Patentansprüche für den Vertragsstaat AT:

1. Verfahren zur Herstellung von Verbindungen der Formel I

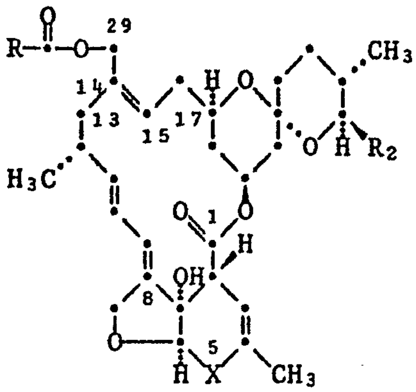

(I)

in welcher

X für $-CH(OR_1)-$ , $-C(O)-$ oder $-C(=N-OH-)-$ steht;

$R_1$ Wasserstoff, eine Silyl-, eine Acylgruppe oder einen Zuckerrest,

$R_2$ Methyl, Ethyl, Isopropyl oder sek.Butyl und

R Wasserstoff, unsubstituierte oder substituierte gerad- oder verzweigtkettige $C_1$-$C_{18}$-Alkyl-Gruppen, unsubstituierte oder substituierte $C_3$-$C_{10}$-Cycloalkyl-Gruppen, unsubstituierte oder substituierte $C_2$-$C_6$-Alkenyl-Gruppen, unsubstituierte oder substituierte $C_2$-$C_6$-Alkinyl-Gruppen oder unsubstituierte oder substituierte Phenyl-Gruppen bedeuten, dadurch gekennzeichnet, dass man entweder eine Verbindung der Formel II

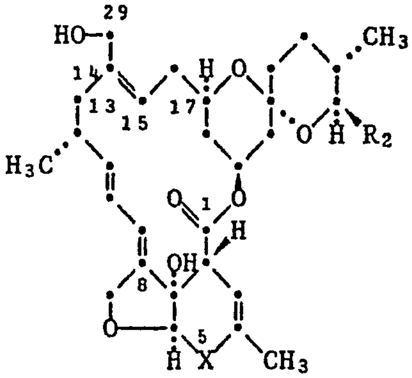

(II)

oder eine Verbindung der Formel III,

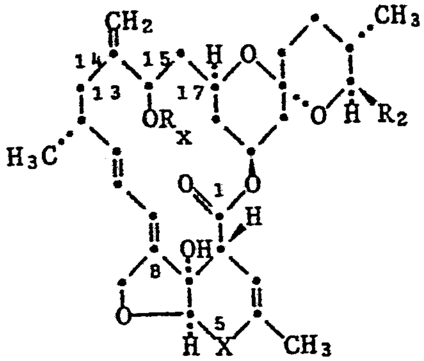

(III)

wobei in den Formeln II und III $R_2$ eine der unter Formel I angegebenen Bedeutungen hat, X für $-CH(OR_1)-$ steht; wobei $R_1$ eine OH-Schutzgruppe bedeutet und $R_x$ in Formel III für Wasserstoff oder eine leicht abspaltbare Gruppe steht, mit einer Säure der Formel IV

R-COOH (IV)

worin R eine der unter Formel I angegebenen Bedeutungen hat, oder einen zur Veresterung befähigten Derivat dieser Säure reagieren lässt oder die Verbindung der Formel III durch Verseifung der $OR_x$-Gruppe zuerst in eine Verbindung der Formel II überführt und anschliessend mit IV umsetzt und die resultierende Verbindung der Formel I, insofern gewünscht, durch Abspaltung der OH-Schutzgruppe in ein 5-Hydroxi-Derivat der Formel I überführt und letzteres, sofern gewünscht, durch Nachsilylierung in ein Silylderivat oder durch Einführung eines Zuckerrestes in ein Zuckerderivat der Formel I überführt und sofern Verbindungen der Formel I, worin X für -C(=N-OH)- steht, das Herstellungsziel darstellen, ein 5-Keton der Formel I mit Hydroxylamin oder einem seiner Salze umsetzt.

2. Verfahren nach Anspruch 1, dadurch gekennzeichnet, dass man als ein zur Veresterung befähigtes Säurederivat der Säure IV

a) ihre Säureamide der Formel V

$RCON(Alkyl)_2$ (V)

worin die Alkylreste 1 bis 4 Kohlenstoffatome umfassen und vorzugsweise Methyl bedeuten,

b) ihre Säurehalogenide der Formel VI

RCOhal (VI),

worin hal Halogen bedeutet und bevorzugt für Chlor oder Brom steht, sowie

c) ihr Säureanhydrid der Formel VII

$(RCO)_2O$ (VII)

worin R die unter Formel I angegebenen Bedeutungen hat, einsetzt.

3. Verfahren nach Anspruch 2, dadurch gekennzeichnet, dass die Reaktion von Verbindungen der Formel II oder III mit einem Säurechlorid oder Säurebromid der Formel VI oder einem Säureanhydrid der Formel VII in einem reaktionsinerten Lösungsmittel bei Temperaturen von 0° bis 100°C durchgeführt wird.

4. Verfahren nach Anspruch 3, dadurch gekennzeichnet, dass als Neutralisationsmittel eine Base eingesetzt wird.

5. Mittel zur Bekämpfung von Schädlingen, dadurch gekennzeichnet, dass es neben Trägermitteln, Verteilungsmitteln oder Träger- und Verteilungsmitteln als Wirkstoff mindestens eine Verbindung der Formel I enthält,

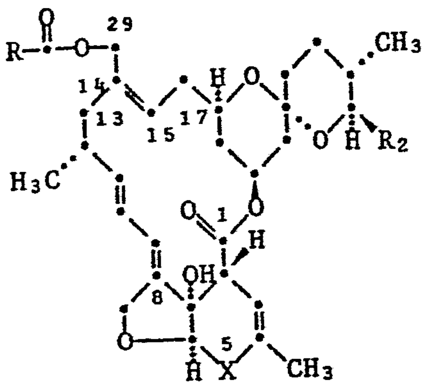

(I)

in welcher

X für $-CH(OR_1)-$, $-C(O)-$ oder $-C(=N-OH)-$ steht;

$R_1$ Wasserstoff, eine Silyl-, eine Acylgruppe oder einen Zuckerrest,

$R_2$ Methyl, Ethyl, Isopropyl oder sek.Butyl und

R Wasserstoff, unsubstituierte oder substituierte gerad- oder verzweigtkettige $C_1$-$C_{18}$-Alkyl-Gruppen, unsubstituierte oder substituierte $C_3$-$C_{10}$-Cycloalkyl-Gruppen, unsubstituierte oder substituierte $C_2$-$C_6$-Alkenyl-Gruppen, unsubstituierte oder substituierte $C_2$-$C_6$-Alkinyl-Gruppen oder unsubstituierte oder substituierte Phenyl-Gruppen bedeuten.

6. Mittel nach Anspruch 5, enthaltend als Wirkstoff eine der Verbindungen der Formel I, worin

X für $-CH(OR_1)-$ oder $-C(O)-$ steht;

$R_1$ Wasserstoff, eine Silyl-, eine Acylgruppe oder einen Zuckerrest,

$R_2$ Methyl, Ethyl, Isopropyl oder sek.Butyl und

R Wasserstoff, unsubstituierte oder substituierte gerad- oder verzweigtkettige $C_1$-$C_{18}$-Alkyl-Gruppen, unsubstituierte oder substituierte $C_3$-$C_{10}$-Cycloalkyl-Gruppen, unsubstituierte oder substituierte $C_2$-$C_6$-Alkenyl-Gruppen, unsubstituierte oder substituierte $C_2$-$C_6$-Alkinyl-Gruppen oder unsubstituierte oder substituierte Phenyl-Gruppen bedeuten.

7. Mittel nach Anspruch 6, enthaltend als Wirkstoff eine der Verbindungen der Formel I, worin X für $-CH(OR_1)-$ steht; $R_1$ Wasserstoff darstellt, $R_2$ für Methyl, Ethyl, Isopropyl oder sek.Butyl steht und R folgende Bedeutungen hat:

- unsubstituiertes oder durch 1 bis 4 Halogenatome oder $C_1$-$C_4$-Alkoxi substituiertes $C_1$-$C_6$-Alkyl, $C_2$-$C_4$-Alkenyl, $C_2$-$C_4$-Alkinyl oder $C_3$-$C_6$-Cycloalkyl;
- unsubstituiertes oder ein bis dreifach durch Halogen, $C_1$-$C_4$-Alkyl, $C_1$-$C_4$-Alkoxi, $C_1$-$C_4$-Alkylthio oder Nitro substituiertes Phenyl.

8. Mittel nach Anspruch 7, enthaltend als Wirkstoff eine der Verbindungen der Formel I, worin X für $-CH(OR_1)-$ steht; $R_1$ Wasserstoff darstellt, $R_2$ für Methyl, Ethyl, Isopropyl oder sek.Butyl steht und R folgende Bedeutungen hat:

- unsubstituiertes oder durch 1 bis 4 Chlor- oder Fluoratome oder Methoxi substituiertes $C_1$-$C_5$-Alkyl, $C_2$-$C_3$-Alkenyl, $C_2$-$C_3$-Alkinyl oder $C_3$-$C_6$-Cycloalkyl;
- unsubstituiertes oder durch Chlor, Fluor, $C_1$-$C_2$-Alkyl, $C_1$-$C_2$-Alkoxi, $C_1$-$C_2$-Alkylthio oder Nitro substituiertes Phenyl.

9. Mittel nach Anspruch 8, enthaltend als Wirkstoff eine der Verbindungen der Formel I, worin X für $-CH(OR_1)-$ steht; $R_1$ Wasserstoff darstellt, $R_2$ für Methyl oder Ethyl steht und R folgende Bedeutungen hat:

- unsubstituiertes oder durch 1 bis 4 Chlor- oder Fluoratome oder Methoxi substituiertes $C_1$-$C_5$-Alkyl, $C_2$-$C_3$-Alkenyl, $C_2$-$C_3$-Alkinyl oder $C_3$-$C_6$-Cycloalkyl;
- unsubstituiertes oder durch Chlor, Fluor, $C_1$-$C_2$-Alkyl, $C_1$-$C_2$-Alkoxi, $C_1$-$C_2$-Alkylthio oder Nitro substituiertes Phenyl.

10. Mittel nach Anspruch 8, enthaltend als Wirkstoff eine der Verbindungen der Formel I, worin X für $-CH(OR_1)-$ steht; $R_1$ Wasserstoff darstellt, $R_2$ für Isopropyl oder sek.Butyl steht und R folgende Bedeutungen hat:

- unsubstituiertes oder durch 1 bis 4 Chlor- oder Fluoratome oder Methoxi substituiertes $C_1$-$C_5$-Alkyl, $C_2$-$C_3$-Alkenyl, $C_2$-$C_3$-Alkinyl oder $C_3$-$C_6$-Cycloalkyl;
- unsubstituiertes oder durch Chlor, Fluor, $C_1$-$C_2$-Alkyl, $C_1$-$C_2$-Alkoxi, $C_1$-$C_2$-Alkylthio oder Nitro substituiertes Phenyl.

11. Mittel nach Anspruch 8, enthaltend als Wirkstoff eine der Verbindungen der Formel I, worin X für $-CH(OR_1)-$ steht; $R_1$ Wasserstoff darstellt, $R_2$ für Methyl oder Ethyl, Isopropyl oder sek.Butyl steht und R folgende Bedeutungen hat:

- unsubstituiertes oder durch 1 bis 3 Chloratome oder Fluoratome oder Methoxi substituiertes $C_1$-$C_5$-Alkyl, $C_2$-$C_3$-Alkenyl, $C_2$-$C_3$-Alkinyl oder $C_3$-$C_6$-Cycloalkyl;
- unsubstituiertes oder durch Chlor, Fluor, $C_1$-$C_2$-Alkyl, $C_1$-$C_2$-Alkoxi, $C_1$-$C_2$-Alkylthio oder Nitro substituiertes Phenyl.

12. Mittel nach Anspruch 11, enthaltend als Wirkstoff eine der Verbindungen der Formel I, worin X für $-CH(OR_1)-$ steht; $R_1$ Wasserstoff darstellt, $R_2$ für Methyl oder Ethyl oder steht und R folgende Bedeutungen hat:

- unsubstituiertes oder durch 1 bis 3 Chloratome oder Fluoratome oder Methoxi substituiertes $C_1$-$C_5$-Alkyl, $C_2$-$C_3$-Alkenyl, $C_2$-$C_3$-Alkinyl oder $C_3$-$C_6$-Cycloalkyl.

13. Mittel nach Anspruch 9, enthaltend als Wirkstoff eine der Verbindungen der Formel I, worin X für $-CH(OR_1)-$ steht; $R_1$ Wasserstoff darstellt, $R_2$ für Isopropyl oder sek.Butyl steht und R folgende Bedeutungen hat:

- unsubstituiertes oder durch 1 bis 3 Chloratome oder Fluoratome oder Methoxi substituiertes $C_1$-$C_5$-Alkyl, $C_2$-$C_3$-Alkenyl, $C_2$-$C_3$-Alkinyl oder $C_3$-$C_6$-Cycloalkyl.

14. Mittel nach Anspruch 6, enthaltend als Wirkstoff eine Verbindung der Formel I, ausgewählt aus der Reihe:
29-tert.Butylcarbonyloxi-milbemycin-D,
29-Cyclopropylcarbonyloxi-milbemycin-$A_4$.
29-tert.Butylcarbonyloxi-milbemycin-$A_4$,
29-iso-Butylcarbonyloxi-milbemycin-$A_4$,
29-(2,2-Dimethylpropyl)carbonyloxi-milbemycin-$A_4$ und
29-Acetoxi-milbemycin-D.

15. Mittel nach Anspruch 6, enthaltend als Wirkstoff eine Verbindung der Formel I, ausgewählt aus der Reihe:
5-O-tert.Butyldimethylsilyl-29-tert.butylcarbonyloxi-milbemycin-D,
5-O-tert.Butyldimethylsilyl-29-cyclopropylcarbonyloxi-milbemycin-$A_4$,
5-O-tert.Butyldimethylsilyl-29-acetoxi-milbemycin-D,
5-O-tert.Butyldimethylsilyl-29-tert.butylcarbonyloxi-milbemycin-$A_4$,
5-O-tert.Butyldimethylsilyl-29-(2,2-dimethylcarbonyloxi-milbemycin-$A_4$,
5-O-tert.Butyldimethylsilyl-29-iso-butylcarbonyloxi-milbemycin-$A_4$ und
5-O-2',3',4',6'-Tetra-O-acetyl-galactose-29-tert.butylcarbonyloxi-milbemycin-D.

16. Verfahren zur Bekämpfung von Schädlingen, dadurch gekennzeichnet, dass man eine pestizid-wirksame Menge mindestens einer Verbindung der Formel I

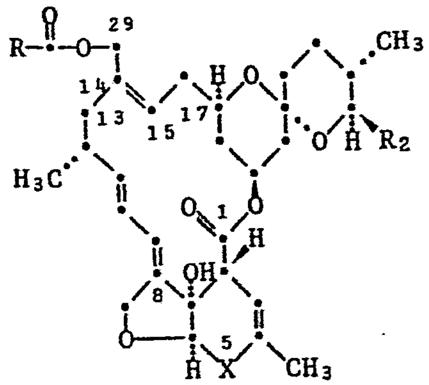

(I)

in welcher

X für $-CH(OR_1)-$, $-C(O)-$ oder $-C(=N-OH)-$ steht;

$R_1$ Wasserstoff, eine Silyl-, eine Acylgruppe oder einen Zuckerrest,

$R_2$ Methyl, Ethyl, Isopropyl oder sek.Butyl und

R Wasserstoff, unsubstituierte oder substituierte gerad- oder verzweigtkettige $C_1$-$C_{18}$-Alkyl-Gruppen, unsubstituierte oder substituierte $C_3$-$C_{10}$-Cycloalkyl-Gruppen, unsubstituierte oder substituierte $C_2$-$C_6$-Alkenyl-Gruppen, unsubstituierte oder substituierte $C_2$-$C_6$-Alkinyl-Gruppen oder unsubstituierte oder substituierte Phenyl-Gruppen bedeuten, auf oder in die Wirtstiere, Wirtspflanzen oder sonstigen Lebensräume der Schädlinge appliziert.

17. Verfahren nach Anspruch 16, dadurch gekennzeichnet, dass man eine Verbindung der Formel I gemäss einem der Ansprüche 5 bis 14 appliziert.

18. Verfahren nach Anspruch 16, dadurch gekennzeichnet, dass die zu bekämpfenden Schädlinge Tiere befallende Endo- oder Ektoparasiten sind.

19. Verfahren nach Anspruch 18, dadurch gekennzeichnet, dass die zu bekämpfenden Schädlinge pflanzenschädigende Parasiten sind.

0237482

20. Verwendung einer Verbindung der Formel II

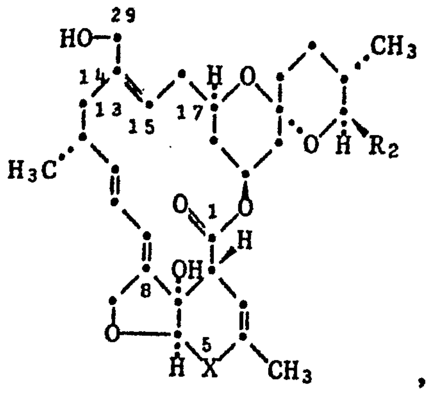

(II)

,

worin X und $R_2$ die unter Formel I angegebenen Bedeutungen haben zur Herstellung von Verbindungen der Formel I.

FO 7.5 HL/bg*

Patentansprüche für den Vertragsstaat ES

1. Verfahren zur Herstellung von Verbindungen der Formel I

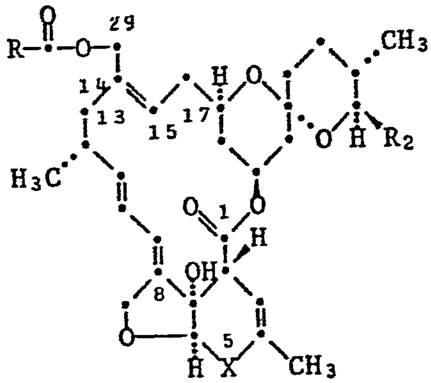

(I)

in welcher

X für -CH($OR_1$)- , -C(O)- oder -C(=N-OH-)- steht;

$R_1$ Wasserstoff, eine Silyl-, eine Acylgruppe oder einen Zuckerrest,

$R_2$ Methyl, Ethyl, Isopropyl oder sek.Butyl und

R Wasserstoff, unsubstituierte oder substituierte gerad- oder verzweigtkettige $C_1$-$C_{18}$-Alkyl-Gruppen, unsubstituierte oder substituierte $C_3$-$C_{10}$-Cycloalkyl-Gruppen, unsubstituierte oder substituierte $C_2$-$C_6$-Alkenyl-Gruppen, unsubstituierte oder substituierte $C_2$-$C_6$-Alkinyl-Gruppen oder unsubstituierte oder substituierte Phenyl-Gruppen bedeuten, dadurch gekennzeichnet, dass man entweder eine Verbindung der Formel II

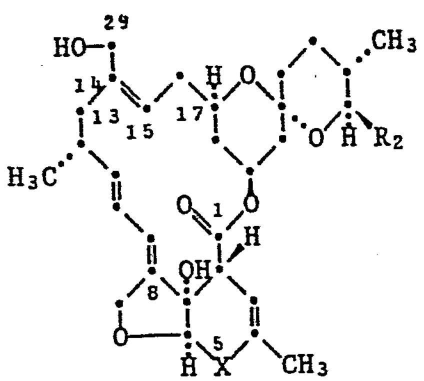

(II)

oder eine Verbindung der Formel III,

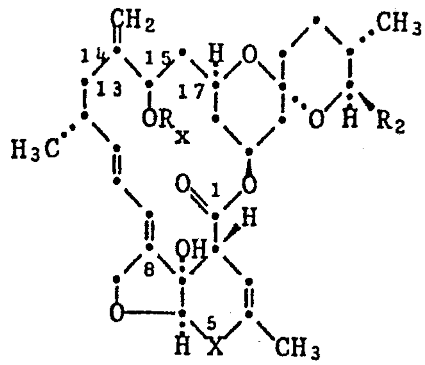

(III)

wobei in den Formeln II und III $R_2$ eine der unter Formel I angegebenen Bedeutungen hat, X für $-CH(OR_1)-$ steht; wobei $R_1$ eine OH-Schutzgruppe bedeutet und $R_x$ in Formel III für Wasserstoff oder eine leicht abspaltbare Gruppe steht, mit einer Säure der Formel IV

R-COOH (IV)

worin R eine der unter Formel I angegebenen Bedeutungen hat, oder einen zur Veresterung befähigten Derivat dieser Säure reagieren lässt oder die Verbindung der Formel III durch Verseifung der $OR_x$-Gruppe zuerst in eine Verbindung der Formel II überführt und anschliessend mit IV umsetzt und die resultierende Verbindung der Formel I, insofern gewünscht, durch Abspaltung der OH-Schutzgruppe in ein 5-Hydroxi-Derivat der Formel I überführt und letzteres, sofern gewünscht, durch Nachsilylierung in ein Silylderivat oder durch Einführung eines Zuckerrestes in ein Zuckerderivat der Formel I überführt und sofern Verbindungen der Formel I, worin X für -C(=N-OH)- steht, das Herstellungsziel darstellen, ein 5-Keton der Formel I mit Hydroxylamin oder einem seiner Salze umsetzt.

2. Verfahren nach Anspruch 1, dadurch gekennzeichnet, dass man als ein zur Veresterung befähigtes Säurederivat der Säure IV

a) ihre Säureamide der Formel V

$RCON(Alkyl)_2$ (V)

worin die Alkylreste 1 bis 4 Kohlenstoffatome umfassen und vorzugsweise Methyl bedeuten,

b) ihre Säurehalogenide der Formel VI

RCOhal (VI),

worin hal Halogen bedeutet und bevorzugt für Chlor oder Brom steht, sowie

c) ihr Säureanhydrid der Formel VII

$(RCO)_2O$ (VII)

worin R die unter Formel I angegebenen Bedeutungen hat, einsetzt.

3. Verfahren nach Anspruch 2, dadurch gekennzeichnet, dass die Reaktion von Verbindungen der Formel II oder III mit einem Säurechlorid oder Säurebromid der Formel VI oder einem Säureanhydrid der Formel VII in einem reaktionsinerten Lösungsmittel bei Temperaturen von 0° bis 100°C durchgeführt wird.

4. Verfahren nach Anspruch 3, dadurch gekennzeichnet, dass als Neutralisationsmittel eine Base eingesetzt wird.

5. Mittel zur Bekämpfung von Schädlingen, dadurch gekennzeichnet, dass es neben Trägermitteln, Verteilungsmitteln oder Träger- und Verteilungsmitteln als Wirkstoff mindestens eine Verbindung der Formel I enthält,

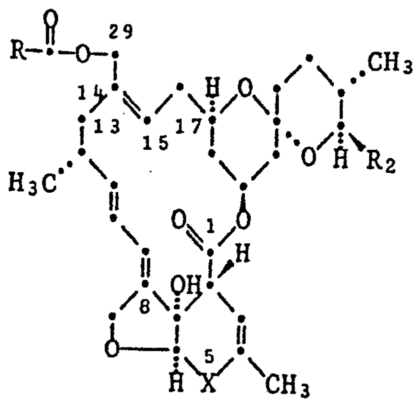

(I)

in welcher

X für $-CH(OR_1)-$, $-C(O)-$ oder $-C(=N-OH)-$ steht;

$R_1$ Wasserstoff, eine Silyl-, eine Acylgruppe oder einen Zuckerrest,

$R_2$ Methyl, Ethyl, Isopropyl oder sek.Butyl und

R Wasserstoff, unsubstituierte oder substituierte gerad- oder verzweigtkettige $C_1$-$C_{18}$-Alkyl-Gruppen, unsubstituierte oder substituierte $C_3$-$C_{10}$-Cycloalkyl-Gruppen, unsubstituierte oder substituierte $C_2$-$C_6$-Alkenyl-Gruppen, unsubstituierte oder substituierte $C_2$-$C_6$-Alkinyl-Gruppen oder unsubstituierte oder substituierte Phenyl-Gruppen bedeuten.

6. Mittel nach Anspruch 5, enthaltend als Wirkstoff eine der Verbindungen der Formel I, worin

X für $-CH(OR_1)-$ oder $-C(O)-$ steht;

$R_1$ Wasserstoff, eine Silyl-, eine Acylgruppe oder einen Zuckerrest,

$R_2$ Methyl, Ethyl, Isopropyl oder sek.Butyl und

R Wasserstoff, unsubstituierte oder substituierte gerad- oder verzweigtkettige $C_1$-$C_{18}$-Alkyl-Gruppen, unsubstituierte oder substituierte $C_3$-$C_{10}$-Cycloalkyl-Gruppen, unsubstituierte oder substituierte $C_2$-$C_6$-Alkenyl-Gruppen, unsubstituierte oder substituierte $C_2$-$C_6$-Alkinyl-Gruppen oder unsubstituierte oder substituierte Phenyl-Gruppen bedeuten.

7. Mittel nach Anspruch 6, enthaltend als Wirkstoff eine der Verbindungen der Formel I, worin X für $-CH(OR_1)-$ steht; $R_1$ Wasserstoff darstellt, $R_2$ für Methyl, Ethyl, Isopropyl oder sek.Butyl steht und R folgende Bedeutungen hat:

- unsubstituiertes oder durch 1 bis 4 Halogenatome oder $C_1$-$C_4$-Alkoxi substituiertes $C_1$-$C_6$-Alkyl, $C_2$-$C_4$-Alkenyl, $C_2$-$C_4$-Alkinyl oder $C_3$-$C_6$-Cycloalkyl;
- unsubstituiertes oder ein bis dreifach durch Halogen, $C_1$-$C_4$-Alkyl, $C_1$-$C_4$-Alkoxi, $C_1$-$C_4$-Alkylthio oder Nitro substituiertes Phenyl.

8. Mittel nach Anspruch 7, enthaltend als Wirkstoff eine der Verbindungen der Formel I, worin X für $-CH(OR_1)-$ steht; $R_1$ Wasserstoff darstellt, $R_2$ für Methyl, Ethyl, Isopropyl oder sek.Butyl steht und R folgende Bedeutungen hat:

- unsubstituiertes oder durch 1 bis 4 Chlor- oder Fluoratome oder Methoxi substituiertes $C_1$-$C_5$-Alkyl, $C_2$-$C_3$-Alkenyl, $C_2$-$C_3$-Alkinyl oder $C_3$-$C_6$-Cycloalkyl;
- unsubstituiertes oder durch Chlor, Fluor, $C_1$-$C_2$-Alkyl, $C_1$-$C_2$-Alkoxi, $C_1$-$C_2$-Alkylthio oder Nitro substituiertes Phenyl.

9. Mittel nach Anspruch 8, enthaltend als Wirkstoff eine der Verbindungen der Formel I, worin X für $-CH(OR_1)-$ steht; $R_1$ Wasserstoff darstellt, $R_2$ für Methyl oder Ethyl steht und R folgende Bedeutungen hat:

- unsubstituiertes oder durch 1 bis 4 Chlor- oder Fluoratome oder Methoxi substituiertes $C_1$-$C_5$-Alkyl, $C_2$-$C_3$-Alkenyl, $C_2$-$C_3$-Alkinyl oder $C_3$-$C_6$-Cycloalkyl;
- unsubstituiertes oder durch Chlor, Fluor, $C_1$-$C_2$-Alkyl, $C_1$-$C_2$-Alkoxi, $C_1$-$C_2$-Alkylthio oder Nitro substituiertes Phenyl.

10. Mittel nach Anspruch 8, enthaltend als Wirkstoff eine der Verbindungen der Formel I, worin X für $-CH(OR_1)-$ steht; $R_1$ Wasserstoff darstellt, $R_2$ für Isopropyl oder sek.Butyl steht und R folgende Bedeutungen hat:

- unsubstituiertes oder durch 1 bis 4 Chlor- oder Fluoratome oder Methoxi substituiertes $C_1$-$C_5$-Alkyl, $C_2$-$C_3$-Alkenyl, $C_2$-$C_3$-Alkinyl oder $C_3$-$C_6$-Cycloalkyl;
- unsubstituiertes oder durch Chlor, Fluor, $C_1$-$C_2$-Alkyl, $C_1$-$C_2$-Alkoxi, $C_1$-$C_2$-Alkylthio oder Nitro substituiertes Phenyl.

11. Mittel nach Anspruch 8, enthaltend als Wirkstoff eine der Verbindungen der Formel I, worin X für $-CH(OR_1)-$ steht; $R_1$ Wasserstoff darstellt, $R_2$ für Methyl oder Ethyl, Isopropyl oder sek.Butyl steht und R folgende Bedeutungen hat:

- unsubstituiertes oder durch 1 bis 3 Chloratome oder Fluoratome oder Methoxi substituiertes $C_1$-$C_5$-Alkyl, $C_2$-$C_3$-Alkenyl, $C_2$-$C_3$-Alkinyl oder $C_3$-$C_6$-Cycloalkyl;
- unsubstituiertes oder durch Chlor, Fluor, $C_1$-$C_2$-Alkyl, $C_1$-$C_2$-Alkoxi, $C_1$-$C_2$-Alkylthio oder Nitro substituiertes Phenyl.

12. Mittel nach Anspruch 11, enthaltend als Wirkstoff eine der Verbindungen der Formel I, worin X für $-CH(OR_1)-$ steht; $R_1$ Wasserstoff darstellt, $R_2$ für Methyl oder Ethyl oder steht und R folgende Bedeutungen hat:

- unsubstituiertes oder durch 1 bis 3 Chloratome oder Fluoratome oder Methoxi substituiertes $C_1$-$C_5$-Alkyl, $C_2$-$C_3$-Alkenyl, $C_2$-$C_3$-Alkinyl oder $C_3$-$C_6$-Cycloalkyl.

13. Mittel nach Anspruch 9, enthaltend als Wirkstoff eine der Verbindungen der Formel I, worin X für $-CH(OR_1)-$ steht; $R_1$ Wasserstoff darstellt, $R_2$ für Isopropyl oder sek.Butyl steht und R folgende Bedeutungen hat:

- unsubstituiertes oder durch 1 bis 3 Chloratome oder Fluoratome oder Methoxi substituiertes $C_1$-$C_5$-Alkyl, $C_2$-$C_3$-Alkenyl, $C_2$-$C_3$-Alkinyl oder $C_3$-$C_6$-Cycloalkyl.

14. Mittel nach Anspruch 6, enthaltend als Wirkstoff eine Verbindung der Formel I, ausgewählt aus der Reihe:
29-tert.Butylcarbonyloxi-milbemycin-D,
29-Cyclopropylcarbonyloxi-milbemycin-$A_4$.
29-tert.Butylcarbonyloxi-milbemycin-$A_4$,
29-iso-Butylcarbonyloxi-milbemycin-$A_4$,
29-(2,2-Dimethylpropyl)carbonyloxi-milbemycin-$A_4$ und
29-Acetoxi-milbemycin-D.

15. Mittel nach Anspruch 6, enthaltend als Wirkstoff eine Verbindung der Formel 1, ausgewählt aus der Reihe:
5-O-tert.Butyldimethylsilyl-29-tert.butylcarbonyloxi-milbemycin-D,
5-O-tert.Butyldimethylsilyl-29-cyclopropylcarbonyloxi-milbemycin-$A_4$,
5-O-tert.Butyldimethylsilyl-29-acetoxi-milbemycin-D,
5-O-tert.Butyldimethylsilyl-29-tert.butylcarbonyloxi-milbemycin-$A_4$,
5-O-tert.Butyldimethylsilyl-29-(2,2-dimethylcarbonyloxi-milbemycin-$A_4$,
5-O-tert.Butyldimethylsilyl-29-iso-butylcarbonyloxi-milbemycin-$A_4$ und
5-O-2',3',4',6'-Tetra-O-acetyl-galactose-29-tert.butylcarbonyloxi-milbemycin-D.

FO 7.5 HL/bg*

Europäisches Patentamt

# EUROPÄISCHER TEILRECHERCHENBERICHT,

der nach Regel 45 des Europäischen Patentübereinkommens für das weitere Verfahren als europäischer Recherchenbericht gilt

Nummer der Anmeldung
0237482

EP 87810115.3

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP - A2/A3 - 0 147 852 (CIBA-GEIGY AG)<br>* Ansprüche 1,11,16-21 *<br>-- | 1,16-19,21,22,24 | C 07 D 493/22<br>C 07 H 17/08<br>A 01 N 43/20<br>A 61 K 31/365<br>//(C 07 D 493/22<br>C 07 D 313:00<br>C 07 D 311:00<br>C 07 D 311:00<br>C 07 D 307:00) |
| A | EP - A1 - 0 142 969 (SANKYO COMPANY LIMITED)<br>* Ansprüche 1,13 *<br>-- | 1,16-19,21,22,24 | |
| A | EP - A2 - 0 165 900 (CIBA-GEIGY AG)<br>* Ansprüche 1,9,10-16 *<br>-- | 1,16-19,21,22,24 | |

RECHERCHIERTE SACHGEBIETE (Int Cl 4)

C 07 D 493/00
C 07 H 17/00

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-17,21-25
Unvollständig recherchierte Patentansprüche: 18,19
Nicht recherchierte Patentansprüche: 20
Grund für die Beschränkung der Recherche:

(Artikel 52(4) EPÜ; Verfahren zur therapeutischen Behandlung des menschlichen oder tierischen Körpers)

| Recherchenort | Abschlußdatum der Recherche | Prüfer |
|---|---|---|
| WIEN | 12-06-1987 | BRUS |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

Europäisches Patentamt

# EUROPÄISCHER TEILRECHERCHENBERICHT

Nummer der Anmeldung 0237482

EP 87810115.3

| EINSCHLÄGIGE DOKUMENTE | | | KLASSIFIKATION DER ANMELDUNG (Int. Cl. 4) |
|---|---|---|---|
| Kategorie | Kennzeichnung des Dokuments mit Angabe, soweit erforderlich, der maßgeblichen Teile | betrifft Anspruch | |
| A,P | CHEMICAL ABSTRACTS, Band 104, Nr. 13, 31. März 1986, Columbus, Ohio, USA<br>IDE,J., MURAMATSU, S.; NAKADA, Y.; KITANO, K.; "5-Didehydromilbemycin 5-oxime derivatives" Seite 724, Spalte 2, Zusammenfassung Nr. 109 603r<br>& JPN. Kokai Tokkyo JP 60-142 991 2. Juli 1985<br>---- | 1,16-19,21,22,24 | |
| | | | RECHERCHIERTE SACHGEBIETE (Int. Cl. 4) |

EPA Form 1505.3 06.78